(19) Europäisches Patentamt
European Patent Office
Office européen des brevets

(11) EP 2 145 904 A1

(12) EUROPÄISCHE PATENTANMELDUNG

(43) Veröffentlichungstag:
**20.01.2010 Patentblatt 2010/03**

(51) Int Cl.:
***C08F 8/12*** *(2006.01)* ***C12N 9/18*** *(2006.01)*

(21) Anmeldenummer: **08160774.9**

(22) Anmeldetag: **18.07.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(71) Anmelder: **BASF SE**
**67056 Ludwigshafen (DE)**

(72) Erfinder:
- **Hauer, Bernhard, Prof. Dr.**
  **67136 Fußgönheim (DE)**
- **Höffken, Hans Wolfgang, Dr.**
  **67069 Ludwigshafen (DE)**
- **Kvarnström Branneby, Cecilia, Dr.**
  **11355 Stockholm (SE)**
- **Schwab, Helmut, Prof.**
  **8010 Graz (AT)**
- **Feichtenhöfer, Sabine, Dr.**
  **8010 Graz (AT)**
- **Buchebner, Marlene**
  **8664 Veitsch (AT)**
- **Pohn, Brigitte**
  **8010 Graz (AT)**

(74) Vertreter: **Reitstötter - Kinzebach**
**Patentanwälte**
**Sternwartstrasse 4**
**81679 München (DE)**

# (54) Verfahren zur enzymkatalysierten Hydrolyse von Polyacrylsäureestern sowie dafür zu verwendende Esterasen

(57) Die vorliegende Erfindung betrifft ein Verfahren zur enzymkatalysierten Hydrolyse von Polyacrylsäureestern, wobei man wenigstens einen Polyacrylsäureester bereitstellt, den wenigstens einen Polyacrylsäureester mit wenigstens einem Enzym inkubiert, das ausgewählt ist unter auf Esterbindungen einwirkenden Enzymen (EC 3.1), bis die in dem Polyacrylsäureester enthaltenen Estergruppen teilweise oder vollständig hydrolytisch gespalten sind, und gegebenenfalls das dadurch erhaltene modifizierte Polymer isoliert. Die vorliegende Erfindung betrifft weiterhin die dabei verwendeten Enzyme und Mutanten davon, die Enzyme kodierende Nukleinsäuren, die Nukleinsäuren umfassende Vektoren, die Vektoren umfassende Mikroorganismen, die Verwendung der Enzyme, der Vektoren oder der Mikroorganismen zur Durchführung eines Verfahrens zur enzymkatalysierten Hydrolyse von Polyacrylsäureestern. Die vorliegende Anmeldung betrifft weiterhin durch das Verfahren erhältliche polymere Reaktionsprodukte sowie Verfahren zur Herstellung von Esterasen.

EP 2 145 904 A1

SEQUENZPROTOKOLL

<110> BASF SE

<120> Verfahren zur enzymkatalysierten Hydrolyse von Polyacrylsäureestern

<130> M/47360

<160> 59

<170> PatentIn version 3.3

<210> 1
<211> 392
<212> PRT
<213> Burkholderia gladioli

<400> 1

```
Met Thr Ala Ala Ser Leu Asp Pro Thr Ala Phe Ser Leu Asp Ala Ala
1               5                   10                  15

Ser Leu Ala Ala Arg Leu Asp Ala Val Phe Asp Gln Ala Leu Arg Glu
            20                  25                  30

Arg Arg Leu Val Gly Ala Val Ala Ile Val Ala Arg His Gly Glu Ile
        35                  40                  45

Leu Tyr Arg Arg Ala Gln Gly Leu Ala Asp Arg Glu Ala Gly Arg Pro
    50                  55                  60

Met Arg Glu Asp Thr Leu Phe Arg Leu Ala Ser Val Thr Lys Pro Ile
65                  70                  75                  80

Val Ala Leu Ala Val Leu Arg Leu Val Ala Arg Gly Glu Leu Ala Leu
                85                  90                  95

Asp Ala Pro Val Thr Arg Trp Leu Pro Glu Phe Arg Pro Arg Leu Ala
            100                 105                 110

Asp Gly Ser Glu Pro Leu Val Thr Ile His His Leu Leu Thr His Thr
        115                 120                 125

Ser Gly Leu Gly Tyr Trp Leu Leu Glu Gly Ala Gly Ser Val Tyr Asp
    130                 135                 140

Arg Leu Gly Ile Ser Asp Gly Ile Asp Leu Arg Asp Phe Asp Leu Asp
145                 150                 155                 160

Glu Asn Leu Arg Arg Leu Ala Ser Ala Pro Leu Ser Phe Ala Pro Gly
                165                 170                 175
```

```
Ser Gly Trp Gln Tyr Ser Leu Ala Leu Asp Val Leu Gly Ala Val Val
            180                 185                 190

Glu Arg Ala Thr Gly Gln Pro Leu Ala Ala Ala Val Asp Ala Leu Val
        195                 200                 205

Ala Gln Pro Leu Gly Met Arg Asp Cys Gly Phe Val Ser Ala Glu Pro
    210                 215                 220

Glu Arg Phe Ala Val Pro Tyr His Asp Gly Gln Pro Glu Pro Val Arg
225                 230                 235                 240

Met Arg Asp Gly Ile Glu Val Pro Leu Pro Glu Gly His Gly Ala Ala
                245                 250                 255

Val Arg Phe Ala Pro Ser Arg Val Phe Glu Pro Gly Ala Tyr Pro Ser
            260                 265                 270

Gly Gly Ala Gly Met Tyr Gly Ser Ala Asp Asp Val Leu Arg Ala Leu
        275                 280                 285

Glu Ala Ile Arg Ala Asn Pro Gly Phe Leu Pro Glu Thr Leu Ala Asp
    290                 295                 300

Ala Ala Arg Arg Asp Gln Ala Gly Val Gly Ala Glu Thr Arg Gly Pro
305                 310                 315                 320

Gly Trp Gly Phe Gly Tyr Leu Ser Ala Val Leu Asp Asp Pro Ala Ala
                325                 330                 335

Ala Gly Thr Pro Gln His Ala Gly Thr Leu Gln Trp Gly Gly Val Tyr
            340                 345                 350

Gly His Ser Trp Phe Val Asp Arg Ala Leu Gly Leu Ser Val Leu Leu
        355                 360                 365

Leu Thr Asn Thr Ala Tyr Glu Gly Met Ser Gly Pro Leu Thr Ile Ala
    370                 375                 380

Leu Arg Asp Ala Val Tyr Ala Arg
385                 390
```

<210> 2
<211> 298
<212> PRT
<213> Burkholderia gladioli

<400> 2

```
Met Asn His Pro Asp Ile Asp Thr His Ser Arg Asn Ala Ala Ala Pro
1               5                   10                  15

Leu Pro Phe Val Leu Val His Gly Ala Trp His Gly Ala Trp Ala Tyr
            20                  25                  30

Glu Arg Leu Gly Ala Ala Leu Ala Ala Arg Gly His Ala Ser Val Ala
        35                  40                  45

His Asp Leu Pro Ala His Gly Ile Asn Ala Arg Tyr Pro Ala Ala Phe
    50                  55                  60

Trp Gln Gly Asp Ala Gln Ala Leu Ala Gln Glu Pro Ser Pro Val Ala
65                  70                  75                  80

Ala Thr Thr Leu Asp Asp Tyr Thr Gly Gln Val Leu Arg Ala Ile Asp
                85                  90                  95

Ala Ala Cys Ala Leu Gly His Pro Arg Val Val Leu Val Gly His Ser
            100                 105                 110

Met Gly Gly Val Ala Ile Thr Ala Ala Ala Glu Arg Ala Pro Glu Arg
        115                 120                 125

Ile Ala Ala Leu Val Tyr Leu Ala Ala Phe Met Pro Ala Ser Gly Val
    130                 135                 140

Pro Gly Leu Asp Tyr Val Arg Ala Pro Glu Asn His Gly Glu Met Leu
145                 150                 155                 160

Ala Ser Leu Ile Cys Ala Ser Pro Arg Ala Ile Gly Ala Leu Arg Ile
                165                 170                 175

Asn Pro Ala Ser Arg Asp Ala Ala Tyr Leu Ala Thr Leu Lys Gln Ala
            180                 185                 190

Leu Phe Glu Asp Val Asp Glu Ala Thr Phe Arg Ala Val Thr Arg Leu
        195                 200                 205

Met Ser Ser Asp Val Pro Thr Ala Pro Phe Ala Thr Pro Ile Ala Thr
    210                 215                 220

Thr Ala Glu Arg Trp Gly Ser Ile Ala Arg His Tyr Val Thr Cys Ala
225                 230                 235                 240

Glu Asp Arg Val Ile Leu Pro Ala Leu Gln Arg Arg Phe Ile Ala Glu
                245                 250                 255
```

```
Ala Asp Ala Phe Leu Pro Glu Arg Pro Thr Arg Val His Ala Leu Asp
            260                 265                 270

Ser Ser His Ser Pro Phe Leu Ser Gln Pro Asp Thr Leu Ala Glu Leu
        275                 280                 285

Leu Thr Gly Ile Ala Arg Asn Thr Ala Ile
    290                 295
```

```
<210>  3
<211>  392
<212>  PRT
<213>  Artificial

<220>
<223>  abgeleitet von SEQ ID NO:1; iinterne Bezeichnung: N27

<220>
<221>  MISC_FEATURE
<222>  (17)..(17)
<223>  Serin 17 des Wildtyps (SEQ ID NO:1) durch Leucin ersetzt

<220>
<221>  MISC_FEATURE
<222>  (132)..(132)
<223>  Glycin 132 des Wildtyps (SEQ ID NO:1) durch Serin ersetzt

<220>
<221>  MISC_FEATURE
<222>  (251)..(251)
<223>  Glutaminsäure  251 des Wildtyps (SEQ ID NO:1) durch Glycin
       ersetzt

<220>
<221>  MISC_FEATURE
<222>  (311)..(311)
<223>  Alanin 311 des Wildtyps (SEQ ID NO:1) durch Valin ersetzt

<220>
<221>  MISC_FEATURE
<222>  (316)..(316)
<223>  Glutaminsäure 316 des Wildtyps (SEQ ID NO:1) durch Lysin ersetzt

<400>  3

Met Thr Ala Ala Ser Leu Asp Pro Thr Ala Phe Ser Leu Asp Ala Ala
1               5                   10                  15

Leu Leu Ala Ala Arg Leu Asp Ala Val Phe Asp Gln Ala Leu Arg Glu
            20                  25                  30

Arg Arg Leu Val Gly Ala Val Ala Ile Val Ala Arg His Gly Glu Ile
        35                  40                  45
```

```
Leu Tyr Arg Arg Ala Gln Gly Leu Ala Asp Arg Glu Ala Gly Arg Pro
    50                  55                  60

Met Arg Glu Asp Thr Leu Phe Arg Leu Ala Ser Val Thr Lys Pro Ile
65                  70                  75                  80

Val Ala Leu Ala Val Leu Arg Leu Val Ala Arg Gly Glu Leu Ala Leu
                85                  90                  95

Asp Ala Pro Val Thr Arg Trp Leu Pro Glu Phe Arg Pro Arg Leu Ala
            100                 105                 110

Asp Gly Ser Glu Pro Leu Val Thr Ile His His Leu Leu Thr His Thr
        115                 120                 125

Ser Gly Leu Ser Tyr Trp Leu Leu Glu Gly Ala Gly Ser Val Tyr Asp
    130                 135                 140

Arg Leu Gly Ile Ser Asp Gly Ile Asp Leu Arg Asp Phe Asp Leu Asp
145                 150                 155                 160

Glu Asn Leu Arg Arg Leu Ala Ser Ala Pro Leu Ser Phe Ala Pro Gly
                165                 170                 175

Ser Gly Trp Gln Tyr Ser Leu Ala Leu Asp Val Leu Gly Ala Val Val
            180                 185                 190

Glu Arg Ala Thr Gly Gln Pro Leu Ala Ala Ala Val Asp Ala Leu Val
        195                 200                 205

Ala Gln Pro Leu Gly Met Arg Asp Cys Gly Phe Val Ser Ala Glu Pro
    210                 215                 220

Glu Arg Phe Ala Val Pro Tyr His Asp Gly Gln Pro Glu Pro Val Arg
225                 230                 235                 240

Met Arg Asp Gly Ile Glu Val Pro Leu Pro Gly Gly His Gly Ala Ala
                245                 250                 255

Val Arg Phe Ala Pro Ser Arg Val Phe Glu Pro Gly Ala Tyr Pro Ser
            260                 265                 270

Gly Gly Ala Gly Met Tyr Gly Ser Ala Asp Asp Val Leu Arg Ala Leu
        275                 280                 285

Glu Ala Ile Arg Ala Asn Pro Gly Phe Leu Pro Glu Thr Leu Ala Asp
    290                 295                 300
```

```
Ala Ala Arg Arg Asp Gln Val Gly Val Gly Ala Lys Thr Arg Gly Pro
305                 310                 315                 320

Gly Trp Gly Phe Gly Tyr Leu Ser Ala Val Leu Asp Asp Pro Ala Ala
                325                 330                 335

Ala Gly Thr Pro Gln His Ala Gly Thr Leu Gln Trp Gly Gly Val Tyr
            340                 345                 350

Gly His Ser Trp Phe Val Asp Arg Ala Leu Gly Leu Ser Val Leu Leu
        355                 360                 365

Leu Thr Asn Thr Ala Tyr Glu Gly Met Ser Gly Pro Leu Thr Ile Ala
    370                 375                 380

Leu Arg Asp Ala Val Tyr Ala Arg
385                 390
```

<210> 4
<211> 392
<212> PRT
<213> Artificial

<220>
<223> abgeleitet von SEQ ID NO:1; interne Bezeichnung NJ70

<220>
<221> MISC_FEATURE
<222> (8)..(8)
<223> Prolin 8 des Wildtyps (SEQ ID NO:1) durch Leucin ersetzt

<220>
<221> MISC_FEATURE
<222> (132)..(132)
<223> Glycin 132 des Wildtyps (SEQ ID NO:1) durch Serin ersetzt

<220>
<221> MISC_FEATURE
<222> (134)..(134)
<223> Tryptophan 134 des Wildtyps (SEQ ID NO:1) durch Arginin ersetzt

<220>
<221> MISC_FEATURE
<222> (155)..(155)
<223> Arginin155 des Wildtyps (SEQ ID NO:1) durch Cystein ersetzt

<220>
<221> MISC_FEATURE
<222> (251)..(251)
<223> Glutaminsäure 251 des Wildtyps (SEQ ID NO:1) durch Glycin ersetzt

<220>
<221> MISC_FEATURE
<222> (311)..(311)
<223> Alanin 311 des Wildtyps (SEQ ID NO:1) durch Valin ersetzt

<220>
<221>  MISC_FEATURE
<222>  (316)..(316)
<223>  Glutaminsäure 316 des Wildtyps (SEQ ID NO:1) durch Lysin ersetzt

<400>  4

```
Met Thr Ala Ala Ser Leu Asp Leu Thr Ala Phe Ser Leu Asp Ala Ala
1               5                   10                  15

Ser Leu Ala Ala Arg Leu Asp Ala Val Phe Asp Gln Ala Leu Arg Glu
            20                  25                  30

Arg Arg Leu Val Gly Ala Val Ala Ile Val Ala Arg His Gly Glu Ile
        35                  40                  45

Leu Tyr Arg Arg Ala Gln Gly Leu Ala Asp Arg Glu Ala Gly Arg Pro
    50                  55                  60

Met Arg Glu Asp Thr Leu Phe Arg Leu Ala Ser Val Thr Lys Pro Ile
65                  70                  75                  80

Val Ala Leu Ala Val Leu Arg Leu Val Ala Arg Gly Glu Leu Ala Leu
                85                  90                  95

Asp Ala Pro Val Thr Arg Trp Leu Pro Glu Phe Arg Pro Arg Leu Ala
            100                 105                 110

Asp Gly Ser Glu Pro Leu Val Thr Ile His His Leu Leu Thr His Thr
        115                 120                 125

Ser Gly Leu Ser Tyr Arg Leu Leu Glu Gly Ala Gly Ser Val Tyr Asp
    130                 135                 140

Arg Leu Gly Ile Ser Asp Gly Ile Asp Leu Cys Asp Phe Asp Leu Asp
145                 150                 155                 160

Glu Asn Leu Arg Arg Leu Ala Ser Ala Pro Leu Ser Phe Ala Pro Gly
                165                 170                 175

Ser Gly Trp Gln Tyr Ser Leu Ala Leu Asp Val Leu Gly Ala Val Val
            180                 185                 190

Glu Arg Ala Thr Gly Gln Pro Leu Ala Ala Ala Val Asp Ala Leu Val
        195                 200                 205

Ala Gln Pro Leu Gly Met Arg Asp Cys Gly Phe Val Ser Ala Glu Pro
    210                 215                 220
```

```
Glu Arg Phe Ala Val Pro Tyr His Asp Gly Gln Pro Glu Pro Val Arg
225                 230                 235                 240

Met Arg Asp Gly Ile Glu Val Pro Leu Pro Gly Gly His Gly Ala Ala
                245                 250                 255

Val Arg Phe Ala Pro Ser Arg Val Phe Glu Pro Gly Ala Tyr Pro Ser
            260                 265                 270

Gly Gly Ala Gly Met Tyr Gly Ser Ala Asp Asp Val Leu Arg Ala Leu
        275                 280                 285

Glu Ala Ile Arg Ala Asn Pro Gly Phe Leu Pro Glu Thr Leu Ala Asp
    290                 295                 300

Ala Ala Arg Arg Asp Gln Val Gly Val Gly Ala Lys Thr Arg Gly Pro
305                 310                 315                 320

Gly Trp Gly Phe Gly Tyr Leu Ser Ala Val Leu Asp Asp Pro Ala Ala
                325                 330                 335

Ala Gly Thr Pro Gln His Ala Gly Thr Leu Gln Trp Gly Gly Val Tyr
            340                 345                 350

Gly His Ser Trp Phe Val Asp Arg Ala Leu Gly Leu Ser Val Leu Leu
        355                 360                 365

Leu Thr Asn Thr Ala Tyr Glu Gly Met Ser Gly Pro Leu Thr Ile Ala
    370                 375                 380

Leu Arg Asp Ala Val Tyr Ala Arg
385                 390
```

<210> 5
<211> 194
<212> PRT
<213> Humicola insolens

<220>
<221> MISC_FEATURE
<223> Cutinase aus Humicula insolens

<400> 5

```
Gly Thr Gly Ala Ile Glu Asn Gly Leu Glu Ser Gly Ser Ala Asn Ala
1               5                   10                  15

Cys Pro Asp Ala Ile Leu Ile Phe Ala Arg Gly Ser Thr Glu Pro Gly
            20                  25                  30
```

```
Asn Met Gly Ile Thr Val Gly Pro Ala Leu Ala Asn Gly Leu Glu Ser
        35                  40                  45

His Ile Arg Asn Ile Trp Ile Gln Gly Val Gly Gly Pro Tyr Asp Ala
    50                  55                  60

Ala Leu Ala Thr Asn Phe Leu Pro Arg Gly Thr Ser Gln Ala Asn Ile
65                  70                  75                  80

Asp Glu Gly Lys Arg Leu Phe Ala Leu Ala Asn Gln Lys Cys Pro Asn
                85                  90                  95

Thr Pro Val Val Ala Gly Gly Tyr Ser Gln Gly Ala Ala Leu Ile Ala
            100                 105                 110

Ala Ala Val Ser Glu Leu Ser Gly Ala Val Lys Glu Gln Val Lys Gly
        115                 120                 125

Val Ala Leu Phe Gly Tyr Thr Gln Asn Leu Gln Asn Arg Gly Gly Ile
    130                 135                 140

Pro Asn Tyr Pro Arg Glu Arg Thr Lys Val Phe Cys Asn Val Gly Asp
145                 150                 155                 160

Ala Val Cys Thr Gly Thr Leu Ile Ile Thr Pro Ala His Leu Ser Tyr
                165                 170                 175

Thr Ile Glu Ala Arg Gly Glu Ala Ala Arg Phe Leu Arg Asp Arg Ile
            180                 185                 190

Arg Ala


<210>  6
<211>  317
<212>  PRT
<213>  Candida antarctica


<220>
<221>  MISC_FEATURE
<223>  Lipase B aus Candida antarctica

<400>  6

Leu Pro Ser Gly Ser Asp Pro Ala Phe Ser Gln Pro Lys Ser Val Leu
1               5                   10                  15

Asp Ala Gly Leu Thr Cys Gln Gly Ala Ser Pro Ser Ser Val Ser Lys
```

```
            20                  25                  30

Pro Ile Leu Leu Val Pro Gly Thr Gly Thr Thr Gly Pro Gln Ser Phe
        35                  40                  45

Asp Ser Asn Trp Ile Pro Leu Ser Ala Gln Leu Gly Tyr Thr Pro Cys
    50                  55                  60

Trp Ile Ser Pro Pro Pro Phe Met Leu Asn Asp Thr Gln Val Asn Thr
65                  70                  75                  80

Glu Tyr Met Val Asn Ala Ile Thr Thr Leu Tyr Ala Gly Ser Gly Asn
                85                  90                  95

Asn Lys Leu Pro Val Leu Thr Trp Ser Gln Gly Gly Leu Val Ala Gln
            100                 105                 110

Trp Gly Leu Thr Phe Phe Pro Ser Ile Arg Ser Lys Val Asp Arg Leu
        115                 120                 125

Met Ala Phe Ala Pro Asp Tyr Lys Gly Thr Val Leu Ala Gly Pro Leu
    130                 135                 140

Asp Ala Leu Ala Val Ser Ala Pro Ser Val Trp Gln Gln Thr Thr Gly
145                 150                 155                 160

Ser Ala Leu Thr Thr Ala Leu Arg Asn Ala Gly Gly Leu Thr Gln Ile
                165                 170                 175

Val Pro Thr Thr Asn Leu Tyr Ser Ala Thr Asp Glu Ile Val Gln Pro
            180                 185                 190

Gln Val Ser Asn Ser Pro Leu Asp Ser Ser Tyr Leu Phe Asn Gly Lys
        195                 200                 205

Asn Val Gln Ala Gln Ala Val Cys Gly Pro Leu Phe Val Ile Asp His
    210                 215                 220

Ala Gly Ser Leu Thr Ser Gln Phe Ser Tyr Val Val Gly Arg Ser Ala
225                 230                 235                 240

Leu Arg Ser Thr Thr Gly Gln Ala Arg Ser Ala Asp Tyr Gly Ile Thr
                245                 250                 255

Asp Cys Asn Pro Leu Pro Ala Asn Asp Leu Thr Pro Glu Gln Lys Val
            260                 265                 270
```

```
Ala Ala Ala Ala Leu Leu Ala Pro Ala Ala Ala Ala Ile Val Ala Gly
        275                 280                 285

Pro Lys Gln Asn Cys Glu Pro Asp Leu Met Pro Tyr Ala Arg Pro Phe
    290                 295                 300

Ala Val Gly Lys Arg Thr Cys Ser Gly Ile Val Thr Pro
305                 310                 315

<210>  7
<211>  294
<212>  PRT
<213>  Burkholderia ambifaria

<400>  7

Met Glu Thr Asn Val Thr Ala Ala Ala Pro Ser Asp His Pro Val Phe
1               5                   10                  15

Val Leu Val His Gly Ala Trp His Gly Ala Trp Cys Tyr Ala His Val
            20                  25                  30

Ala Ala Ala Leu Ala Glu Arg Gly Tyr Leu Ser Ile Ala Arg Asp Leu
        35                  40                  45

Pro Ala His Gly Ile Asn Ala Arg Phe Pro Ala Ser Tyr Leu Glu Arg
    50                  55                  60

Pro Leu Asp Lys Asp Ala Phe Gly Ala Glu Pro Ser Pro Val Ala Asn
65                  70                  75                  80

Thr Thr Leu Asp Asp Tyr Ala Thr Gln Val Met Glu Ala Val Asp Asp
                85                  90                  95

Ala Tyr Ala Leu Gly Arg Gly Lys Val Val Leu Val Gly His Ser Met
            100                 105                 110

Gly Gly Leu Ala Ile Thr Ala Ala Ala Glu Arg Ala Pro Glu Lys Ile
        115                 120                 125

Ala Lys Ile Val Tyr Leu Ala Ala Phe Met Pro Ala Ser Gly Val Pro
    130                 135                 140

Gly Leu Asp Tyr Val Arg Ala Pro Glu Asn Lys Gly Glu Met Leu Ala
145                 150                 155                 160

Pro Leu Met Leu Ala Ser Pro Arg Val Ala Gly Ala Leu Arg Ile Asp
                165                 170                 175
```

Pro Arg Ser Gly Asp Ala Ala Tyr Arg Ala Met Ala Lys Arg Ala Leu
180 185 190

Tyr Asp Asp Ala Ala Gln Ala Asp Phe Glu Ala Met Ala Asn Leu Met
195 200 205

Thr Cys Asp Val Pro Ala Ala Pro Phe Ala Thr Ala Ile Pro Thr Thr
210 215 220

Ala Ala Arg Trp Gly Ala Ile Asp Arg His Tyr Ile Lys Cys Leu Glu
225 230 235 240

Asp Arg Val Ile Leu Pro Ala Leu Gln Gln Arg Phe Ile Asp Glu Ala
245 250 255

Asp Ala Phe Val Pro Gly Asn Pro Thr His Val His Gln Leu Asp Ser
260 265 270

Ser His Ser Pro Phe Val Ser Gln Pro Ala Val Leu Ala Gly Val Leu
275 280 285

Val Asp Ile Ala Lys Ser
290

<210> 8
<211> 311
<212> PRT
<213> Burkholderia cenocepacia

<400> 8

Met Ala Ala Gly Ser Ser Lys Met Thr Pro Phe Ser Lys Asn Thr Asp
1 5 10 15

Thr Met Glu Thr Asn Asp Asn Ala Thr Pro Gln Ser Asp His Pro Val
20 25 30

Phe Val Leu Val His Gly Ala Trp His Gly Ala Trp Cys Tyr Ala His
35 40 45

Val Ala Ala Ala Leu Ala Ala Arg Gly Tyr Leu Ser Ile Ala Arg Asp
50 55 60

Leu Pro Ala His Gly Ile His Ala Arg Phe Pro Ala Ser Tyr Leu Ala
65 70 75 80

Arg Pro Leu Asp Lys Asp Ala Phe Gly Ala Glu Pro Ser Pro Val Ala
85 90 95

```
Asn Thr Thr Leu Asp Asp Tyr Ala Thr Gln Val Met Gln Ala Val Asp
            100                 105                 110

Asp Ala Tyr Ala Leu Gly Arg Gly Lys Val Val Leu Val Gly His Ser
        115                 120                 125

Met Gly Gly Leu Ala Ile Thr Ala Ala Ala Glu Arg Ala Pro Glu Lys
    130                 135                 140

Ile Ala Lys Ile Val Tyr Leu Ala Ala Phe Met Pro Ala Ser Gly Val
145                 150                 155                 160

Pro Gly Leu Asp Tyr Val Arg Ala Pro Glu Asn Lys Gly Glu Leu Leu
                165                 170                 175

Gly Pro Leu Met Leu Ala Ser Pro Arg Val Ala Gly Ala Leu Arg Val
            180                 185                 190

Asp Pro His Ser Gly Asp Ala Ala Tyr Arg Glu Leu Met Lys Arg Ala
        195                 200                 205

Leu Tyr Glu Asp Val Pro Gln Ala Asp Phe Asp Ala Val Ala Asn Leu
    210                 215                 220

Met Ser Cys Asp Val Pro Ala Ala Pro Phe Ala Thr Ala Ile Pro Thr
225                 230                 235                 240

Thr Ala Ala Arg Trp Gly Ala Ile Asp Arg His Tyr Val Lys Cys Leu
                245                 250                 255

Gln Asp Arg Val Ile Leu Pro Ala Leu Gln Gln Arg Phe Ile Asp Glu
            260                 265                 270

Ala Asp Ala Phe Ala Pro Gly Asn Pro Thr His Val His Gln Leu Asp
        275                 280                 285

Ser Ser His Ser Pro Phe Val Ser Gln Pro Ala Val Leu Ala Gly Val
    290                 295                 300

Leu Ala Asp Ile Ala Lys Ser
305                 310
```

<210> 9
<211> 294
<212> PRT
<213> Burkhholderia cenocepacia

<400> 9

```
Met Glu Thr Asn Asp Thr Ala Thr Pro Gln Ser Asp His Pro Ile Phe
1               5                   10                  15

Val Leu Val His Gly Ala Trp His Gly Ala Trp Cys Tyr Ala His Val
            20                  25                  30

Ala Ala Ala Leu Ala Ala Arg Gly Tyr Leu Ser Ile Ala Arg Asp Leu
        35                  40                  45

Pro Ala His Gly Ile His Ala Arg Phe Pro Ala Ser Tyr Leu Ala Arg
    50                  55                  60

Pro Leu Asp Lys Asp Ala Phe Gly Ala Glu Pro Ser Pro Val Ala Asn
65                  70                  75                  80

Thr Thr Leu Asp Asp Tyr Ala Thr Gln Val Met Gln Ala Val Asp Asp
                85                  90                  95

Ala Tyr Ala Leu Gly Arg Gly Lys Val Val Leu Val Gly His Ser Met
            100                 105                 110

Gly Gly Leu Ala Ile Thr Ala Ala Ala Glu Arg Ala Pro Glu Lys Ile
        115                 120                 125

Ala Lys Ile Val Tyr Leu Ala Ala Phe Met Pro Ala Ser Gly Val Pro
    130                 135                 140

Gly Leu Asp Tyr Val Arg Ala Pro Glu Asn Lys Gly Glu Leu Leu Gly
145                 150                 155                 160

Pro Leu Met Leu Ala Ser Pro Arg Val Ala Gly Ala Leu Arg Val Asp
                165                 170                 175

Pro Arg Ser Gly Asp Ala Ala Tyr Arg Glu Leu Met Lys Arg Ala Leu
            180                 185                 190

Tyr Glu Asp Val Pro Gln Pro Asp Phe Asp Ala Val Ala Asn Leu Met
        195                 200                 205

Ser Cys Asp Val Pro Ala Ala Pro Phe Ala Thr Ala Ile Pro Thr Thr
    210                 215                 220

Ala Ala Arg Trp Gly Ala Ile Asp Arg His Tyr Ile Lys Cys Leu Gln
225                 230                 235                 240

Asp Arg Val Ile Leu Pro Ala Leu Gln Gln Arg Phe Ile Asp Glu Ala
                245                 250                 255
```

```
Asp Ala Phe Ala Pro Gly Asn Pro Thr His Val His Gln Leu Asp Ser
            260                 265                 270

Ser His Ser Pro Phe Val Ser Gln Pro Ala Val Leu Ala Gly Val Leu
        275                 280                 285

Ala Asp Ile Ala Lys Ser
    290

<210>  10
<211>  294
<212>  PRT
<213>  Burkholderia cenocepacia

<400>  10

Met Glu Thr Asn Asp Thr Ala Thr Pro Gln Ser Asp His Pro Val Phe
1               5                   10                  15

Val Leu Val His Gly Ala Trp His Gly Ala Trp Cys Tyr Ala His Val
            20                  25                  30

Ala Ala Ala Leu Ala Ala Arg Gly Tyr Leu Ser Ile Ala Arg Asp Leu
        35                  40                  45

Pro Ala His Gly Ile His Ala Arg Phe Pro Ala Ser Tyr Leu Ala Arg
    50                  55                  60

Pro Leu Asp Lys Asp Ala Phe Gly Ala Glu Pro Ser Pro Val Ala Asn
65                  70                  75                  80

Thr Thr Leu Asp Asp Tyr Ala Thr Gln Val Met Gln Ala Val Asp Asp
                85                  90                  95

Ala Tyr Ala Leu Gly Arg Gly Lys Val Val Leu Val Gly His Ser Met
            100                 105                 110

Gly Gly Leu Ala Ile Thr Ala Ala Ala Glu Arg Ala Pro Glu Lys Ile
        115                 120                 125

Ala Lys Ile Val Tyr Leu Ala Ala Phe Met Pro Ala Ser Gly Val Pro
    130                 135                 140

Gly Leu Asp Tyr Val Arg Ala Pro Glu Asn Lys Gly Glu Leu Leu Gly
145                 150                 155                 160

Pro Leu Met Leu Ala Ser Pro Arg Val Ala Gly Ala Leu Arg Val Asp
                165                 170                 175
```

```
Pro His Ser Gly Asp Ala Ala Tyr Arg Glu Leu Met Lys Arg Ala Leu
            180                 185                 190

Tyr Glu Asp Val Pro Gln Ala Asp Phe Asp Ala Val Ala Asn Leu Met
        195                 200                 205

Ser Cys Asp Val Pro Ala Ala Pro Phe Ala Thr Ala Ile Pro Thr Thr
    210                 215                 220

Ala Ala Arg Trp Gly Ala Ile Asp Arg His Tyr Ile Lys Cys Leu Gln
225                 230                 235                 240

Asp Arg Val Ile Leu Pro Ala Leu Gln Gln Arg Phe Ile Asp Glu Ala
                245                 250                 255

Asp Ala Phe Ser Pro Gly Asn Pro Thr His Val His Gln Leu Asp Ser
            260                 265                 270

Ser His Ser Pro Phe Val Ser Gln Pro Ala Val Leu Ala Gly Val Leu
        275                 280                 285

Ser Gly Ile Ala Lys Ser
    290


<210>  11
<211>  294
<212>  PRT
<213>  Burkholderia cenocepacia

<400>  11

Met Glu Thr Asn Asp Asn Ala Thr Pro Gln Ser Asp His Pro Val Phe
1               5                   10                  15

Val Leu Val His Gly Ala Trp His Gly Ala Trp Cys Tyr Ala His Val
            20                  25                  30

Ala Ala Ala Leu Ala Ala Arg Gly Tyr Leu Ser Ile Ala Arg Asp Leu
        35                  40                  45

Pro Ala His Gly Ile His Ala Arg Phe Pro Ala Ser Tyr Leu Ala Arg
    50                  55                  60

Pro Leu Asp Lys Asp Ala Phe Gly Ala Glu Pro Ser Pro Val Ala Asn
65                  70                  75                  80

Thr Thr Leu Asp Asp Tyr Ala Thr Gln Val Met Gln Ala Val Asp Asp
                85                  90                  95
```

```
Ala Tyr Ala Leu Gly Arg Gly Lys Val Val Leu Val Gly His Ser Met
            100                 105                 110

Gly Gly Leu Ala Ile Thr Ala Ala Ala Glu Arg Ala Pro Glu Lys Ile
        115                 120                 125

Ala Lys Ile Val Tyr Leu Ala Ala Phe Met Pro Ala Ser Gly Val Pro
    130                 135                 140

Gly Leu Asp Tyr Val Arg Ala Pro Glu Asn Lys Gly Glu Leu Leu Gly
145                 150                 155                 160

Pro Leu Met Leu Ala Ser Pro Arg Val Ala Gly Ala Leu Arg Val Asp
                165                 170                 175

Pro His Ser Gly Asp Ala Ala Tyr Arg Glu Leu Met Lys Arg Ala Leu
            180                 185                 190

Tyr Glu Asp Val Pro Gln Ala Asp Phe Asp Ala Val Ala Asn Leu Met
        195                 200                 205

Ser Cys Asp Val Pro Ala Ala Pro Phe Ala Thr Ala Ile Pro Thr Thr
    210                 215                 220

Ala Ala Arg Trp Gly Ala Ile Asp Arg His Tyr Val Lys Cys Leu Gln
225                 230                 235                 240

Asp Arg Val Ile Leu Pro Ala Leu Gln Gln Arg Phe Ile Asp Glu Ala
                245                 250                 255

Asp Ala Phe Ala Pro Gly Asn Pro Thr His Val His Gln Leu Asp Ser
            260                 265                 270

Ser His Ser Pro Phe Val Ser Gln Pro Ala Val Leu Ala Gly Val Leu
        275                 280                 285

Ala Asp Ile Ala Lys Ser
    290


<210>  12
<211>  294
<212>  PRT
<213>  Burkholderia cepacia

<400>  12

Met Glu Thr Asn Val Thr Ala Ala Ala Pro Ser Asp His Pro Val Phe
1               5                   10                  15
```

```
Val Leu Val His Gly Ala Trp His Gly Ala Trp Cys Tyr Ala His Val
            20                  25                  30

Ala Ala Ala Leu Ala Glu Arg Gly Tyr Leu Ser Ile Ala Arg Asp Leu
        35                  40                  45

Pro Ala His Gly Ile Asn Ala Arg Phe Pro Ala Ser Tyr Leu Glu Arg
    50                  55                  60

Pro Leu Asp Lys Asp Ala Phe Gly Ala Glu Pro Ser Pro Val Ala Asn
65                  70                  75                  80

Thr Thr Leu Asp Asp Tyr Ala Thr Gln Val Met Glu Ala Val Asp Asp
                85                  90                  95

Ala Tyr Ala Leu Gly His Gly Lys Val Val Leu Val Gly His Ser Met
            100                 105                 110

Gly Gly Leu Ala Ile Thr Ala Ala Ala Glu Arg Ala Pro Glu Lys Ile
        115                 120                 125

Ala Lys Ile Val Tyr Leu Ala Ala Phe Met Pro Ala Ser Gly Val Pro
    130                 135                 140

Gly Leu Asp Tyr Val Arg Ala Pro Glu Asn Lys Gly Glu Met Leu Ala
145                 150                 155                 160

Pro Leu Met Leu Ala Ser Pro Arg Val Ala Gly Ala Leu Arg Ile Asp
                165                 170                 175

Pro Arg Ser Gly Asp Ala Ala Tyr Arg Ala Leu Ala Lys Arg Ala Leu
            180                 185                 190

Tyr Asp Asp Ala Ala Gln Ala Asp Phe Glu Ala Met Ala Asn Leu Met
        195                 200                 205

Thr Cys Asp Val Pro Ala Ala Pro Phe Ala Thr Ala Ile Pro Thr Thr
    210                 215                 220

Ala Ala Arg Trp Gly Ala Ile Asp Arg His Tyr Ile Lys Cys Leu Glu
225                 230                 235                 240

Asp Arg Val Ile Leu Pro Ala Leu Gln Gln Arg Phe Ile Asp Glu Ala
                245                 250                 255

Asp Ala Phe Val Pro Gly Asn Pro Thr His Val His Gln Leu Asp Thr
            260                 265                 270
```

```
Ser His Ser Pro Phe Val Ser Gln Pro Ala Val Leu Ala Ala Val Leu
            275                 280                 285

Val Asp Ile Ala Lys Ser
    290


<210>  13
<211>  294
<212>  PRT
<213>  Burkholderia dolosa

<400>  13

Met Glu Thr Asn Val Thr Ala Thr Ser Pro Ala Ala His Pro Val Phe
1               5                   10                  15

Val Leu Val His Gly Ala Trp His Gly Ala Trp Cys Phe Ala His Val
            20                  25                  30

Ala Ala Ala Leu Ala Ala Arg Gly His Leu Ser Ile Ala Arg Asp Leu
        35                  40                  45

Pro Ala His Gly Ile Asn Ala Arg Phe Pro Ala Ser Tyr Leu Glu Arg
    50                  55                  60

Pro Leu Asp Lys Asp Ala Phe Gly Ala Glu Pro Ser Pro Val Ala Asn
65                  70                  75                  80

Thr Thr Leu Asp Asp Tyr Ala Ser His Val Leu Gln Ala Val Asp Asp
                85                  90                  95

Ala Tyr Ala Leu Gly Arg Gly Lys Val Val Leu Val Gly His Ser Met
            100                 105                 110

Gly Gly Leu Ala Ile Thr Ala Ala Ala Glu Arg Ala Pro Glu Lys Ile
        115                 120                 125

Ala Lys Leu Val Tyr Leu Ala Ala Phe Met Pro Ala Ser Gly Val Pro
    130                 135                 140

Gly Leu Asp Tyr Val Arg Ala Ala Glu Asn Arg Gly Glu Met Leu Gly
145                 150                 155                 160

Pro Leu Met Leu Ala Ser Pro Arg Val Ala Gly Ala Leu Arg Ile Asp
                165                 170                 175

Pro Arg Ser Gly Asp Ala Ala Tyr Arg Glu Thr Val Lys Arg Ala Leu
            180                 185                 190
```

```
Tyr Asp Asp Val Pro Gln Ala Asp Phe Asp Ala Val Ala Asn Leu Met
        195                 200                 205

Ser Cys Asp Val Pro Ala Ala Pro Phe Ala Thr Ala Ile Pro Thr Thr
    210                 215                 220

Ala Ala Arg Trp Gly Ala Ile Asp Arg His Tyr Ile Lys Cys Leu Gln
225                 230                 235                 240

Asp Arg Val Ile Leu Pro Ala Leu Gln Gln Arg Phe Ile Asp Glu Ala
                245                 250                 255

Asp Ala Phe Ala Pro Gly Asn Pro Thr His Val His Gln Leu Asp Ser
            260                 265                 270

Ser His Ser Pro Phe Val Ser Gln Pro Ala Val Leu Ala Gly Val Leu
        275                 280                 285

Ala Asp Ile Ala Lys Ser
    290

<210>  14
<211>  281
<212>  PRT
<213>  Burkholderia mallei

<400>  14

Met Pro Phe Val Leu Val His Gly Ala Trp His Gly Ala Trp Ala Tyr
1               5                   10                  15

Glu Arg Val Ile Pro Ala Leu Ala Ala His Gly His Ala Ala Val Ala
            20                  25                  30

Arg Asp Leu Pro Ala His Gly Val Asn Ala Arg Phe Pro Ala Ser Phe
        35                  40                  45

Ala Lys Arg Pro Leu Asp Ala Ala Ala Phe Ala Ser Glu Pro Ser Pro
    50                  55                  60

Val Ala Gly Thr Thr Leu Asp Asp Tyr Val Asp His Val Leu Arg Thr
65                  70                  75                  80

Val Asp Gln Ala Arg Ala Leu Gly His Glu Arg Val Val Leu Val Gly
                85                  90                  95

His Ser Met Gly Gly Leu Ala Ile Thr Met Ala Ala Glu Arg Ala Pro
            100                 105                 110
```

Glu Lys Ile Ala Lys Leu Val Tyr Leu Ala Ala Phe Met Pro Thr Ala
115 120 125

Gly Thr Lys Gly Leu Asp Tyr Val Arg Ala Pro Glu Asn Arg Gly Glu
130 135 140

Met Leu Gly Pro Leu Met Met Ala Ser Pro Lys Ala Thr Gly Ala Leu
145 150 155 160

Arg Met Asp Pro Arg Ser Asp Asp Pro Ala Tyr Arg Ala Ala Ala Lys
165 170 175

Arg Ala Leu Cys Asp Asp Ala Ser Asp Ala Asp His Ala Ala Val Gly
180 185 190

His Leu Leu Gly Cys Asp Val Pro Ala Ala Pro Phe Ala Ala Arg Ile
195 200 205

Glu Thr Thr Ala Ala Arg Trp Gly Ala Leu Glu Arg His Tyr Ile Lys
210 215 220

Cys Leu Arg Asp Lys Val Leu Leu Pro Ala Leu Gln Gln Arg Phe Ile
225 230 235 240

Asp Glu Ala Asp Ala Leu Ala Pro Gly Asn Arg Thr His Val His Thr
245 250 255

Leu Asp Ser Ser His Ser Pro Phe Ile Ala His Ala Gly Ala Val Ala
260 265 270

Asp Thr Leu Ala Ala Ile Ala Arg Gly
275 280

<210> 15
<211> 336
<212> PRT
<213> Burkholderia mallei

<400> 15

Met Gly His Asp Arg Thr Pro Arg His Arg Asp Ser Ser Leu Arg Trp
1 5 10 15

Arg Arg Leu Ala Ser Arg Pro Ala Ile Arg Thr Met Thr Asp Arg Gln
20 25 30

Glu Thr Asp Leu Pro Thr Glu Pro Arg Asp Thr Val Ala Glu Arg Arg
35 40 45

```
Glu Pro Ala Ala Ser Ser Ser Leu Pro Phe Val Leu Val His Gly Ala
    50                  55                  60

Trp His Gly Ala Trp Ala Tyr Glu Arg Val Ile Pro Ala Leu Ala Ala
65                  70                  75                  80

His Gly His Ala Ala Val Ala Arg Asp Leu Pro Ala His Gly Val Asn
                85                  90                  95

Ala Arg Phe Pro Ala Ser Phe Ala Lys Arg Pro Leu Asp Ala Ala Ala
            100                 105                 110

Phe Ala Ser Glu Pro Ser Pro Val Ala Gly Thr Thr Leu Asp Asp Tyr
        115                 120                 125

Val Asp His Val Leu Arg Thr Val Asp Gln Ala Arg Ala Leu Gly His
    130                 135                 140

Glu Arg Val Val Leu Val Gly His Ser Met Gly Gly Leu Ala Ile Thr
145                 150                 155                 160

Met Ala Ala Glu Arg Ala Pro Glu Lys Ile Ala Lys Leu Val Tyr Leu
                165                 170                 175

Ala Ala Phe Met Pro Thr Ala Gly Thr Lys Gly Leu Asp Tyr Val Arg
            180                 185                 190

Ala Pro Glu Asn Arg Gly Glu Met Leu Gly Pro Leu Met Met Ala Ser
        195                 200                 205

Pro Lys Ala Thr Gly Ala Leu Arg Met Asp Pro Arg Ser Asp Asp Pro
    210                 215                 220

Ala Tyr Arg Ala Ala Ala Lys Arg Ala Leu Cys Asp Asp Ala Ser Asp
225                 230                 235                 240

Ala Asp His Ala Ala Val Gly His Leu Leu Gly Cys Asp Val Pro Ala
                245                 250                 255

Ala Pro Phe Ala Ala Arg Ile Glu Thr Thr Ala Ala Arg Trp Gly Ala
            260                 265                 270

Leu Glu Arg His Tyr Ile Lys Cys Leu Arg Asp Lys Val Leu Leu Pro
        275                 280                 285

Ala Leu Gln Gln Arg Phe Ile Asp Glu Ala Asp Ala Leu Ala Pro Gly
```

```
    290                 295                 300

Asn Arg Thr His Val His Thr Leu Asp Ser Ser His Ser Pro Phe Ile
305                 310                 315                 320

Ala His Ala Gly Ala Val Ala Asp Thr Leu Ala Ala Ile Ala Arg Gly
                325                 330                 335
```

<210> 16
<211> 358
<212> PRT
<213> Burkholderia mallei

<400> 16

```
Met Thr Ile Pro Glu Thr Gln Ser Met Pro Gly Glu Ala Gly Pro Arg
1               5                   10                  15

Arg Ser Gly Asp Thr Val Met Gly His Asp Arg Thr Pro Arg His Arg
            20                  25                  30

Asp Ser Ser Leu Arg Trp Arg Arg Leu Ala Ser Arg Pro Ala Ile Arg
        35                  40                  45

Thr Met Thr Asp Arg Gln Glu Thr Asp Leu Pro Thr Glu Pro Arg Asp
    50                  55                  60

Thr Val Ala Glu Arg Arg Glu Pro Ala Ala Ser Ser Ser Leu Pro Phe
65                  70                  75                  80

Val Leu Val His Gly Ala Trp His Gly Ala Trp Ala Tyr Glu Arg Val
                85                  90                  95

Ile Pro Ala Leu Ala Ala His Gly His Ala Ala Val Ala Arg Asp Leu
            100                 105                 110

Pro Ala His Gly Val Asn Ala Arg Phe Pro Ala Ser Phe Ala Lys Arg
        115                 120                 125

Pro Leu Asp Ala Ala Ala Phe Ala Ser Glu Pro Ser Pro Val Ala Gly
    130                 135                 140

Thr Thr Leu Asp Asp Tyr Val Asp His Val Leu Arg Thr Val Asp Gln
145                 150                 155                 160

Ala Arg Ala Leu Gly His Glu Arg Val Val Leu Val Gly His Ser Met
                165                 170                 175

Gly Gly Leu Ala Ile Thr Met Ala Ala Glu Arg Ala Pro Glu Lys Ile
```

180 185 190

Ala Lys Leu Val Tyr Leu Ala Ala Phe Met Pro Thr Ala Gly Thr Lys
195 200 205

Gly Leu Asp Tyr Val Arg Ala Pro Glu Asn Arg Gly Glu Met Leu Gly
210 215 220

Pro Leu Met Met Ala Ser Pro Lys Ala Thr Gly Ala Leu Arg Met Asp
225 230 235 240

Pro Arg Ser Asp Asp Pro Ala Tyr Arg Ala Ala Ala Lys Arg Ala Leu
245 250 255

Cys Asp Asp Ala Ser Asp Ala Asp His Ala Ala Val Gly His Leu Leu
260 265 270

Gly Cys Asp Val Pro Ala Ala Pro Phe Ala Ala Arg Ile Glu Thr Thr
275 280 285

Ala Ala Arg Trp Gly Ala Leu Glu Arg His Tyr Ile Lys Cys Leu Arg
290 295 300

Asp Lys Val Leu Leu Pro Ala Leu Gln Gln Arg Phe Ile Asp Glu Ala
305 310 315 320

Asp Ala Leu Ala Pro Gly Asn Arg Thr His Val His Thr Leu Asp Ser
325 330 335

Ser His Ser Pro Phe Ile Ala His Ala Gly Ala Val Ala Asp Thr Leu
340 345 350

Ala Ala Ile Ala Arg Gly
355

<210> 17
<211> 309
<212> PRT
<213> Burkholderia mallei

<400> 17

Met Thr Asp Arg Gln Glu Thr Asp Leu Pro Thr Glu Pro Arg Asp Thr
1 5 10 15

Val Ala Glu Arg Arg Glu Pro Ala Ala Ser Ser Ser Leu Pro Phe Val
20 25 30

Leu Val His Gly Ala Trp His Gly Ala Trp Ala Tyr Glu Arg Val Ile

```
        35                  40                  45

Pro Ala Leu Ala Ala His Gly His Ala Ala Val Ala Arg Asp Leu Pro
    50                  55                  60

Ala His Gly Val Asn Ala Arg Phe Pro Ala Ser Phe Ala Lys Arg Pro
65                  70                  75                  80

Leu Asp Ala Ala Ala Phe Ala Ser Glu Pro Ser Pro Val Ala Gly Thr
                85                  90                  95

Thr Leu Asp Asp Tyr Val Asp His Val Leu Arg Thr Val Asp Gln Ala
            100                 105                 110

Arg Ala Leu Gly His Glu Arg Val Val Leu Val Gly His Ser Met Gly
        115                 120                 125

Gly Leu Ala Ile Thr Met Ala Ala Glu Arg Ala Pro Glu Lys Ile Ala
    130                 135                 140

Lys Leu Val Tyr Leu Ala Ala Phe Met Pro Thr Ala Gly Thr Lys Gly
145                 150                 155                 160

Leu Asp Tyr Val Arg Ala Pro Glu Asn Arg Gly Glu Met Leu Gly Pro
                165                 170                 175

Leu Met Met Ala Ser Pro Lys Ala Thr Gly Ala Leu Arg Met Asp Pro
            180                 185                 190

Arg Ser Asp Asp Pro Ala Tyr Arg Ala Ala Ala Lys Arg Ala Leu Cys
        195                 200                 205

Asp Asp Ala Ser Asp Ala Asp His Ala Ala Val Gly His Leu Leu Gly
    210                 215                 220

Cys Asp Val Pro Ala Ala Pro Phe Ala Ala Arg Ile Glu Thr Thr Ala
225                 230                 235                 240

Ala Arg Trp Gly Ala Leu Glu Arg His Tyr Ile Lys Cys Leu Arg Asp
                245                 250                 255

Lys Val Leu Leu Pro Ala Leu Gln Gln Arg Phe Ile Asp Glu Ala Asp
            260                 265                 270

Ala Leu Ala Pro Gly Asn Arg Thr His Val His Thr Leu Asp Ser Ser
        275                 280                 285
```

His Ser Pro Phe Ile Ala His Ala Gly Ala Val Ala Asp Thr Leu Ala
    290                 295                 300

Ala Ile Ala Arg Gly
305

<210> 18
<211> 301
<212> PRT
<213> Burkholderia mallei

<400> 18

Met Pro Thr Glu Pro Arg Asp Thr Val Ala Glu Arg Arg Glu Pro Ala
1               5                   10                  15

Ala Ser Ser Ser Leu Pro Phe Val Leu Val His Gly Ala Trp His Gly
            20                  25                  30

Ala Trp Ala Tyr Glu Arg Val Ile Pro Ala Leu Ala Ala His Gly His
        35                  40                  45

Ala Ala Val Ala Arg Asp Leu Pro Ala His Gly Val Asn Ala Arg Phe
    50                  55                  60

Pro Ala Ser Phe Ala Lys Arg Pro Leu Asp Ala Ala Ala Phe Ala Ser
65                  70                  75                  80

Glu Pro Ser Pro Val Ala Gly Thr Thr Leu Asp Asp Tyr Val Asp His
                85                  90                  95

Val Leu Arg Thr Val Asp Gln Ala Arg Ala Leu Gly His Glu Arg Val
            100                 105                 110

Val Leu Val Gly His Ser Met Gly Gly Leu Ala Ile Thr Met Ala Ala
        115                 120                 125

Glu Arg Ala Pro Glu Lys Ile Ala Lys Leu Val Tyr Leu Ala Ala Phe
    130                 135                 140

Met Pro Thr Ala Gly Thr Lys Gly Leu Asp Tyr Val Arg Ala Pro Glu
145                 150                 155                 160

Asn Arg Gly Glu Met Leu Gly Pro Leu Met Met Ala Ser Pro Lys Ala
                165                 170                 175

Thr Gly Ala Leu Arg Met Asp Pro Arg Ser Asp Asp Pro Ala Tyr Arg
            180                 185                 190

Ala Ala Ala Lys Arg Ala Leu Cys Asp Asp Ala Ser Asp Ala Asp His
195 200 205

Ala Ala Val Gly His Leu Leu Gly Cys Asp Val Pro Ala Ala Pro Phe
210 215 220

Ala Ala Arg Ile Glu Thr Thr Ala Ala Arg Trp Gly Ala Leu Glu Arg
225 230 235 240

His Tyr Ile Lys Cys Leu Arg Asp Lys Val Leu Leu Pro Ala Leu Gln
245 250 255

Gln Arg Phe Ile Asp Glu Ala Asp Ala Leu Ala Pro Gly Asn Arg Thr
260 265 270

His Val His Thr Leu Asp Ser Ser His Ser Pro Phe Ile Ala His Ala
275 280 285

Gly Ala Val Ala Asp Thr Leu Ala Ala Ile Ala Arg Gly
290 295 300

<210> 19
<211> 294
<212> PRT
<213> Burkholderia multivorans

<400> 19

Met Glu Thr Asn Val Thr Ala Pro Ser Gln Ser Asp His Pro Val Phe
1 5 10 15

Val Leu Val His Gly Ala Trp His Gly Ala Trp Cys Phe Ala His Val
20 25 30

Ala Ala Ala Leu Ala Ala Arg Gly His Leu Ser Ile Ala Arg Asp Leu
35 40 45

Pro Ala His Gly Leu His Ala Arg Phe Pro Val Ser Tyr His Val Arg
50 55 60

Pro Leu Asp Lys Asp Ala Phe Gly Ala Glu Pro Ser Pro Val Ala Asn
65 70 75 80

Thr Thr Leu Asp Asp Tyr Ala Thr Gln Val Met Gln Ala Val Asp Asp
85 90 95

Ala Tyr Ala Leu Gly Arg Gly Lys Val Ile Leu Val Gly His Ser Met
100 105 110

Gly Gly Leu Ala Ile Thr Ala Ala Ala Glu Arg Ala Pro Asp Lys Ile
        115                 120                 125

Ala Lys Leu Val Tyr Leu Ala Ala Phe Met Pro Ala Ala Gly Val Pro
    130                 135                 140

Gly Leu Asp Tyr Val Arg Ala Pro Glu Asn Arg Gly Glu Leu Leu Gly
145                 150                 155                 160

Pro Leu Met Leu Ala Ser Pro Arg Val Ala Gly Ala Leu Arg Ile Asp
                165                 170                 175

Pro Arg Ser Gly Asp Ala Asp Tyr Arg Ala Ala Thr Arg Arg Ala Leu
            180                 185                 190

Cys Asp Asp Val Pro Gln Ala Asp Phe Asp Ala Val Ala Asn Leu Met
        195                 200                 205

Ser Cys Asp Val Pro Ala Ala Pro Phe Ala Thr Ala Ile Pro Thr Thr
    210                 215                 220

Ala Ala Arg Trp Gly Ala Ile Asp Arg His Tyr Ile Lys Cys Leu Gln
225                 230                 235                 240

Asp Arg Val Leu Leu Pro Ala Leu Gln Gln Arg Phe Ile Asp Glu Ala
                245                 250                 255

Asp Ala Phe Ala Pro Gly Asn Pro Thr His Val His Gln Leu Asp Ser
            260                 265                 270

Ser His Ser Pro Phe Met Ser Gln Pro Ala Val Leu Ala Gly Val Leu
        275                 280                 285

Ala Asp Ile Ala Lys His
    290

<210>  20
<211>  281
<212>  PRT
<213>  Burkholderia pseudomallei

<400>  20

Met Pro Phe Val Leu Val His Gly Ala Trp His Gly Ala Trp Ala Tyr
1               5                   10                  15

Glu Arg Val Ile Pro Ala Leu Ala Ala His Gly His Ala Ala Val Ala
            20                  25                  30

```
Arg Asp Leu Pro Ala His Gly Val Asn Ala Arg Phe Pro Ala Ser Phe
        35                  40                  45

Ala Lys Arg Pro Leu Asp Ala Ala Ala Phe Ala Ser Glu Pro Ser Pro
    50                  55                  60

Val Ala Gly Thr Thr Leu Asp Asp Tyr Val Asp His Val Leu Arg Thr
65                  70                  75                  80

Val Asp Gln Ala Arg Ala Leu Gly His Glu Arg Val Val Leu Val Gly
                85                  90                  95

His Ser Met Gly Gly Leu Ala Ile Thr Met Ala Ala Glu Arg Ala Pro
            100                 105                 110

Glu Lys Ile Ala Lys Leu Val Tyr Leu Ala Ala Phe Met Pro Thr Ala
        115                 120                 125

Gly Thr Lys Gly Leu Asp Tyr Val Arg Ala Pro Glu Asn Gln Gly Glu
    130                 135                 140

Met Leu Gly Pro Leu Met Met Ala Ser Pro Lys Ala Thr Gly Ala Leu
145                 150                 155                 160

Arg Met Asp Pro Arg Ser Asp Asp Pro Ala Tyr Arg Ala Ala Ala Lys
                165                 170                 175

Arg Ala Leu Cys Asp Asp Ala Ser Asp Ala Asp His Ala Ala Val Gly
            180                 185                 190

His Leu Leu Gly Cys Asp Val Pro Ala Ala Pro Phe Ala Ala Arg Ile
        195                 200                 205

Glu Thr Thr Ala Ala Arg Trp Gly Ala Leu Glu Arg His Tyr Ile Lys
    210                 215                 220

Cys Leu Arg Asp Lys Val Leu Leu Pro Ala Leu Gln Gln Arg Phe Ile
225                 230                 235                 240

Asp Glu Ala Asp Ala Leu Ala Pro Gly Asn Arg Thr His Val His Thr
                245                 250                 255

Leu Asp Ser Ser His Ser Pro Phe Ile Ala His Ala Gly Ala Val Ala
            260                 265                 270

Asp Thr Leu Ala Ala Ile Ala Arg Gly
        275                 280
```

<210> 21
<211> 281
<212> PRT
<213> Burkholderia pseudomallei

<400> 21

```
Met Pro Phe Val Leu Val His Gly Ala Trp His Gly Ala Trp Ala Tyr
1               5                   10                  15

Glu Arg Val Ile Pro Ala Leu Ala Ala His Gly His Ala Ala Val Ala
            20                  25                  30

Arg Asp Leu Pro Ala His Gly Val Asn Ala Arg Phe Pro Ala Ser Phe
        35                  40                  45

Ala Lys Arg Pro Leu Asp Ala Ala Ala Phe Ala Ser Glu Pro Ser Pro
    50                  55                  60

Val Ala Gly Thr Thr Leu Asp Asp Tyr Val Asp His Val Leu His Thr
65                  70                  75                  80

Val Asp Gln Ala Arg Ala Leu Gly His Glu Arg Val Val Leu Val Gly
                85                  90                  95

His Ser Met Gly Gly Leu Ala Ile Thr Met Ala Ala Glu Arg Ala Pro
            100                 105                 110

Glu Lys Ile Ala Lys Leu Val Tyr Leu Ala Ala Phe Met Pro Thr Ala
        115                 120                 125

Gly Thr Lys Gly Leu Asp Tyr Val Arg Ala Pro Glu Asn Gln Gly Glu
    130                 135                 140

Met Leu Gly Pro Leu Met Met Ala Ser Pro Lys Ala Thr Gly Ala Leu
145                 150                 155                 160

Arg Met Asp Pro Arg Ser Asp Asp Pro Ala Tyr Arg Ala Ala Ala Lys
                165                 170                 175

Arg Ala Leu Cys Asp Asp Ala Ser Asp Ala Asp His Ala Ala Val Gly
            180                 185                 190

His Leu Leu Gly Cys Asp Val Pro Ala Ala Pro Phe Ala Ala Arg Ile
        195                 200                 205

Glu Thr Thr Ala Ala Arg Trp Gly Ala Leu Glu Arg His Tyr Ile Lys
    210                 215                 220
```

```
Cys Leu Arg Asp Lys Val Leu Leu Pro Ala Leu Gln Gln Arg Phe Ile
225                 230                 235                 240

Asp Glu Ala Asp Ala Leu Ala Pro Gly Asn Arg Thr His Val His Thr
                245                 250                 255

Leu Asp Ser Ser His Ser Pro Phe Ile Ala His Ala Gly Ala Val Ala
            260                 265                 270

Asp Thr Leu Ala Ala Ile Ala Arg Gly
        275                 280


<210>  22
<211>  281
<212>  PRT
<213>  Burkholderia pseudomallei

<400>  22

Met Pro Phe Val Leu Val His Gly Ala Trp His Gly Ala Trp Ala Tyr
1               5                   10                  15

Glu Arg Val Ile Pro Ala Leu Ala Ala His Gly His Ala Ala Val Ala
            20                  25                  30

Arg Asp Leu Pro Ala His Gly Val Asn Ala Arg Phe Pro Ala Ser Phe
        35                  40                  45

Ala Lys Arg Pro Leu Asp Ala Ala Ala Phe Ala Ser Glu Pro Ser Pro
    50                  55                  60

Val Ala Gly Thr Thr Leu Asp Asp Tyr Val Asp His Val Leu His Thr
65                  70                  75                  80

Val Asp Gln Ala Arg Ala Leu Gly His Glu Arg Val Val Leu Val Gly
                85                  90                  95

His Ser Met Gly Gly Leu Ala Ile Thr Met Ala Ala Glu Arg Ala Pro
            100                 105                 110

Glu Lys Ile Ala Lys Leu Val Tyr Leu Ala Ala Phe Met Pro Thr Ala
        115                 120                 125

Gly Thr Lys Gly Leu Asp Tyr Val Arg Ala Pro Glu Asn Arg Gly Glu
    130                 135                 140

Met Leu Gly Pro Leu Met Met Ala Ser Pro Lys Ala Thr Gly Ala Leu
145                 150                 155                 160
```

```
Arg Met Asp Pro Arg Ser Asp Asp Pro Ala Tyr Arg Ala Ala Ala Lys
                165                 170                 175

Arg Ala Leu Cys Asp Asp Ala Ser Asp Ala Asp His Ala Ala Val Gly
            180                 185                 190

His Leu Leu Gly Cys Asp Val Pro Ala Ala Pro Phe Ala Ala Arg Ile
        195                 200                 205

Glu Thr Thr Ala Ala Arg Trp Gly Ala Leu Glu Arg His Tyr Ile Lys
    210                 215                 220

Cys Leu Arg Asp Lys Val Leu Leu Pro Ala Leu Gln Gln Arg Phe Ile
225                 230                 235                 240

Asp Glu Ala Asp Ala Leu Ala Pro Gly Asn Arg Thr His Val His Thr
                245                 250                 255

Leu Asp Ser Ser His Ser Pro Phe Ile Ala His Ala Gly Ala Val Ala
            260                 265                 270

Asp Thr Leu Ala Ala Ile Ala Arg Gly
        275                 280


<210>  23
<211>  336
<212>  PRT
<213>  Burkholderia pseudomallei

<400>  23

Met Gly His Asp Arg Thr Pro Arg His Arg Asp Ser Ser Leu Arg Trp
1               5                   10                  15

Arg Arg Leu Ala Ser Arg Pro Ala Ile Arg Thr Met Thr Asp Arg Gln
            20                  25                  30

Glu Thr Asp Leu Pro Thr Glu Pro Arg Asp Thr Val Ala Glu Arg Arg
        35                  40                  45

Glu Pro Ala Ala Ser Ser Ser Leu Pro Phe Val Leu Val His Gly Ala
    50                  55                  60

Trp His Gly Ala Trp Ala Tyr Glu Arg Val Ile Pro Ala Leu Ala Ala
65                  70                  75                  80

His Gly His Ala Ala Val Ala Arg Asp Leu Pro Ala His Gly Val Asn
                85                  90                  95
```

Ala Arg Phe Pro Ala Ser Phe Ala Lys Arg Pro Leu Asp Ala Ala Ala
100 105 110

Phe Ala Ser Glu Pro Ser Pro Val Ala Gly Thr Thr Leu Asp Asp Tyr
115 120 125

Val Asp His Val Leu His Thr Val Asp Gln Ala Arg Ala Leu Gly His
130 135 140

Glu Arg Val Val Leu Val Gly His Ser Met Gly Gly Leu Ala Ile Thr
145 150 155 160

Met Ala Ala Glu Arg Ala Pro Glu Lys Ile Ala Lys Leu Val Tyr Leu
165 170 175

Ala Ala Phe Met Pro Thr Ala Gly Thr Lys Gly Leu Asp Tyr Val Arg
180 185 190

Ala Pro Glu Asn Gln Gly Glu Met Leu Gly Pro Leu Met Met Ala Ser
195 200 205

Pro Lys Ala Thr Gly Ala Leu Arg Met Asp Pro Arg Ser Asp Asp Pro
210 215 220

Ala Tyr Arg Ala Ala Ala Lys Arg Ala Leu Cys Asp Asp Ala Ser Asp
225 230 235 240

Ala Asp His Ala Ala Val Gly His Leu Leu Gly Cys Asp Val Pro Ala
245 250 255

Ala Pro Phe Ala Ala Arg Ile Glu Thr Thr Ala Ala Arg Trp Gly Ala
260 265 270

Leu Glu Arg His Tyr Ile Lys Cys Leu Arg Asp Lys Val Leu Leu Pro
275 280 285

Ala Leu Gln Gln Arg Phe Ile Asp Glu Ala Asp Ala Leu Ala Pro Gly
290 295 300

Asn Arg Thr His Val His Thr Leu Asp Ser Ser His Ser Pro Phe Ile
305 310 315 320

Ala His Ala Gly Ala Val Ala Asp Thr Leu Ala Ala Ile Ala Arg Gly
325 330 335

<210> 24
<211> 301

<212> PRT
<213> Burkholderia pseudomallei

<400> 24

```
Met Pro Thr Glu Pro Arg Asp Thr Val Ala Glu Arg Arg Glu Pro Ala
1               5                   10                  15

Ala Ser Ser Ser Leu Pro Phe Val Leu Val His Gly Ala Trp His Gly
            20                  25                  30

Ala Trp Ala Tyr Glu Arg Val Ile Pro Ala Leu Ala Ala His Gly His
        35                  40                  45

Ala Ala Val Ala Arg Asp Leu Pro Ala His Gly Val Asn Ala Arg Phe
    50                  55                  60

Pro Ala Ser Phe Ala Lys Arg Pro Leu Asp Ala Ala Ala Phe Ala Ser
65                  70                  75                  80

Glu Pro Ser Pro Val Ala Gly Thr Thr Leu Asp Asp Tyr Val Asp His
                85                  90                  95

Val Leu Arg Thr Val Asp Gln Ala Arg Ala Leu Gly His Glu Arg Val
            100                 105                 110

Val Leu Val Gly His Ser Met Gly Gly Leu Ala Ile Thr Met Ala Ala
        115                 120                 125

Glu Arg Ala Pro Glu Lys Ile Ala Lys Leu Val Tyr Leu Ala Ala Phe
    130                 135                 140

Met Pro Thr Ala Gly Thr Lys Gly Leu Asp Tyr Val Arg Ala Pro Glu
145                 150                 155                 160

Asn Gln Gly Glu Met Leu Gly Pro Leu Met Met Ala Ser Pro Lys Ala
                165                 170                 175

Thr Gly Ala Leu Arg Met Asp Pro Arg Ser Asp Asp Pro Ala Tyr Arg
            180                 185                 190

Ala Ala Ala Lys Arg Ala Leu Cys Asp Asp Ala Ser Asp Ala Asp His
        195                 200                 205

Ala Ala Val Gly His Leu Leu Gly Cys Asp Val Pro Ala Ala Pro Phe
    210                 215                 220

Ala Ala Arg Ile Glu Thr Thr Ala Ala Arg Trp Gly Ala Leu Glu Arg
225                 230                 235                 240
```

His Tyr Ile Lys Cys Leu Arg Asp Lys Val Leu Leu Pro Ala Leu Gln
245 250 255

Gln Arg Phe Ile Asp Glu Ala Asp Ala Leu Ala Pro Gly Asn Arg Thr
260 265 270

His Val His Thr Leu Asp Ser Ser His Ser Pro Phe Ile Ala His Ala
275 280 285

Gly Ala Val Ala Asp Thr Leu Ala Ala Ile Ala Arg Gly
290 295 300

<210> 25
<211> 301
<212> PRT
<213> Burkholderia pseudomallei

<400> 25

Met Pro Thr Glu Pro Arg Asp Thr Val Ala Glu Arg Arg Glu Pro Ala
1 5 10 15

Ala Ser Ser Ser Leu Pro Phe Val Leu Val His Gly Ala Trp His Gly
20 25 30

Ala Trp Ala Tyr Glu Arg Val Ile Pro Ala Leu Ala Ala His Gly His
35 40 45

Ala Ala Val Ala Arg Asp Leu Pro Ala His Gly Val Asn Ala Arg Phe
50 55 60

Pro Ala Ser Phe Ala Lys Arg Pro Leu Asp Ala Ala Ala Phe Ala Ser
65 70 75 80

Glu Pro Ser Pro Val Ala Gly Thr Thr Leu Asp Asp Tyr Val Asp His
85 90 95

Val Leu His Thr Val Asp Gln Ala Arg Ala Leu Gly His Glu Arg Val
100 105 110

Val Leu Val Gly His Ser Met Gly Gly Leu Ala Ile Thr Met Ala Ala
115 120 125

Glu Arg Ala Pro Glu Lys Ile Ala Lys Leu Val Tyr Leu Ala Ala Phe
130 135 140

Met Pro Thr Ala Gly Thr Lys Gly Leu Asp Tyr Val Arg Ala Pro Glu
145 150 155 160

```
Asn Arg Gly Glu Met Leu Gly Pro Leu Met Met Ala Ser Pro Lys Ala
                165                 170                 175

Thr Gly Ala Leu Arg Met Asp Pro Arg Ser Asp Asp Pro Ala Tyr Arg
            180                 185                 190

Ala Ala Ala Lys Arg Ala Leu Cys Asp Asp Ala Ser Asp Ala Asp His
        195                 200                 205

Ala Ala Val Gly His Leu Leu Gly Cys Asp Val Pro Ala Ala Pro Phe
    210                 215                 220

Ala Ala Arg Ile Glu Thr Thr Ala Ala Arg Trp Gly Ala Leu Glu Arg
225                 230                 235                 240

His Tyr Ile Lys Cys Leu Arg Asp Lys Val Leu Leu Pro Ala Leu Gln
                245                 250                 255

Gln Arg Phe Ile Asp Glu Ala Asp Ala Leu Ala Pro Gly Asn Arg Thr
            260                 265                 270

His Val His Thr Leu Asp Ser Ser His Ser Pro Phe Ile Ala His Ala
        275                 280                 285

Gly Ala Val Ala Asp Thr Leu Ala Ala Ile Ala Arg Gly
    290                 295                 300


<210>  26
<211>  301
<212>  PRT
<213>  Burkholderia pseudomallei

<400>  26

Met Pro Thr Glu Pro Arg Asp Ile Val Ala Glu Arg Arg Glu Pro Ala
1               5                   10                  15

Ala Ser Ser Ser Leu Pro Phe Val Leu Val His Gly Ala Trp His Gly
            20                  25                  30

Ala Trp Ala Tyr Glu Arg Val Ile Pro Ala Leu Ala Ala His Gly His
        35                  40                  45

Ala Ala Val Ala Arg Asp Leu Pro Ala His Gly Val Asn Ala Arg Phe
    50                  55                  60

Pro Ala Ser Phe Ala Lys Arg Pro Leu Asp Ala Ala Ala Phe Ala Ser
65                  70                  75                  80
```

Glu Pro Ser Pro Val Ala Gly Thr Thr Leu Asp Asp Tyr Val Asp His
85 90 95

Val Leu Arg Thr Val Asp Gln Ala Arg Ala Leu Gly His Glu Arg Val
100 105 110

Val Leu Val Gly His Ser Met Gly Gly Leu Ala Ile Thr Met Ala Ala
115 120 125

Glu Arg Ala Pro Glu Lys Ile Ala Lys Leu Val Tyr Leu Ala Ala Phe
130 135 140

Met Pro Thr Ala Gly Thr Lys Gly Leu Asp Tyr Val Arg Ala Pro Glu
145 150 155 160

Asn Arg Gly Glu Met Leu Gly Pro Leu Met Met Ala Ser Pro Lys Ala
165 170 175

Thr Gly Ala Leu Arg Met Asp Pro Arg Ser Asp Asp Pro Ala Tyr Arg
180 185 190

Ala Ala Ala Lys Arg Ala Leu Cys Asp Asp Ala Ser Asp Ala Asp His
195 200 205

Ala Ala Val Gly His Leu Leu Gly Cys Asp Val Pro Ala Ala Pro Phe
210 215 220

Ala Ala Arg Ile Glu Thr Thr Ala Ala Arg Trp Gly Ala Leu Glu Arg
225 230 235 240

His Tyr Ile Lys Cys Leu Arg Asp Lys Val Leu Leu Pro Ala Leu Gln
245 250 255

Gln Arg Phe Ile Asp Glu Ala Asp Ala Leu Ala Pro Gly Asn Arg Thr
260 265 270

His Val His Thr Leu Asp Ser Ser His Ser Pro Phe Ile Ala His Ala
275 280 285

Gly Ala Val Ala Asp Thr Leu Ala Ala Ile Ala Arg Gly
290 295 300

<210> 27
<211> 294
<212> PRT
<213> Burkholderia sp.

<400> 27

```
Met Glu Thr Asn Ala Ser Ala Thr Pro Gln Ser Asp His Pro Val Phe
1               5                   10                  15

Val Leu Val His Gly Ala Trp His Gly Ala Trp Ser Tyr Ala His Val
            20                  25                  30

Ala Ala Ala Leu Ala Ala Arg Gly His Leu Ser Ile Ala Arg Asp Leu
        35                  40                  45

Pro Ala His Gly Ile Asn Ala Arg Phe Pro Ala Ser Tyr Phe Ala Arg
    50                  55                  60

Pro Leu Asp Lys Asp Ala Phe Gly Ala Glu Pro Ser Pro Val Ala Asn
65                  70                  75                  80

Thr Thr Leu Asp Asp Tyr Ala Thr Gln Val Met Gln Ala Val Asp Asp
                85                  90                  95

Ala Tyr Ala Leu Gly His Gly Lys Val Val Leu Val Gly His Ser Met
            100                 105                 110

Gly Gly Leu Ala Ile Thr Ala Ala Ala Glu Arg Ala Pro Glu Lys Ile
        115                 120                 125

Ala Lys Ile Val Tyr Leu Ala Ala Phe Met Pro Ala Ser Gly Val Pro
    130                 135                 140

Gly Leu Asp Tyr Val Arg Ala Pro Glu Asn Lys Gly Glu Met Leu Gly
145                 150                 155                 160

Pro Leu Met Leu Ala Ser Pro Arg Val Ala Gly Ala Leu Arg Ile Asp
                165                 170                 175

Pro Arg Ser Gly Asp Ala Ala Tyr Arg Asp Leu Ala Lys Arg Ala Leu
            180                 185                 190

Tyr Asp Asp Val Pro Gln Ala Asp Phe Glu Ala Val Ala Asn Leu Met
        195                 200                 205

Thr Cys Asp Val Pro Ala Ala Pro Phe Ala Thr Ala Ile Pro Thr Thr
    210                 215                 220

Ala Ala Arg Trp Gly Ala Ile Asp Arg His Tyr Ile Lys Cys Leu Gln
225                 230                 235                 240

Asp Arg Val Ile Leu Pro Ala Leu Gln Gln Arg Phe Ile Asp Glu Ala
```

245 250 255

Asp Ala Phe Ala Pro Gly Asn Pro Thr His Val His Gln Leu Asp Ser
260 265 270

Ser His Ser Pro Phe Met Ser Gln Pro Ala Val Leu Ala Gly Val Leu
275 280 285

Ala Asp Ile Ala Lys Ser
290

<210> 28
<211> 301
<212> PRT
<213> Burkholderia thailandensis

<400> 28

Met Pro Thr Glu Pro Arg Asp Thr Pro Ala Asp Arg Arg Ala Pro Ala
1 5 10 15

Ala Pro Ser Pro Leu Pro Phe Val Leu Val His Gly Ala Trp His Gly
20 25 30

Ala Trp Ala Tyr Glu Arg Val Ile Pro Ala Leu Ala Ala His Gly His
35 40 45

Ala Ala Val Ala Arg Asp Leu Pro Ala His Gly Ile Asn Ala Arg Phe
50 55 60

Pro Ala Ser Phe Phe Glu Arg Pro Leu Asp Ala Ala Ala Phe Ala Ser
65 70 75 80

Glu Pro Ser Pro Val Ala Gly Thr Thr Leu Asp Asp Tyr Val Asp His
85 90 95

Val Leu His Thr Ile Asp Gln Ala Arg Ala Leu Gly His Glu Arg Val
100 105 110

Val Leu Val Gly His Ser Met Gly Gly Leu Ala Ile Thr Met Ala Ala
115 120 125

Glu Arg Ala Pro Glu Lys Ile Ala Lys Ile Val Tyr Leu Ala Ala Phe
130 135 140

Met Pro Thr Ala Gly Thr Lys Gly Leu Asp Tyr Val Arg Ala Pro Glu
145 150 155 160

Asn Gln Gly Glu Met Leu Ala Pro Leu Met Met Ala Ser Pro Lys Ala

165 170 175

Thr Gly Ala Leu Arg Met Asp Pro Arg Ser Glu Asp Pro Ala Tyr Arg
180 185 190

Ala Ala Ala Lys Arg Ala Leu Cys Asp Asp Ala Asn Asp Ala Asp His
195 200 205

Ala Ala Val Gly His Leu Leu Ser Cys Asp Thr Pro Ala Ala Pro Phe
210 215 220

Ala Ala Arg Ile Glu Thr Thr Ala Ala Arg Trp Gly Ala Ile Glu Arg
225 230 235 240

His Tyr Ile Lys Cys Leu Arg Asp Arg Val Leu Leu Pro Ala Leu Gln
245 250 255

Gln Arg Phe Ile Asp Glu Ala Asp Ala Leu Ala Pro Gly Asn Arg Thr
260 265 270

His Val His Thr Leu Asp Ser Ser His Ser Pro Phe Ile Ala Gln Pro
275 280 285

Gly Ala Leu Ala Asp Thr Leu Ala Ala Ile Ala Arg Gly
290 295 300

<210> 29
<211> 294
<212> PRT
<213> Burkholderia vietnamensis

<400> 29

Met Glu Thr Asn Val Thr Ala Ala Gln Gln Ser Asp His Pro Val Phe
1 5 10 15

Val Leu Val His Gly Ala Trp His Gly Ala Trp Ser Tyr Ala Pro Val
20 25 30

Ala Ala Ala Leu Ala Ala Arg Gly Tyr Leu Ser Ile Ala Arg Asp Leu
35 40 45

Pro Ala His Gly Ile Asn Ala Arg Phe Pro Ala Ser Tyr Leu Ala Arg
50 55 60

Pro Leu Asp Lys Asp Ala Phe Gly Ala Glu Pro Ser Pro Val Ala Asn
65 70 75 80

Thr Thr Leu Asp Asp Tyr Ala Thr Gln Val Leu Gln Ala Val Asp Asp

```
                85                  90                  95

Ala Tyr Ala Leu Gly Arg Gly Lys Val Val Leu Val Gly His Ser Met
            100                 105                 110

Gly Gly Leu Ala Ile Thr Ala Ala Ala Glu Arg Ala Pro Glu Lys Ile
        115                 120                 125

Ala Lys Leu Val Tyr Leu Ala Ala Phe Met Pro Ala Ser Gly Val Pro
    130                 135                 140

Gly Leu Asp Tyr Val Arg Ala Pro Glu Asn His Gly Asp Met Leu Gly
145                 150                 155                 160

Ala Leu Met Leu Ala Ser Pro Arg Val Ala Gly Ala Leu Arg Ile Asp
                165                 170                 175

Pro Arg Ser Gly Asp Ala Ala Tyr Arg Ala Gln Leu Lys Gln Ala Leu
            180                 185                 190

Tyr Asp Asp Val Pro Gln Ala Asp Phe Asp Ala Val Ala Asn Leu Met
        195                 200                 205

Thr Cys Asp Val Pro Ala Ala Pro Phe Ala Thr Ala Ile Pro Thr Ser
    210                 215                 220

Ala Ala Arg Trp Gly Ala Ile Asp Arg His Tyr Ile Lys Cys Leu Ala
225                 230                 235                 240

Asp Arg Val Leu Leu Pro Ala Leu Gln Gln Arg Phe Ile Asp Glu Ala
                245                 250                 255

Asp Ala Phe Ala Pro Asp Asn Pro Thr His Val His Gln Leu Asp Ser
            260                 265                 270

Ser His Ser Pro Phe Met Ser Gln Pro Ala Val Leu Ala Gly Val Leu
        275                 280                 285

Ala Asp Ile Ala Lys Ser
    290
```

<210> 30
<211> 282
<212> PRT
<213> Mycobacterium smegmatis

<400> 30

```
Met Asn Asp Ser Arg Asn Ser Gly Thr Leu Ala Val Leu Val His Gly
```

```
1               5                   10                  15

Ala Trp His Ser Ser Leu His Trp Ala Ala Ala Gln Arg Gly Leu Ala
            20                  25                  30

Arg Arg Gly Val Ala Ser Ile Ala Val Asp Leu Pro Gly His Gly Leu
        35                  40                  45

Asp Ala Pro Val Pro Ser Gly Tyr Leu Thr Ala Gly Gln Pro Gly Leu
    50                  55                  60

Glu Thr Glu Lys Ser Ala Leu Ala Asp Ile Thr Met Asp Asp Leu Ala
65                  70                  75                  80

Asp Ala Val Val Asp Ala Leu Ala Glu Val Arg Ser Arg Phe Ala Arg
                85                  90                  95

Val Leu Leu Val Ala His Ser Ala Gly Gly Gly Pro Ala Ser Leu Ala
            100                 105                 110

Ala Glu Lys Ala Pro Glu Leu Val Asp His Leu Val Tyr Leu Ala Ala
        115                 120                 125

Phe Val Pro Ala Ala Arg Pro Arg Phe Thr Asp Tyr Ile Asn Ala Pro
    130                 135                 140

Glu Asn Ala Asp Val Val Ala Leu Pro Ile Phe Ser Asp Pro Ala Asn
145                 150                 155                 160

Leu Gly Ala His Arg Leu Asn Pro Leu Ser Ser Asp Ala Ile Glu Val
                165                 170                 175

Asp Ala Ile Arg Arg Ala Phe Leu Thr Asp Met Pro Pro Asp Ala Pro
            180                 185                 190

Glu Gly Trp Arg His Leu Leu His Pro Asp Glu Pro Tyr Ala Ser Leu
        195                 200                 205

Ser Ala Pro Val Pro Val Thr Pro Arg Arg Trp Gly Arg Ile Pro Arg
    210                 215                 220

Thr Tyr Ile Arg Leu Asp Gly Asp Arg Ala Leu Ala Pro Thr Thr Gln
225                 230                 235                 240

Asn Leu Met Ile Ala Glu Ala Asp Arg Leu Thr Pro Asp Asn Pro Phe
                245                 250                 255
```

Gly Val Arg Ser Leu Pro Gly Asp His Ser Pro Met Val His Arg Pro
260 265 270

Gly Glu Leu Ala Asp Leu Leu Ala Gly Ile
275 280

<210> 31
<211> 294
<212> PRT
<213> Saccharopolyspora erythraea

<400> 31

Met Ile Asp Leu Val Thr His Thr Ser Pro Thr Phe Val Leu Val Thr
1 5 10 15

Gly Ser Gly Ala Thr Ser Phe Leu Trp Asn Pro Leu Val Thr Glu Ile
20 25 30

Val Leu Arg Gly His Arg Ala Leu Pro Val Glu Leu Pro Gly His Gly
35 40 45

Phe Asp Ala Val Phe Pro Asp Gly Tyr Gly Ser Pro Gln Asp Thr Glu
50 55 60

Val Phe Ala Gly Ala Pro Ser Pro Leu Ala Ala Leu Thr Leu Asp Asp
65 70 75 80

Tyr Ala Asp His Ala Leu Gly Val Val Arg Arg Ala Ala Glu His Gly
85 90 95

Pro Val Val Leu Val Gly His Ser Leu Gly Gly Ala Thr Val Thr Arg
100 105 110

Val Ala Asn Ala Ala Pro Glu Leu Leu Ala His Val Val Tyr Leu Cys
115 120 125

Ala Tyr Cys Cys Val Asp Glu Pro Ser Val Ala Ala Tyr Ala Pro Ser
130 135 140

Ala Pro Ala Pro Gly Ser Pro Leu Glu Arg Ala Arg Arg Ile Ala Phe
145 150 155 160

Leu Gly Asp Pro Arg Gly Thr Gly Val Met Arg Thr Asn Pro Arg Thr
165 170 175

Gly Asp Pro Asp Val Leu Ala Val Gln His Glu Leu Leu Met Ala Asp
180 185 190

Leu Asp Ala Ala Arg Val Pro Ala Val Leu Ala Tyr Ala Thr Gln Pro
195 200 205

Asp Glu Pro Leu Arg Val Val Leu Ala Asp Ala Arg Val Asp Pro Ala
210 215 220

Thr Trp Gly Arg Leu Pro Arg Thr Tyr Val Arg Thr Ser Arg Asp Glu
225 230 235 240

Val Val Pro Pro Ala Leu Gln Asp Arg Met Ile Ala Glu Ala Asp Arg
245 250 255

Arg Thr Pro Gly Asn Thr Phe Thr Ser His Thr Val Glu Ala Ser His
260 265 270

Phe Ala Pro Leu Thr His Pro Ala Glu Ile Ala Asp Ile Leu Val Gly
275 280 285

Ala Leu Arg Gln Glu Gly
290

<210> 32
<211> 289
<212> PRT
<213> Saccharopolyspora erythraea

<400> 32

Met Thr Arg Pro Ser Ser Ser Glu Thr Val Phe Leu Phe Val His Gly
1 5 10 15

Ala Trp His Ser Ser Leu His Trp Ala Glu Thr Leu Arg Ala Leu Ala
20 25 30

Leu Gln Asp Leu Ala Gly Ile Ala Ile Asp Leu Pro Gly Ser Gly Leu
35 40 45

Gly Ala Pro Val Pro Thr Gly Tyr Phe Gln Pro Gly Gln Pro Gly Leu
50 55 60

Ala Thr Glu Lys Ser Ala Leu Ala Glu Val Thr Pro Ala Gln Ile Thr
65 70 75 80

Asp Val Val Leu Asp Ala Leu Ala Ala Val Arg Thr Arg Phe Arg Asn
85 90 95

Val Val Leu Val Ala His Ser Ala Gly Gly Ala Pro Ala Ser Ala Ala
100 105 110

Ala Glu Arg Ala Pro Glu Leu Val Asp His Leu Val Tyr Leu Ser Ser
115 120 125

Phe Val Pro Ala Gly Arg Pro Arg Phe Ser Asp Tyr Ile Glu Ala Glu
130 135 140

Gln Asn Ala Gly Ala Val Arg Ile Pro Pro Leu Gly Asp Pro Ala Glu
145 150 155 160

Leu Gly Ala Phe Arg Ile Asn Pro Leu Ser Pro Asp Pro Ala Asp Ile
165 170 175

Asp Val Ile Arg Arg Ala Phe Leu Asn Asp Leu Pro Ala Thr Ala Pro
180 185 190

Asp Thr Trp Arg Gln Phe Leu His Pro Asp Gln Pro Phe Thr Ser Val
195 200 205

Thr Thr Pro Val Pro Val Thr Pro Glu Arg Trp Gly Arg Ile Ala Arg
210 215 220

Thr Phe Ile Arg Leu Thr Asp Asp Leu Ala Leu Pro Leu Val Thr Gln
225 230 235 240

Asp Leu Met Ile Asp Glu Ala Asn Gln Val Val Pro Asp Leu Pro Leu
245 250 255

Gln Val Arg Thr Leu Pro Gly Gly His Ser Pro Phe Val Thr Arg Pro
260 265 270

Ala Glu Leu Ala Ala Val Leu Ala Ser Val Ala Leu Gln Arg Thr Ala
275 280 285

Arg

<210> 33
<211> 281
<212> PRT
<213> Saccharopolyspora erythraea

<400> 33

Met Ser His Thr Phe Val Leu Val His Gly Ser Asn Cys Asn Ser Phe
1 5 10 15

Thr Trp Ala Pro Met Gln Arg Glu Leu Ala Leu Leu Gly His Arg Ser
20 25 30

```
Leu Ala Val Asp Leu Pro Gly His Gly Phe Ala Ala Gly Trp His Pro
        35                  40                  45

Ser Tyr Gln Ala Pro Gln Asp Pro Ala Ala Leu Ala Ser Ala Pro Ser
    50                  55                  60

Gly Gln Ala Gly Arg Thr Val Ala Glu Cys Val Glu His Val Val Glu
65                  70                  75                  80

Val Val Arg Arg Ala Ala Ala His Gly Pro Val Thr Leu Val Gly His
                85                  90                  95

Ser Arg Gly Gly Leu Thr Leu Thr Gly Val Gly Asn Ala Val Pro Glu
            100                 105                 110

Leu Val Asp Arg Leu Val Tyr Val Ser Ala Trp Cys Cys Val Asp Ser
        115                 120                 125

Thr Val Ala Glu Tyr Val Ala Ser Pro Glu Asn Ala Asp Ser Val Leu
    130                 135                 140

Gly Glu Ala Ala Gly Val Met Val Gly Asp Ile Ala Ala Leu Gly Ala
145                 150                 155                 160

Ile Arg Met Asn Trp Arg Thr Ala Asp Ala Gly Leu Leu Ala Thr Leu
                165                 170                 175

Lys Thr Ala Met Leu Ala Asp Gly Thr Asp Glu Glu Phe Leu Ala Phe
            180                 185                 190

Leu Asn Thr Leu Glu Pro Asp Glu Ser Leu Asp Ala Gly Gly Ile Gly
        195                 200                 205

Val Gln Ala Asp Ala Ala Thr Trp Gly Arg Val Pro Arg Ser Tyr Val
    210                 215                 220

Arg Leu Thr Arg Asp Arg Ser Leu Pro Val Ala Leu Gln Asp Arg Phe
225                 230                 235                 240

Ile Ala Glu Ala Asp Ala Leu Thr Pro Gly Asn Pro Phe Asp Val His
                245                 250                 255

Ser Val Glu Gly Ser His Val Gly Phe Leu Val His Pro Gln Glu Val
            260                 265                 270

Ala Gly Ile Leu Ala Glu Leu Ala Ala
        275                 280
```

<210> 34
<211> 308
<212> PRT
<213> Stigmatella aurantiaca

<400> 34

```
Met Asn Arg Arg Asn Leu Leu Lys Ser Ala Val Leu Thr Thr Ala Thr
1               5                   10                  15

Leu Pro Leu Ala Gly Gly Gly Ala Val Ala Leu Ala Ala Pro Arg Ala
            20                  25                  30

Ala Ser Lys Thr Phe Leu Leu Val His Gly Ala Trp His Asn Ala Leu
        35                  40                  45

His Trp Gly Arg Val Ala Gln His Leu Ser Ala Leu Gly His Arg Val
    50                  55                  60

Leu Ser Ile Asp Leu Pro Gly His Gly Leu Asn Ala Arg Phe Pro Ser
65                  70                  75                  80

Ala Tyr Ile Thr Gly Glu Trp Ala Lys Phe Ala Glu Glu Pro Ser Pro
                85                  90                  95

Gln Arg Asp Ile Ser Leu Asp Glu Cys Ala Ser Ala Val Val Asp Ala
            100                 105                 110

Leu Arg Ala Leu Lys Gly Gly Pro Arg Pro Ile Leu Val Gly His Ser
        115                 120                 125

Met Gly Gly Thr Val Ile Thr Arg Val Gly Glu Leu Ala Pro Asp Gln
    130                 135                 140

Val Gly Arg Leu Val Tyr Leu Ser Ala Tyr Cys Pro Leu Arg Leu Lys
145                 150                 155                 160

Lys Pro Ser Ala Tyr Gly Ala Leu Pro Glu Ala Lys Thr Asp Gln Gly
                165                 170                 175

Ser Thr Leu Ile Ile Gly Asn Pro Ala Ala Leu Gly Ala Val Arg Ile
            180                 185                 190

Asn Pro Arg Gly Asn Ala Ser Tyr Leu Glu Ala Leu Arg Ser Ala Tyr
        195                 200                 205

Tyr Asn Asp Val Glu Met Arg Glu Phe Leu Pro Phe Ala Leu Ala Leu
    210                 215                 220
```

```
Thr Pro Asp Leu Pro Ala Ala Leu Trp Thr Ser Glu Val Val Ala Thr
225                 230                 235                 240

Arg Glu Arg Trp Gly Arg Ile Pro Arg Ser Tyr Ile Arg Cys Thr Gln
                245                 250                 255

Asp Arg Ala Leu Met Pro Gly Leu Gln Asp Leu Met Ile Arg Glu Ala
            260                 265                 270

Asp Ala Phe Thr Pro Thr Asn Thr Phe Glu Gln Lys Thr Leu Glu Thr
        275                 280                 285

Ser His Ser Pro Phe Ala Ser Gln Pro Ala Arg Leu Ala Glu Leu Leu
    290                 295                 300

Thr Ser Leu Arg
305


<210>  35
<211>  287
<212>  PRT
<213>  Streptomyces ambofaciens

<400>  35

Met Gln Pro Thr Phe Val Leu Val His Gly Ala Phe Ala Asn Ser Phe
1               5                   10                  15

Ser Phe Ala Pro Leu Gln Ala Glu Leu Gly Leu Leu Gly His Arg Ser
            20                  25                  30

Val Ala Val Asp Leu Pro Gly His Gly Phe Ala Ala Ser Tyr Ser His
        35                  40                  45

Ala Tyr Gln Ala Pro Gln Asp Ala Glu Gly Leu Ala Thr Ala Pro Gly
    50                  55                  60

Ser Leu Lys Gly Val Thr Leu Ala Asp Asn Ala Ala His Val Ile Gly
65                  70                  75                  80

Val Leu Glu Arg Ala Lys Glu His Gly Pro Val Ile Leu Val Ala His
                85                  90                  95

Ser Arg Gly Gly Ile Thr Ala Thr Ala Val Ala Asn Ala Arg Pro Asp
            100                 105                 110

Leu Ile Asp Arg Ile Val Tyr Val Ala Ala Trp Cys Pro Val Arg Leu
        115                 120                 125
```

```
Asp Val Asn Asp Tyr Tyr Ala Glu Pro Glu Met Ala Thr Val Asp Ala
    130                 135                 140

Ala Ser Val Gly Leu Ala Met Ala Gly Asn Pro Ala Glu Leu Gly Leu
145                 150                 155                 160

Leu Arg Val Asn Phe Arg Thr Ala Asp Gln Ala Ala Leu Ala Ala Leu
                165                 170                 175

Lys Ala Ala Phe Leu Ala Asp Gly Thr Glu Glu Glu Phe Leu Thr Phe
            180                 185                 190

Leu Asn Thr Phe Gln Pro Asp Glu Asn Leu Asp Val Gly Gly Ala Ala
        195                 200                 205

Asp Arg Ala Gln Ala Ala Thr Trp Gly Arg Val Pro Lys Thr Phe Val
    210                 215                 220

Arg Leu Ala Asp Asp Ala Ser Met Pro Leu Val Met Gln Asp Arg Leu
225                 230                 235                 240

Ile Arg Glu Gly Asp Glu Leu Thr Pro Asp Asn Pro Tyr Asp Val Arg
                245                 250                 255

Thr Leu Gly Gly Ser His Leu Lys Trp Leu Val Asp Pro Ala Pro Ala
            260                 265                 270

Ala Arg Val Leu Gly Glu Leu Ser Ala Leu Thr Ala Gly Ala Ser
        275                 280                 285
```

<210> 36
<211> 327
<212> PRT
<213> Streptomyces coelicolor

<400> 36

```
Met Phe Met Thr Asp Asp Thr Asn Glu Glu Gly Glu Gly Ala Thr Arg
1               5                   10                  15

Arg Thr Ala Leu Arg Gly Leu Gly Leu Ala Val Gly Gly Met Ala Leu
            20                  25                  30

Ala Ala Gly Pro Gly Thr Ser Pro Ala Ala Ala Ala Pro Arg Arg Arg
        35                  40                  45

Leu Val Thr Tyr Val Leu Val His Gly Thr His Ser Ala Gly Ala Phe
    50                  55                  60
```

```
Trp Thr Pro Ile Ala Arg Glu Leu Gly Leu Arg Gly His Arg Val Val
65                  70                  75                  80

Met Val Asp Gln Pro Arg His Gly Ala Glu Ala Phe Val Ala Glu Ser
                85                  90                  95

Tyr Gln Arg Gln Asp Leu Ala Ala Met Ala Val Glu Pro Ser Pro Leu
            100                 105                 110

Lys Gly Leu Gly Leu Asp Asp Tyr Glu Ala Arg Val Ala Gly Ile Val
        115                 120                 125

Arg Arg Ala Ala Arg Asn Gly Pro Val Val Leu Val Gly His Ser Leu
    130                 135                 140

Gly Gly Val Ser Val Ser Arg Val Gly Glu Ala Val Pro His Leu Leu
145                 150                 155                 160

His His Ile Cys Tyr Met Ala Ala Phe Cys Pro Ser Arg Val Leu Pro
                165                 170                 175

Thr Ala Asp Ala Cys Thr Ala Ala Pro Glu Asn Ala Asn Ala Val Ser
            180                 185                 190

Pro Val Glu Leu Thr Val Gly Asp Pro Asp Arg Leu Gly Val Leu Arg
        195                 200                 205

Leu Asn Phe Arg Thr Gly Val Ser Gly Glu Leu Ala Leu Leu Lys Glu
    210                 215                 220

Met Ile Cys Ala Asp Tyr Pro Asp Ala Asp Phe Arg Arg Ile Leu Ala
225                 230                 235                 240

Gly Met Gln Thr Asp Glu Pro Val Ala Ala Tyr Ala Gly Arg Ala Val
                245                 250                 255

Gly Arg Ala Gly Arg Trp Gly Arg Ile Pro Arg Thr Tyr Leu Arg Phe
            260                 265                 270

Gly Arg Asp Arg Thr Ile Ala Thr Ala Leu Gln Asp Arg Val Ile Ala
        275                 280                 285

Glu Ala Asp Ala Ala Thr Pro Gly Asn Gly Phe Arg Val His Asp Phe
    290                 295                 300

Pro Glu Ala Ser His Val Gly Pro Leu Asp Pro Thr Pro Val Ala Asp
305                 310                 315                 320
```

```
Val Leu Asp Arg Leu Ala Gly
                325


<210>  37
<211>  388
<212>  PRT
<213>  Artificial

<220>
<223>  Deletionsmutante EstB_Short4; abgeleitet von SEQ ID NO:3


<220>
<221>  MISC_FEATURE
<223>  entspricht EstB_N27, wobei die Aminosären 317-319 (RGP) von
       EstB_N27 (SEQ ID NO:3) deletiert wurden

<400>  37

Met Thr Ala Ala Ser Leu Asp Pro Thr Ala Phe Ser Leu Asp Ala Ala
1               5                   10                  15


Leu Leu Ala Ala Arg Leu Asp Ala Val Phe Asp Gln Ala Leu Arg Glu
            20                  25                  30


Arg Arg Leu Val Gly Ala Val Ala Ile Val Ala Arg His Gly Glu Ile
        35                  40                  45


Leu Tyr Arg Arg Ala Gln Gly Leu Ala Asp Arg Glu Ala Gly Arg Pro
    50                  55                  60


Met Arg Glu Asp Thr Leu Phe Arg Leu Ala Ser Val Thr Lys Pro Ile
65                  70                  75                  80


Val Ala Leu Ala Val Leu Arg Leu Val Ala Arg Gly Glu Leu Ala Leu
                85                  90                  95


Asp Ala Pro Val Thr Arg Trp Leu Pro Glu Phe Arg Pro Arg Leu Ala
            100                 105                 110


Asp Gly Ser Glu Pro Leu Val Thr Ile His His Leu Leu Thr His Thr
        115                 120                 125


Ser Gly Leu Ser Tyr Trp Leu Leu Glu Gly Ala Gly Ser Val Tyr Asp
    130                 135                 140


Arg Leu Gly Ile Ser Asp Gly Ile Asp Leu Arg Asp Phe Asp Leu Asp
145                 150                 155                 160


Glu Asn Leu Arg Arg Leu Ala Ser Ala Pro Leu Ser Phe Ala Pro Gly
```

```
                165                 170                 175

Ser Gly Trp Gln Tyr Ser Leu Ala Leu Asp Val Leu Gly Ala Val Val
            180                 185                 190

Glu Arg Ala Thr Gly Gln Pro Leu Ala Ala Ala Val Asp Ala Leu Val
        195                 200                 205

Ala Gln Pro Leu Gly Met Arg Asp Cys Gly Phe Val Ser Ala Glu Pro
    210                 215                 220

Glu Arg Phe Ala Val Pro Tyr His Asp Gly Gln Pro Glu Pro Val Arg
225                 230                 235                 240

Met Arg Asp Gly Ile Glu Val Pro Leu Pro Gly Gly His Gly Ala Ala
                245                 250                 255

Val Arg Phe Ala Pro Ser Arg Val Phe Glu Pro Gly Ala Tyr Pro Ser
            260                 265                 270

Gly Gly Ala Gly Met Tyr Gly Ser Ala Asp Asp Val Leu Arg Ala Leu
        275                 280                 285

Glu Ala Ile Arg Ala Asn Pro Gly Phe Leu Pro Glu Thr Leu Ala Asp
    290                 295                 300

Ala Ala Arg Arg Asp Gln Val Gly Val Gly Ala Lys Thr Gly Trp Gly
305                 310                 315                 320

Phe Gly Tyr Leu Ser Ala Val Leu Asp Asp Pro Ala Ala Ala Gly Thr
                325                 330                 335

Pro Gln His Ala Gly Thr Leu Gln Trp Gly Gly Val Tyr Gly His Ser
            340                 345                 350

Trp Phe Val Asp Arg Ala Leu Gly Leu Ser Val Leu Leu Leu Thr Asn
        355                 360                 365

Thr Ala Tyr Glu Gly Met Ser Gly Pro Leu Thr Ile Ala Leu Arg Asp
    370                 375                 380

Ala Val Tyr Ala
385
```

<210> 38
<211> 388
<212> PRT
<213> Artificial

<220>
<223> EstB_N27 Short 5; abgeleitet von SEQ ID NO:3

<220>
<221> MISC_FEATURE
<223> Aminosäuren 248-255 (PLPGGHGA) von SEQ ID NO:3 wurden ersetzt durch SLGTT

<400> 38

```
Met Thr Ala Ala Ser Leu Asp Pro Thr Ala Phe Ser Leu Asp Ala Ala
1               5                   10                  15

Leu Leu Ala Ala Arg Leu Asp Ala Val Phe Asp Gln Ala Leu Arg Glu
            20                  25                  30

Arg Arg Leu Val Gly Ala Val Ala Ile Val Ala Arg His Gly Glu Ile
        35                  40                  45

Leu Tyr Arg Arg Ala Gln Gly Leu Ala Asp Arg Glu Ala Gly Arg Pro
    50                  55                  60

Met Arg Glu Asp Thr Leu Phe Arg Leu Ala Ser Val Thr Lys Pro Ile
65                  70                  75                  80

Val Ala Leu Ala Val Leu Arg Leu Val Ala Arg Gly Glu Leu Ala Leu
                85                  90                  95

Asp Ala Pro Val Thr Arg Trp Leu Pro Glu Phe Arg Pro Arg Leu Ala
            100                 105                 110

Asp Gly Ser Glu Pro Leu Val Thr Ile His His Leu Leu Thr His Thr
        115                 120                 125

Ser Gly Leu Ser Tyr Trp Leu Leu Glu Gly Ala Gly Ser Val Tyr Asp
    130                 135                 140

Arg Leu Gly Ile Ser Asp Gly Ile Asp Leu Arg Asp Phe Asp Leu Asp
145                 150                 155                 160

Glu Asn Leu Arg Arg Leu Ala Ser Ala Pro Leu Ser Phe Ala Pro Gly
                165                 170                 175

Ser Gly Trp Gln Tyr Ser Leu Ala Leu Asp Val Leu Gly Ala Val Val
            180                 185                 190

Glu Arg Ala Thr Gly Gln Pro Leu Ala Ala Ala Val Asp Ala Leu Val
        195                 200                 205
```

```
Ala Gln Pro Leu Gly Met Arg Asp Cys Gly Phe Val Ser Ala Glu Pro
    210                 215                 220

Glu Arg Phe Ala Val Pro Tyr His Asp Gly Gln Pro Glu Pro Val Arg
225                 230                 235                 240

Met Arg Asp Gly Ile Glu Val Ser Leu Gly Thr Thr Ala Val Arg Phe
                245                 250                 255

Ala Pro Ser Arg Val Phe Glu Pro Gly Ala Tyr Pro Ser Gly Gly Ala
            260                 265                 270

Gly Met Tyr Gly Ser Ala Asp Asp Val Leu Arg Ala Leu Glu Ala Ile
        275                 280                 285

Arg Ala Asn Pro Gly Phe Leu Pro Glu Thr Leu Ala Asp Ala Ala Arg
    290                 295                 300

Arg Asp Gln Val Gly Val Gly Ala Lys Thr Arg Gly Pro Gly Trp Gly
305                 310                 315                 320

Phe Gly Tyr Leu Ser Ala Val Leu Asp Asp Pro Ala Ala Ala Gly Thr
                325                 330                 335

Pro Gln His Ala Gly Thr Leu Gln Trp Gly Gly Val Tyr Gly His Ser
            340                 345                 350

Trp Phe Val Asp Arg Ala Leu Gly Leu Ser Val Leu Leu Leu Thr Asn
        355                 360                 365

Thr Ala Tyr Glu Gly Met Ser Gly Pro Leu Thr Ile Ala Leu Arg Asp
    370                 375                 380

Ala Val Tyr Ala
385


<210>  39
<211>  35
<212>  DNA
<213>  Artificial

<220>
<223>  PCR-Primer


<220>
<221>  misc_feature
<223>  führt den Aminosäureaustausch Phe138Val ein

<400>  39
```

gctgctcttg tatatcttgc tgcggtcatg cctgc 35

<210> 40
<211> 35
<212> DNA
<213> Artificial

<220>
<223> PCR-Primer

<220>
<221> misc_feature
<223> führt den Aminosäureaustausch Phe138Ala ein

<400> 40
gctgctcttg tatatcttgc tgcggccatg cctgc 35

<210> 41
<211> 35
<212> DNA
<213> Artificial

<220>
<223> PCR-Primer

<220>
<221> misc_feature
<223> führt den Aminosäureaustausch Glu154Ala ein

<400> 41
gtaagagcac cagcaaacca tggcgaaatg ctggc 35

<210> 42
<211> 27
<212> DNA
<213> Artificial

<220>
<223> PCR-Primer

<220>
<221> misc_feature
<223> führt den Aminosäureaustausch Leu163Ala ein

<400> 42
ctggcctcgg cgatctgcgc cagccct 27

<210> 43
<211> 25
<212> DNA
<213> Artificial

<220>
<223> PCR-Primer

<220>
<221> misc_feature
<223> führt den Aminosäureaustausch Leu189Ala ein

<400> 43
cggcctatct cgccacggcg aagca 25

<210> 44
<211> 35
<212> DNA
<213> Artificial

<220>
<223> PCR-Primer

<220>
<221> misc_feature
<223> führt die Aminosäureaustausche Leu189AlaLeu193Ala ein

<400> 44
gccacggcga agcaggcggc gttcgaggat gttga 35

<210> 45
<211> 25
<212> DNA
<213> Artificial

<220>
<223> PCR-Primer

<220>
<221> misc_feature
<223> führt den Aminosäureaustausch Leu193Ala ein

<400> 45
gcaggcggcg ttcgaggatg ttgac 25

<210> 46
<211> 30
<212> DNA
<213> Artificial

<220>
<223> PCR-Primer

<220>
<221> misc_feature
<223> führt den Aminosäureaustausch Val150Ala ein

<400> 46
gtacctggtc ttgattacgc gagagctcct 30

<210> 47
<211> 25
<212> DNA
<213> Artificial

<220>
<223> PCR-Primer

<220>
<221> misc_feature
<223> führt den Aminosäureaustausch Thr188Ser ein

<400> 47
gcctatctcg cctcgctgaa gcagg 25

<210> 48
<211> 25
<212> DNA
<213> Artificial

<220>
<223> PCR-Primer

<220>
<221> misc_feature
<223> führt den Aminosäureaustausch Leu160Ala ein

<400> 48
cgaaatggcg gcctcgctga tctgc 25

<210> 49
<211> 35
<212> DNA
<213> Artificial

<220>
<223> PCR-Primer

<220>
<221> misc_feature
<223> führt die Aminosäureaustausche Thr188AlaLeu189AlaLeu193Ala ein

<400> 49
tatctcgcct cggcgaagca ggcggcgttc gagga 35

<210> 50
<211> 1179
<212> DNA
<213> Burkholderia gladioli

<220>
<221> misc_feature
<223> für SEQ ID NO:1 kodierende Sequenz

<400> 50
atgaccgctg cctcgctcga cccgaccgct ttctccctcg atgccgcctc gctggccgcg 60

cgtctcgatg ccgtgttcga ccaggcgctg cgcgaacggc gcctggtcgg cgcggtggcg 120

atcgtcgcgc ggcacggcga gatcctgtat cgccgcgccc agggcctggc cgaccgagag 180

```
gcaggtcggc cgatgcgcga ggacacgctg ttccggctcg cttcggtgac caagccgatc    240

gtcgcgctgg cggtgctgcg gctggtggcg cgcggcgaac tcgcgctcga cgcgccggtc    300

acgcgctggt tgcccgaatt ccggccgcgg ctggccgacg gcagcgagcc gctcgtcacg    360

attcaccacc tgctcacgca cacgtcgggg ctcggctact ggctgctcga gggcgccggc    420

tccgtgtacg accggctcgg catctcggac ggcatcgacc tgcgcgactt cgatctcgac    480

gaaaacctgc gccgcctcgc ctcggcgccg ctgtccttcg cgccgggcag cggctggcag    540

tattcgctgg cgctcgacgt gctcggcgcg gtggtcgagc gcgccaccgg gcagccgctg    600

gccgcggcgg tggacgcgtt ggtcgcccag ccgctcggca tgcgcgattg cggtttcgtc    660

tcggcggagc ccgagcgctt cgccgtgcct taccacgacg gccagccgga gccggtgcgc    720

atgcgcgacg gcatcgaggt gccgctgccg gaaggccacg gcgcggccgt gcgtttcgcg    780

ccctcccgcg tgttcgagcc gggcgcctat ccctcgggcg gcgccggcat gtacggctcg    840

gccgacgacg tcctgcgcgc gctcgaggcg atccgcgcca atcccggttt cctgcccgag    900

acgctggccg acgcggcgcg ccgcgaccag gccggagtcg gcgccgagac gcgcggcccc    960

ggctggggct tcggctacct gagcgcggtg ctcgacgatc cggccgcggc cggcaccccg   1020

cagcacgccg ggacgctgca atggggcggc gtctatggcc attcctggtt cgtcgaccgc   1080

gcgctgggac tcagcgtgct gctgctcacc aataccgcct acgaaggcat gtcgggcccg   1140

ctgacgatcg ccttgcgcga cgccgtctac gcgcgctga                          1179


<210>  51
<211>  897
<212>  DNA
<213>  Burkholderia gladioli


<220>
<221>  misc_feature
<223>  für SEQ ID NO:2 kodierende Sequenz

<400>  51
atgaaccatc ccgatatcga cactcattca cgaaatgccg ccgcgccttt accctttgtg     60

ttggtccatg gagcctggca tggagcctgg gcctatgaac gattaggagc ggcgttggcg    120

gcgcgtggac atgccagtgt cgcgcatgat ttacccgcgc atggaattaa tgcccgatat    180

ccggccgcgt tttggcaagg agatgcgcaa gcgttagcgc aagaaccgtc tccggtcgcg    240

gccacaactt tagacgatta tacaggacaa gtgttacgag cgatcgatgc tgcttgtgct    300

cttggtcacc cgagagtagt gcttgtaggt catagtatgg gtggtgtagc tatcacagcc    360

gcggctgaaa gagctccgga aagaatcgct gctcttgtat atcttgctgc gttcatgcct    420

gctagtggtg tacctggtct tgattacgtg agagctcctg aaaaccatgg cgaaatgctg    480
```

```
gcctcgctga tctgcgccag ccctcgcgcg atcggcgcgc tgcgcatcaa cccggccagc    540

cgcgacgcgg cctatctcgc cacgctgaag caggcgctgt tcgaggatgt tgacgaggcg    600

acgttccgcg ccgtgacacg gctgatgtcc tcggacgtgc cgaccgcgcc attcgccacg    660

ccgatcgcga ccacggccga gcgctggggc tcgatcgcgc gccactacgt gacctgcgcc    720

gaggatcgcg tgatcctgcc ggcgctgcag cggcgcttca tcgccgaggc cgacgccttc    780

ctgcccgagc ggccgacgcg cgtccacgca ctcgacagca gccattcgcc gttcctgtcc    840

cagcccgaca cgctcgccga gttgctgacg ggcatcgcgc gcaacacggc gatctga       897


<210>  52
<211>  585
<212>  DNA
<213>  Humicola insolens


<220>
<221>  misc_feature
<223>  für Cutinase gemäß SEQ ID NO:5 kodierende Sequenz

<400>  52
ggtaccggcg caatcgaaaa cggcctggaa tccggctccg caaacgcatg cccagatgca     60

atcctgatct tcgcacgcgg ctccaccgaa ccaggcaaca tgggcatcac cgtgggccca    120

gcactggcaa acggcctgga atcccacatc cgcaacatct ggatccaggg cgtgggcggc    180

ccatacgatg cagcactggc aaccaacttc ctgccacgcg gcacctccca ggcaaacatc    240

gatgaaggca agcgcctgtt cgcactggca aaccagaagt gcccaaacac cccagtggtg    300

gcaggcggct actcccaggg cgcagcactg atcgcagcag cagtgtccga actgtccggc    360

gcagtgaagg aacaggtgaa gggcgtggca ctgttcggct acacccagaa cctgcagaac    420

cgcggcggca tcccaaacta cccacgcgaa cgcaccaagg tgttctgcaa cgtgggcgat    480

gcagtgtgca ccggcaccct gatcatcacc ccagcacacc tgtcctacac catcgaagca    540

cgcggcgaag cagcacgctt cctgcgcgat cgcatccgcg cataa                    585


<210>  53
<211>  954
<212>  DNA
<213>  Candida antarctica


<220>
<221>  misc_feature
<223>  kodiert für Lipase B gemäß SEQ ID NO:6

<400>  53
ctaccttccg gttcggaccc tgccttttcg cagcccaagt cggtgctcga tgcgggtctg     60

acctgccagg gtgcttcgcc atcctcggtc tccaaaccca tccttctcgt ccccggaacc    120

ggcaccacag gtccacagtc gttcgactcg aactggatcc ccctctctgc gcagctgggt    180
```

```
tacacaccct gctggatctc acccccgccg ttcatgctca acgacaccca ggtcaacacg    240

gagtacatgg tcaacgccat caccacgctc tacgctggtt cgggcaacaa caagcttccc    300

gtgctcacct ggtcccaggg tggtctggtt gcacagtggg gtctgacctt cttccccagt    360

atcaggtcca aggtcgatcg acttatggcc tttgcgcccg actacaaggg caccgtcctc    420

gccggccctc tcgatgcact cgcggttagt gcaccctccg tatggcagca aaccaccggt    480

tcggcactca ctaccgcact ccgaaacgca ggtggtctga cccagatcgt gcccaccacc    540

aacctctact cggcgaccga cgagatcgtt cagcctcagg tgtccaactc gccactcgac    600

tcatcctacc tcttcaacgg aaagaacgtc caggcacagg ctgtgtgtgg gccgctgttc    660

gtcatcgacc atgcaggctc gctcacctcg cagttctcct acgtcgtcgg tcgatccgcc    720

ctgcgctcca ccacgggcca ggctcgtagt gcagactatg gcattacgga ctgcaaccct    780

cttcccgcca atgatctgac tcccgagcaa aaggtcgccg cggctgcgct cctggcgccg    840

gcggctgcag ccatcgtggc gggtccaaag cagaactgcg agcccgacct catgccctac    900

gcccgcccct ttgcagtagg caaaaggacc tgctccggca tcgtcacccc ctga          954
```

```
<210>  54
<211>  1179
<212>  DNA
<213>  Artificial

<220>
<223>  Gene coding for modified EstB_N27 (according to SEQ ID NO:3)


<220>
<221>  misc_feature
<223>  kodiert für N27 gemäß SEQ ID NO:3

<220>
<221>  misc_feature
<222>  (1)..(42)
<223>  kodiert für Aminosäuren, die im aktiven Protein fehlen

<220>
<221>  misc_feature
<222>  (49)..(51)
<223>  Codon TCG des Wildtyps (SEQ ID NO:50) zu TTG mutiert, was zu
       Ser17Leu führt

<220>
<221>  misc_feature
<222>  (60)..(60)
<223>  stiller Nukleotidaustausch G to A

<220>
<221>  misc_feature
<222>  (394)..(396)
<223>  Codon GGC des Wildtyps (SEQ ID NO:50) zu AGC mutiert, was zu
       Gly132Ser führt
```

<220>
<221> misc_feature
<222> (513)..(513)
<223> stiller Nukleotidaustausch G to A

<220>
<221> misc_feature
<222> (751)..(753)
<223> Codon GAA des Wildtyps (SEQ ID NO:50) zu GAA, was zu Glu251Gly führt

<220>
<221> misc_feature
<222> (931)..(933)
<223> Codon GCC des Wildtyps (SEQ ID NO:50) zu GTC mutiert, was zu Ala311Val führt

<220>
<221> misc_feature
<222> (946)..(948)
<223> Codon GAG des Wildtyps (SEQ ID NO:50) zu AAG mutiert, was zu Glu316Lys führt

<400> 54
```
atgaccgctg cctcgctcga cccgaccgct ttctccctcg atgccgcctt gctggccgca     60

cgtctcgatg ccgtgttcga ccaggcgctg cgcgaacggc gcctggtcgg cgcggtggcg    120

atcgtcgcgc ggcacggcga gatcctgtat cgccgcgccc agggcctggc cgaccgagag    180

gcaggtcggc cgatgcgcga ggacacgctg ttccggctcg cttcggtgac caagccgatc    240

gtcgcgctgg cggtgctgcg actggtggcg cgcggcgaac tcgcgctcga cgcgccggtc    300

acgcgctggt tgcccgaatt ccggccgcgg ctggccgacg gcagcgagcc gctcgtcacg    360

attcaccacc tgctcacgca cacgtcgggg ctcagctact ggctgctcga gggcgccggc    420

tccgtgtacg accggctcgg catctcggac ggcatcgacc tgcgcgactt cgatctcgac    480

gaaaacctgc gccgcctcgc ctcggcgccg ctatccttcg cgccgggcag cggctggcag    540

tattcgctgg cgctcgacgt gctcggcgcg gtggtcgagc gcgccaccgg gcagccgctg    600

gcggcggcgg tggacgcgtt ggtcgcccag ccgctcggca tgcgcgattg cggtttcgtc    660

tcggcggagc ccgagcgctt cgccgtgcct taccacgacg gccagccgga gccggtgcgc    720

atgcgcgacg gcatcgaggt gccgctgccg ggaggccacg gcgcggccgt gcgtttcgcg    780

ccctcccgcg tgttcgagcc gggcgcctat ccctcgggcg gcgccggcat gtacggctcg    840

gccgacgacg tcctgcgcgc gctcgaggcg atccgcgcca atcccggttt cctgcccgag    900

acgctggccg acgcggcgcg ccgcgaccag gtcggagtcg gcgccaagac gcgcggcccc    960

ggctggggct tcggctacct gagcgcggtg ctcgacgatc cggccgcggc cggcaccccg   1020

cagcacgccg ggacgctgca atggggcggc gtctatggcc attcctggtt cgtcgaccgc   1080

gcgctgggac tcagcgtgct gctgctcacc aataccgcct acgaaggcat gtcgggcccg   1140

ctgacgatcg ccttgcgcga cgccgtctac gcgcgctga                          1179
```

<210> 55
<211> 1179
<212> DNA
<213> Artificial

<220>
<223> DNA coding for EstB_NJ70 (SE ID NO:4)

<220>
<221> misc_feature
<223> kodiert für NJ_70 gemäß SEQ ID NO:4

<400> 55
atgaccgctg cctcactcga cctgaccgcc ttctccctcg atgccgcctc gctggccgcg 60

cgtctcgatg ccgtgttcga ccaggcgctg cgcgaacggc gcctggtcgg cgcggtggcg 120

atcgtcgcgc ggcacggcga gatcctgtat cgccgcgccc agggcctggc cgaccgagag 180

gcaggtcggc cgatgcgcga ggacacgctg ttccggctcg cttcggtgac caagccgatc 240

gtcgcgctgg cggtgctgcg gctggtggcg cgcggcgagc tcgcgctcga cgcgccggtc 300

acgcgctggt tgcccgaatt ccggccgcgg ctggccgacg gcagcgagcc gctcgtcacg 360

attcaccacc tgctcacgca cacgtcgggg ctcagctaca ggctgctcga gggcgccggc 420

tccgtgtacg accggctcgg catctcggac ggcatcgacc tgtgcgactt cgatctcgac 480

gaaaacctgc gccgcctcgc ctcggcgccg ctgtccttcg cgccgggcag cggctggcag 540

tattcgctgg cgctcgacgt gctcggcgcg gtggtcgagc gcgccaccgg gcagccgctg 600

gccgcggcgg tggacgcgtt ggtcgcccag ccgctcggca tgcgcgattg cggtttcgtc 660

tcggcggagc ccgagcgctt cgccgtgcct taccacgacg gccagcctga gccggtgcgc 720

atgcgcgacg gcatcgaggt gccgctgccg ggaggccacg gcgcggccgt gcgtttcgcg 780

ccctcccgcg tgttcgagcc gggcgcctat ccctcgggcg gcgccggcat gtacggctcg 840

gccgacgacg tcctgcgcgc gctcgaggcg atccgcgcca atcccggttt cctgcccgag 900

acgctggccg acgcggcgcg ccgcgaccag gtcggagtcg gcgccaagac gcgcggcccc 960

ggctggggct tcggctactt gagcgcggtg ctcgacgatc cggccgcggc cggcaccccg 1020

cagcacgccg ggacgctgca atggggcggc gtctatggcc attcctggtt cgtcgaccgc 1080

gcgctgggac tcagcgtgct gctgctcacc aataccgcct acgaaggcat gtcgggcccg 1140

ctgacgatcg ccttgcgcga cgccgtctac gcgcgctga 1179

<210> 56
<211> 1170
<212> DNA
<213> Artificial

<220>

<223>  für EstB_N27_Short4 (SEQ ID NO:37) kodierende Sequenz

<220>
<221>  misc_feature
<222>  (951)..(952)
<223>  Nukleotide CGCGGCCCC der Positionen 952 bis 960 von N27 (SEQ ID NO:54) sind zwischen den Nukleotiden 952 and 952 of N27_Short4 (SEQ ID NO:56) deletiert

<400>  56
```
atgaccgctg cctcgctcga cccgaccgct ttctccctcg atgccgcctt gctggccgca      60

cgtctcgatg ccgtgttcga ccaggcgctg cgcgaacggc gcctggtcgg cgcggtggcg     120

atcgtcgcgc ggcacggcga gatcctgtat cgccgcgccc agggcctggc cgaccgagag     180

gcaggtcggc cgatgcgcga ggacacgctg ttccggctcg cttcggtgac caagccgatc     240

gtcgcgctgg cggtgctgcg actggtggcg cgcggcgaac tcgcgctcga cgcgccggtc     300

acgcgctggt tgcccgaatt ccggccgcgg ctggccgacg gcagcgagcc gctcgtcacg     360

attcaccacc tgctcacgca cacgtcgggg ctcagctact ggctgctcga gggcgccggc     420

tccgtgtacg accggctcgg catctcggac ggcatcgacc tgcgcgactt cgatctcgac     480

gaaaacctgc gccgcctcgc ctcggcgccg ctatccttcg cgccgggcag cggctggcag     540

tattcgctgg cgctcgacgt gctcggcgcg gtggtcgagc gcgccaccgg gcagccgctg     600

gcggcggcgg tggacgcgtt ggtcgcccag ccgctcggca tgcgcgattg cggtttcgtc     660

tcggcggagc ccgagcgctt cgccgtgcct taccacgacg gccagccgga gccggtgcgc     720

atgcgcgacg gcatcgaggt gccgctgccg ggaggccacg gcgcggccgt gcgtttcgcg     780

ccctcccgcg tgttcgagcc gggcgcctat ccctcgggcg gcgccggcat gtacggctcg     840

gccgacgacg tcctgcgcgc gctcgaggcg atccgcgcca atcccggttt cctgcccgag     900

acgctggccg acgcggcgcg ccgcgaccag gtcggagtcg gcgccaagac gggctggggc     960

ttcggctacc tgagcgcggt gctcgacgat ccggccgcgg ccggcacccc gcagcacgcc    1020

gggacgctgc aatggggcgg cgtctatggc cattcctggt tcgtcgaccg cgcgctggga    1080

ctcagcgtgc tgctgctcac caataccgcc tacgaaggca tgtcgggccc gctgacgatc    1140

gccttgcgcg acgccgtcta cgcgcgctga                                     1170
```

<210>  57
<211>  1170
<212>  DNA
<213>  Artificial

<220>
<223>  kodiert für EstB_N27_Short5 (SEQ ID NO:38)

<220>
<221>  misc_feature

<223> kodiert für EstB_N27_Short5

<220>
<221> misc_feature
<222> (746)..(759)
<223> Sequenz ctgGGGACGACGgcc der Nukleotide 746 bis 759 von N27_Short 5 entspricht den Nukleotiden 745 bis 768 von EstB-wt (SEQ ID NO:50) , d.h. ctgCCGGAAGGCCACGGCGCGgcc

<400> 57
atgaccgctg cctcgctcga cccgaccgct ttctccctcg atgccgcctt gctggccgca 60

cgtctcgatg ccgtgttcga ccaggcgctg cgcgaacggc gcctggtcgg cgcggtggcg 120

atcgtcgcgc ggcacggcga gatcctgtat cgccgcgccc agggcctggc cgaccgagag 180

gcaggtcggc cgatgcgcga ggacacgctg ttccggctcg cttcggtgac caagccgatc 240

gtcgcgctgg cggtgctgcg actggtggcg cgcggcgaac tcgcgctcga cgcgccggtc 300

acgcgctggt tgcccgaatt ccggccgcgg ctggccgacg gcagcgagcc gctcgtcacg 360

attcaccacc tgctcacgca cacgtcgggg ctcagctact ggctgctcga gggcgccggc 420

tccgtgtacg accggctcgg catctcggac ggcatcgacc tgcgcgactt cgatctcgac 480

gaaaacctgc gccgcctcgc ctcggcgccg ctatccttcg cgccgggcag cggctggcag 540

tattcgctgg cgctcgacgt gctcggcgcg gtggtcgagc gcgccaccgg gcagccgctg 600

gcggcggcgg tggacgcgtt ggtcgcccag ccgctcggca tgcgcgattg cggtttcgtc 660

tcggcggagc ccgagcgctt cgccgtgcct taccacgacg gccagccgga gccggtgcgc 720

atgcgcgacg gcatcgaggt gtcgctgggg acgacggccg tgcgtttcgc gccctcccgc 780

gtgttcgagc cgggcgccta tccctcgggc ggcgccggca tgtacggctc ggccgacgac 840

gtcctgcgcg cgctcgaggc gatccgcgcc aatcccggtt tcctgcccga gacgctggcc 900

gacgcggcgc gccgcgacca ggtcggagtc ggcgccaaga cgcgcggccc cggctggggc 960

ttcggctacc tgagcgcggt gctcgacgat ccggccgcgg ccggcacccc gcagcacgcc 1020

gggacgctgc aatggggcgg cgtctatggc cattcctggt tcgtcgaccg cgcgctggga 1080

ctcagcgtgc tgctgctcac caataccgcc tacgaaggca tgtcgggccc gctgacgatc 1140

gccttgcgcg acgccgtcta cgcgcgctga 1170

<210> 58
<211> 257
<212> PRT
<213> Hevea brasiliensis

<220>
<221> MISC_FEATURE
<223> Hydroxynitril-Lyase, GenBank-Nr. AAC49184

<400> 58

```
Met Ala Phe Ala His Phe Val Leu Ile His Thr Ile Cys His Gly Ala
1               5                   10                  15

Trp Ile Trp His Lys Leu Lys Pro Leu Leu Glu Ala Leu Gly His Lys
            20                  25                  30

Val Thr Ala Leu Asp Leu Ala Ala Ser Gly Val Asp Pro Arg Gln Ile
        35                  40                  45

Glu Glu Ile Gly Ser Phe Asp Glu Tyr Ser Glu Pro Leu Leu Thr Phe
    50                  55                  60

Leu Glu Ala Leu Pro Pro Gly Glu Lys Val Ile Leu Val Gly Glu Ser
65                  70                  75                  80

Cys Gly Gly Leu Asn Ile Ala Ile Ala Ala Asp Lys Tyr Cys Glu Lys
                85                  90                  95

Ile Ala Ala Ala Val Phe His Asn Ser Val Leu Pro Asp Thr Glu His
            100                 105                 110

Cys Pro Ser Tyr Val Val Asp Lys Leu Met Glu Val Phe Pro Asp Trp
        115                 120                 125

Lys Asp Thr Thr Tyr Phe Thr Tyr Thr Lys Asp Gly Lys Glu Ile Thr
    130                 135                 140

Gly Leu Lys Leu Gly Phe Thr Leu Leu Arg Glu Asn Leu Tyr Thr Leu
145                 150                 155                 160

Cys Gly Pro Glu Glu Tyr Glu Leu Ala Lys Met Leu Thr Arg Lys Gly
                165                 170                 175

Ser Leu Phe Gln Asn Ile Leu Ala Lys Arg Pro Phe Phe Thr Lys Glu
            180                 185                 190

Gly Tyr Gly Ser Ile Lys Lys Ile Tyr Val Trp Thr Asp Gln Asp Glu
        195                 200                 205

Ile Phe Leu Pro Glu Phe Gln Leu Trp Gln Ile Glu Asn Tyr Lys Pro
    210                 215                 220

Asp Lys Val Tyr Lys Val Glu Gly Gly Asp His Lys Leu Gln Leu Thr
225                 230                 235                 240

Lys Thr Lys Glu Ile Ala Glu Ile Leu Gln Glu Val Ala Asp Thr Tyr
                245                 250                 255
```

Asn

<210> 59
<211> 258
<212> PRT
<213> Manihot esculenta

<220>
<221> MISC_FEATURE
<223> Hydroxynitril-Lyase, Swiss-Prot Nr. P52705

<400> 59

```
Met Val Thr Ala His Phe Val Leu Ile His Thr Ile Cys His Gly Ala
1               5                   10                  15

Trp Ile Trp His Lys Leu Lys Pro Ala Leu Glu Arg Ala Gly His Lys
            20                  25                  30

Val Thr Ala Leu Asp Met Ala Ala Ser Gly Ile Asp Pro Arg Gln Ile
        35                  40                  45

Glu Gln Ile Asn Ser Phe Asp Glu Tyr Ser Glu Pro Leu Leu Thr Phe
    50                  55                  60

Leu Glu Lys Leu Pro Gln Gly Glu Lys Val Ile Ile Val Gly Glu Ser
65                  70                  75                  80

Cys Ala Gly Leu Asn Ile Ala Ile Ala Ala Asp Arg Tyr Val Asp Lys
                85                  90                  95

Ile Ala Ala Gly Val Phe His Asn Ser Leu Leu Pro Asp Thr Val His
            100                 105                 110

Ser Pro Ser Tyr Thr Val Glu Lys Leu Leu Glu Ser Phe Pro Asp Trp
        115                 120                 125

Arg Asp Thr Glu Tyr Phe Thr Phe Thr Asn Ile Thr Gly Glu Thr Ile
    130                 135                 140

Thr Thr Met Lys Leu Gly Phe Val Leu Leu Arg Glu Asn Leu Phe Thr
145                 150                 155                 160

Lys Cys Thr Asp Gly Glu Tyr Glu Leu Ala Lys Met Val Met Arg Lys
                165                 170                 175

Gly Ser Leu Phe Gln Asn Val Leu Ala Gln Arg Pro Lys Phe Thr Glu
            180                 185                 190
```

```
Lys Gly Tyr Gly Ser Ile Lys Lys Val Tyr Ile Trp Thr Asp Gln Asp
        195                 200                 205

Lys Ile Phe Leu Pro Asp Phe Gln Arg Trp Gln Ile Ala Asn Tyr Lys
    210                 215                 220

Pro Asp Lys Val Tyr Gln Val Gln Gly Gly Asp His Lys Leu Gln Leu
225                 230                 235                 240

Thr Lys Thr Glu Glu Val Ala His Ile Leu Gln Glu Val Ala Asp Ala
                245                 250                 255

Tyr Ala
```

## Beschreibung

Die vorliegende Erfindung betrifft ein Verfahren zur enzymkatalysierten Hydrolyse von Polyacrylsäureestern, die dabei verwendeten Enzyme und Mutanten davon, die Enzyme kodierende Nukleinsäuren, die Nukleinsäuren umfassende Vektoren, die Vektoren umfassende Mikroorganismen, die Verwendung der Enzyme, der Vektoren oder der Mikroorganismen zur Durchführung eines Verfahrens zur enzymkatalysierten Hydrolyse von Polyacrylsäureestern. Die vorliegende Anmeldung betrifft weiterhin durch das Verfahren erhältliche polymere Reaktionsprodukte sowie Verfahren zur Herstellung von Esterasen.

## Stand der Technik

Polyacrylsäureester (Polyacrylate) sind Verbindungen mit zahlreichen Anwendungen. Für Polyacrylate aus Homopolymeren gibt es eher eingeschränkte Anwendungsmöglichkeiten, während es bei Polyacrylaten aus Copolymeren möglich ist, durch Auswahl der zu verwendenden Comonomere (wie beispielsweise Methylacrylate, Styrol, Acrylnitril, Vinylacetat, Vinylchlorid, Vinylidenchlorid und Butadien) die Eigenschaften der Polyacrylate in vielfältiger Weise zu beeinflussen und damit verschiedensten Verwendungsmöglichkeiten zugänglich zu machen.

Chemische Verfahren zur Spaltung von Polyacrylsäureestern, beispielsweise alkalische Verseifung (US 3,926,891), sind dem Fachmann bekannt. Die US-Patentanmeldung 2004/0082023 A1 beschreibt ein Verfahren zur enzymatischen Veresterung von Carboxylgruppen tragenden Polymeren unter Verwendung von Enzymen wie Lipasen oder Esterasen. Polyacrylate werden als mögliche Polymere nicht explizit genannt, ebenso wenig wie die Eignung des Verfahrens zur enzymkatalysierten Esterspaltung von Polyacrylatestern.

O'Sullivan und Birkinshaw beschrieben den Versuch, Poly-(n-butylcyanoacrylat)-Nanopartikel mit Hilfe von Esterase aus Schweineleber zu hydrolysieren (O'Sullivan, Birkinshaw, Polymer Degradation and Stability 78: 7-15, 2002). Belucci et al. berichteten von der schonenden Entfernung von Acrylharz-Beschichtungen von der Oberfläche von Bildern unter Verwendung von Lipase (Belluci et al., Studies in Conservation 44: 278-281, 1999).

Dem Fachmann ist eine Vielzahl von Esterasen bekannt. Esterasen von *Burkholderia gladioli* sind beispielsweise beschrieben in Peterson et al., J. Biotechnol. 89:11-25 (2001), Valinger et al, J. Biotechnol. 129:98-108 (2007), Ivancic et al, J. Biotechnol. 129 :109-122 (2007) und Reiter et al, Appl. Microbiol. Biotechnol. 54:778-785 (2000). Eine Eignung dieser Esterasen zur Spaltung polymerer Substrate ist bisher nicht beschrieben worden.

Die der vorliegenden Erfindung zugrundeliegende Aufgabe besteht somit in der Bereitstellung eines Verfahrens zur enzymkatalysierten Hydrolyse von Polyacrylsäureestern sowie dafür geeigneter Enzyme, deren Nukleinsäuren, die Nukleinsäuren enthaltender Vektoren bzw. die Vektoren enthaltender Mikroorganismen, sowie durch das Verfahren erhältlicher Reaktionsprodukte.

## Kurze Beschreibung der Erfindung

Die oben genannte Aufgabe wurde überraschenderweise gelöst durch Verwendung von Esterasen, insbesondere unter Carboxylesterasen, Triacyllipasen und Cutinasen ausgewählten Enzymen, in einem Verfahren zur enzymkatalysierten Hydrolyse von Polyacrylsäureestern sowie durch die Bereitstellung entsprechender Esterasen und der dafür kodierenden Nukleinsäuren.

## Figurenbeschreibung

Figur 1 zeigt ein Strukturmodell von EstB. Zwei Loops, die als den Zugang zum katalytisch aktiven Zentrum bedeckend identifiziert wurden, sind hell hervorgehoben und durch Pfeile markiert. Der aufgehellte und ebenfalls mit einem Pfeil kenntlich gemachte Aminosäurerest stellt das nukleophile Serin des aktiven Zentrums dar.

Figur 2 zeigt ein Strukturmodell von EstC gemäß SEQ ID NO:2, wobei Phenylalanin 138 durch Alanin ersetzt wurde (Phe138Ala; entsprechend der Mutante 1 K22 in Figur 5). Angegeben ist das Rückgrat der Polypeptidkette. Die durch einen Pfeil gekennzeichnete Aminosäure Phenylalanin in Position 138 ist als Kalottenmodell ausgeführt, die in der Abbildung unterhalb von Phe138 als Kalottenmodell angegebenen Aminosäuren entsprechen den zum katalytisch aktiven Zentrum gehörenden Aminosäuren Ser112, Asp242 und His275.

Figur 3 zeigt ein Strukturmodell von EstC gemäß SEQ ID NO:2, wobei Leucin 193 durch Alanin ersetzt wurde (Leu193Ala; entsprechend der Mutante 2K20 in Figur 5). Angegeben ist das Rückgrat der Polypeptidkette. Die durch einen Pfeil gekennzeichnete Aminosäure Leucin in Position 193 ist als Kalottenmodell ausgeführt, die in der Abbildung oberhalb von Leu193 als Kalottenmodell angegebenen Aminosäuren entsprechen den zum katalytisch aktiven Zentrum gehörenden Aminosäuren Ser112, Asp242 und His275.

Figur 4 zeigt ein Strukturmodell von EstC gemäß SEQ ID NO:2, wobei Phenylalanin 138 durch Alanin, Leucin 193 durch Alanin, und Threonin 188 durch Serin ersetzt wurden (Phe138Ala, Leu193Ala, Thr188Ser; entsprechend Mutante B48 in Figur 5). Angegeben ist das Rückgrat der Polypeptidkette. Durch einen Pfeil gekennzeichnet sind die als Kalottenmodell ausgeführten Aminosäuren Phe138, Thr188 und Leu193. Die übrigen als Kalottenmodell angegebenen Aminosäuren entsprechen den zum katalytisch aktiven Zentrum gehörenden Aminosäuren Ser112, Asp242 und His275.

Figur 5 zeigt aktive Mutanten und Aktivitäten in der Übersicht. Die Ergebnisse wurden erhalten durch pH-Shift-Assays mit Esterase C (Fig. 5a) und Esterase B (Fig. 5b) und verschiedenen funktionalen Mutanten davon. Für die Aktivität wurden folgende Symbole verwendet:

+++ = Sehr aktiv, ++ = aktiv, + = schwach aktiv, o = nicht aktiv

EstB und deren Mutanten wurden bei der Erhebung dieser Daten nicht auf $\alpha$-Naphthylacetat getestet, jedoch liegen den Erfindern Daten vor, dass alle drei EstB-Varianten Aktivität hinsichtlich dieses Substrats aufweisen.

Figur 6 zeigt die enzymkatalysierte Esterspaltung von PBA nach Zugabe von Rohlysat des Esterase-produzierenden Stammes NJ70 (entsprechend 1000 Einheiten EstB_NJ70) im Vergleich zur chemischen Esterspaltung nach Zugabe von Rohlysat nach Denaturierung.

Figur 7 zeigt Autotitrationen von EstB-Esterasen (Wildtyp und Mutanten) mit dem Substrat PBA. Eine eventuell inhibierende Wirkung von Sokalan (= Polyacrylsäure) wurde untersucht.

Figur 8 zeigt die pH-Abhängigkeit der enzymkatalysierten Esterspaltung. Alle Messungen wurden mit 1000 Einheiten EstB_NJ70 (bestimmt mit p-NPB) und 750 $\mu$l PBA durchgeführt.

Figur 9 zeigt die Temperaturabhängigkeit der enzymkatalysierten Esterspaltung. Alle Autotitrationen wurden mit 1000 Einheiten EstB_NJ70 (bestimmt mit p-NPB) und 750 $\mu$l PBA durchgeführt.

Figur 10 zeigt den Reaktionsverlauf der Esterspaltung durch EstB_NJ70 in Lösung bzw. immobilisierte EstB_NJ70. Gezeigt sind die Ergebnisse von Autotitrationen mit 4,66 mmol PBA von entweder nicht immobilisierter oder auf Eupergit immobilisierter EstB_NJ70.

Figur 11 zeigt die Spaltung von Polyacrylsäuremethylestern unterschiedlicher Kettenlänge,
wobei zur Kontrolle denaturiertes Enzym eingesetzt wurde.

## Detaillierte Beschreibung der Erfindung

### I. Erläuterungen allgemeiner Begriffe

Als "Esterasen" werden im Rahmen der vorliegenden Erfindung, sofern keine weitere Spezifizierung angegeben ist, allgemein Enzyme bezeichnet, welche die Hydrolyse von Esterbindungen katalysieren.

Unter "auf Esterbindungen einwirkenden Enzymen" sind Enzyme der Klasse 3.1 gemäß EC-Klassifizierung zu verstehen. Unter "Carbonsäureesterhydrolasen" sind Enzyme der Klasse 3.1.1 gemäß EC-Klassifizierung zu verstehen. Unter "Carboxylesterasen", "Triacylglycerinlipasen" bzw. "Cutinasen" sind Enzyme der EC-Klassen 3.1.1.1, 3.1.1.3 beziehungsweise 3.1.1.74 zu verstehen.

Für "Esterasen der Familie VIII" gilt die in Petersen et al, J Biotechnol 89:11-25 (2001) sowie die in Arpigny und Jaeger, Biochem J 343:177-183 (1999) angegebene Definition. Esterasen der Familie VIII sind somit gekennzeichnet durch ein aktives Zentrum (active site), die ein Ser-X-X-Lys-Motif aufweist (wobei X für jede beliebige Aminosäure steht) und somit Ähnlichkeit mit der aktiven Stelle von $\beta$-Lactamasen der Klasse C aufweisen, wobei die Esterase-Aktivität durch einen der in Petersen et al. (ibid.) beschriebenen Esterase-Aktivitäts-Assays gegebenenfalls nachgewiesen werden kann.

Im Rahmen der Erfindung werden unter Typ C Esterasen Enzyme verstanden, die wenigstens 50% Identität auf Aminosäureebene mit EstC aus *Burkholderia gladioli* (Reiter et al., Appl Microbiol Biotechnol 54:778-785 (2000) gemäß SEQ ID NO:2 aufweisen, weiterhin wenigstens 20% Identität auf Aminosäure-Ebene mit der Hydroxynitril-Lyase aus *Hevea brasiliensis* (Hasslacher M et al, J Biol Chem 271:5884-5891 (1996), GenBank-Accession-Nr. AAC49184), SEQ ID NO:58, und/oder der Hydroxynitril-Lyase aus *Manihot esculenta* (Hughes et al., Arch Biochem Biophys 311: 96-502 (1994), Swiss-Prot-Accession-Nr. P52705), SEQ ID NO:59.

Im Rahmen der vorliegenden Erfindung umfasst eine "enzymkatalysierte Hydrolyse" eines Polyacrylsäureesters auch Reaktionen, die eine partielle Autolyse einschließen. Bei "partieller Autolyse" wird ein Anteil von 0 - 90 Mol-%, 0 - 50 Mol-%, 0 - 25 Mol-%, 0 - 20 Mol-%, insbesondere 0 - 15 Mol-%, 0 - 10 Mol-%, 0 - 5 Mol-% oder 0 - 1 Mol-% der Estergruppen autolytisch gespalten.

Unter einem Halogen wird Fluor, Chlor, Brom oder lod verstanden.

Unter einem Loop oder einer Loop-Struktur wird ein Abschnitt von in der Primärstruktur aufeinanderfolgenden Aminosäuren verstanden, der in der Tertiärstruktur des Proteins ein schleifenförmiges Strukturelement ausbildet.

Unter der Hydrolyse von Polyacrylsäureestern oder der Hydrolyse-Aktivität gegenüber Polyacrylsäureestern wird die unvollständige oder vollständige Hydrolyse der Esterbindungen der Polyacrylsäureester verstanden.

Unter der Angabe "etwa" wird der angegebene Wert, gegebenenfalls mit einer Abweichung von bis zu 25% nach oben oder unten, insbesondere bis zu 10% nach oben oder unten, oder bis zu 5 % nach oben oder unten verstanden.

Unter dem mittleren Molekulargewicht wird, sofern keine anderen Angaben gemacht werden, das Gewichtsmittel des Molekulargewichts verstanden.

Unter einem alternierenden Copolymer wird ein aus zwei Monomeren A und B bestehendes Copolymer verstanden, wobei die Monomere in streng alternierender Folge $(AB)_n$ vorliegen. Unter einem statistischen Copolymer wird ein Copolymer verstanden, bei dem der Einbau der Monomere (z.B. A und B) in das bei der Copolymerisation entstehende Makromolekül statistisch, d.h. in der Reihenfolge rein zufällig erfolgt. Unter einem Gradientencopolymer wird ein Copolymer verstanden, bei dem ein Gradient der Verteilung der Monomerbausteine (z.B. der Bausteine A und B) entlang der Ketten der Copolymere besteht. Unter einem Blockpolymer wird ein Polymer verstanden, dessen Moleküle aus linear verknüpften Blöcken bestehen. Unter einem Block versteht man dabei einen Abschnitt eines Polymermoleküls, der mehrere identische Repetiereinheiten umfasst und mindestens ein konstitutionelles oder konfiguratives Merkmal besitzt, das sich von denen der angrenzenden Abschnitte (Blöcke) unterscheidet. Die Blöcke sind direkt oder durch konstitutionelle Einheiten, die nicht Teil der Blöcke sind, miteinander verbunden. Unter Blockcopolymeren werden Blockpolymere verstanden, die aus mehr als einer Art von Monomer bestehen und die für z.B. aus zwei Monomeren-Arten A und B aufgebaute Blockcopolymere durch die allgemeine Formel $-A_k-B_l-A_m-B_n-$ zu beschreiben sind, wobei k, l, m und n für die Anzahl der Repetiereinheiten in den einzelnen Blöcken stehen. Unter Pfropfcopolymeren werden nach dem Verfahren der Pfropfcopolymerisation hergestellte Polymere bezeichnet, wobei für deren Aufbau charakteristisch ist, dass sie an ihrer Hauptkette Seitenketten tragen, die von einer Länge sind, dass sie bereits für sich als Polymere anzusprechen wären. Haupt- und Seitenketten können chemisch identisch oder verschiedenartig sein.

Unter Alkoholderivaten werden von Alkoholen abgeleitete Moleküle verstanden, beispielsweise Alkohole, bei denen eine oder mehrere Hydroxygruppen durch andere funktionelle Gruppen, wie beispielsweise Aminogruppen oder Sulfhydrylgruppen, ersetzt sind.

Es werden unter Anderem folgende Abkürzungen verwendet:

DG: 2,4-Dimethylglutarsäure-dimethylester
DB: 2,4-Dimethylglutarsäure-dibutylester
PMA: Polymethylacrylat
PBA: Polybutylacrylat
α -N: α-Naphthylacetat
p-NPA: para-Nitrophenylacetat
p-NPB: para-Nitrophenylbutyrat

**<u>II. Spezielle Gegenstände der Erfindung</u>**

Ein erster Gegenstand der Erfindung betrifft Verfahren zur enzymkatalysierten Hydrolyse von Polyacrylsäureestern, wobei man wenigstens einen Polyacrylsäureester bereitstellt, den wenigstens einen Polyacrylsäureester mit wenigstens einem Enzym inkubiert, das ausgewählt ist unter auf Esterbindungen einwirkenden Enzymen (EC 3.1), bis die in dem Polyacrylsäureester enthaltenen Estergruppen teilweise oder vollständig hydrolytisch gespalten sind, und gegebenenfalls das dadurch erhaltene modifizierte Polymer isoliert. Bei dem Verfahren kann der Polyacrylsäureester ein Homopolymer oder ein Copolymer aus zwei oder mehr unterschiedlichen Monomeren sein.

Gemäß weiteren Ausführungsformen ist das Enzym ausgewählt unter Carbonsäureesterhydrolasen (EC 3.1.1). Gemäß besonders bevorzugten Ausführungsformen sind die Carbonsäureesterhydrolasen ausgewählt unter Carboxylesterasen (E.C 3.1.1.1), Triacylglycerinlipasen (EC. 3.1.1.3) und Cutinasen (EC 3.1.1.74).
Gemäß weiteren Ausführungsformen ist der Polyacrylsäureester ein Homopolymer oder ein Copolymer. Beispiele für Copolymere sind alternierende Copolymere, statistische Copolymere, Gradientencopolymere, Blockcopolymere oder Pfropfcopolymere. Als Monomere der Copolymere kommen beispielsweise alle hier offenbarten Monomere in Frage.

Gemäß einer bevorzugten Ausführungsform des Verfahrens umfasst das Polymer MonomerBausteine der allgemeinen Formel I

$$R^1R^2C{=}CR^3\text{-}COOR^4 \qquad (I),$$

worin
$R^1$, $R^2$ und $R^3$ gleich oder verschieden sind und ausgewählt sind unter H, einem geradkettigen $C_1$-$C_{20}$ Hydrocarbylrest und einem verzweigten $C_3$-$C_{20}$ Hydrocarbylrest, und $R^4$ ausgewählt ist unter H, einem geradkettigen $C_1$-$C_{20}$ oder $C_1$-$C_6$ Hydrocarbylrest, einem verzweigten $C_3$-$C_{20}$ oder $C_3$-$C_6$ Hydrocarbylrest und einem cyclischen $C_3$-$C_{20}$ oder $C_5$-$C_7$ Hydrocarbylrest,
wobei der Hydrocarbylrest gegebenenfalls substituiert ist durch eine oder mehrere gleiche oder verschiedene Gruppen, die ausgewählt sind unter Hydroxy-, Amino-, Epoxidgruppen und Halogenatomen,

wobei im Polymer in wenigstens einem Monomerbaustein der Formel I $R^4$ ausgewählt ist unter einem geradkettigen $C_1$-$C_{20}$ oder $C_1$-$C_6$ Hydrocarbylrest, einem verzweigten $C_3$-$C_{20}$ oder $C_3$-$C_6$ Hydrocarbylrest und einem cyclischen $C_3$-$C_{20}$ oder $C_5$-$C_7$ Hydrocarbylrest, wobei der Hydrocarbylrest gegebenenfalls substituiert ist durch eine oder mehrere gleiche oder verschiedene Gruppen, die ausgewählt sind unter Hydroxy-, Amino-, Epoxid-, Thiolgruppen und Halogenatomen.

Geradkettige $C_1$-$C_{20}$ Hydrocarbylreste umfassen Methyl-, Ethyl-, Propyl-, Butyl-, Pentyl-, Hexyl-, Heptyl-, Octyl-, Nonyl-, Decyl-, Undecyl-, Dodecyl-, Tridecyl-, Tetradecyl-, Pentadecyl-, Hexadecyl-, Heptadecyl-, Octadecyl-, Nonodecyl- und Eicosylreste. Verzweigte $C_3$-$C_{20}$ Hydrocarbylreste umfassen beispielsweise Isopropyl-, Isobutyl-, Isopentyl-, 2,2-Dimethylpropyl-, Isohexyl-, 3-Methylpentyl-, 2,2-Dimethylbutyl-, 2,3-Dimethylbutyl-, Isoheptyl, 3-Methylhexyl-, 2,2-Dimethylpentyl-, 2,3-Dimethylpentyl-, 2,4-Dimethylpentyl-, 2,2,3-Trimethylbutyl-, Isooctyl-, 3-Methylheptyl-, 4-Methylheptyl-, 2,2-Dimethylhexyl-, 2,4-Dimethylhexyl-, 2,5-Dimethylhexyl-, 2,2,3-Trimethylpentyl-, 2,2,4-Trimethylpentyl-, 2,2,5-Trimethylpentyl- und Isononylreste. Beispiele für $C_3$-$C_{20}$ Cycloalkylreste sind Cyclopropyl-, Cyclobutyl-, Cyclopentyl-, Cyclohexyl-, Cycloheptyl-, Cyclooctyl-, Cyclononyl-, Cyclodecyl-, Cycloundecyl-, Cyclododecyl-, Cyclotridecyl-, Cyclotetradecyl-, Cyclopentadecyl-, Cyclohexadecyl-, Cycloheptadecyl-, Cyclooctadecyl-, Cyclononadecyl- und Cycloeicosylreste. Nicht-limitierende Beispiele für substitutierte Hydroxycarbylreste sind Hydroxymethyl-, Hydroxyethyl-, Hydroxypropyl-, Hydroxybutyl-, Hydroxylpentyl-, Hydroxyhexyl-, Hydroxyheptyl-, Hydroxyoctyl-, Hydroxynonyl- und Hydroxydecylreste. Das Polymer der allgemeinen Formel (I) kann also bei Vorliegen mehrerer Hydroxylgruppen ein Polyol sein. Gemäß weiteren, nicht-limitierenden Beispielen sind die Hydroxycarbylreste ausgewählt unter Kohlenhydraten, also insbesondere Polyhydroxyaldehyden und Polyhydroxyketonen, die als Monomere, Oligomere oder Polymere vorliegen können. Dabei können in einem Polyacrylsäureester gleiche oder unterschiedliche Monomere vorkommen, und Oligomere bzw. Polymere können gleiche oder unterschiedliche Monomere umfassen. Kohlenhydrate sind dem Fachmann bekannt, und nicht-limitierende Beispiele für Polyhydroxyaldehyde umfassen Threose, Ribose, Arabinose, Xylose, Lyxose, nicht-limitierende Beispiele für Polyhydroxyketone umfassen Dihydroxyaceton, Erythrulose, Ribulose, Xylulose, Fructose, Sorbose, Mannoheptulose.

Gemäß einer weiteren Ausführungsform ist das erfindungsgemäße Verfahren **dadurch gekennzeichnet, dass** der Polyacrylsäureester zusätzlich zu den Monomeren der Formel I wenigstens eine weitere davon verschiedene Monomerkomponente in einem molaren Anteil von 0 bis 15 Mol-% enthält, welche vorzugsweise ausgewählt ist unter N-Vinylformamid, Methacrylsäure, Methacrylsäureester, Itaconsäure, Itaconsäureester, Vinylphosphonsäure, Vinylsulfonsäure, Vinylalkohol, N-Vinylimidazol, N-Vinylformamid, Styrol, Maleinsäure, Maleinsäurester, Ethylen, Propylen Acrylamid und substituierten Acrylamiden, wobei der Substituent ausgewählt ist unter einem geradkettigen $C_1$-$C_{20}$ oder $C_1$-$C_6$ Hydrocarbylrest, einem verzweigten $C_3$-$C_{20}$ oder $C_3$-$C_6$ Hydrocarbylrest und einem cyclischen $C_3$-$C_{20}$ oder $C_5$-$C_7$Hydrocarbylrest, wobei der Hydrocarbylrest gegebenenfalls substituiert ist durch eine oder mehrere gleiche oder verschiedene Gruppen, die ausgewählt sind unter Hydroxy-, Amino-, Epoxid-, Thiolgruppen und Halogenatomen.

In einer Ausführungsform des Verfahrens sind die Acrylsäuregruppen der Polyacrylsäureester vor der Hydrolyse vollständig oder im Wesentlichen vollständig verestert.

Bei den erfindungsgemäßen Verfahren beträgt das mittlere Molekulargewicht des Polyacrylsäureesters bis zu etwa 3.000.000, beispielsweise von etwa 1.000 bis zu etwa 3.000.000, insbesondere bis zu etwa 200.000, 150.000, 100.000 oder 50.000. Der Polyacrylsäureester ist vorzugsweise ausgewählt unter Polyacrylsäuremethylester mit einem mittleren Molekulargewicht von etwa 20.000 bis etwa 3.000.000, insbesondere etwa 30.000 bis etwa 50.000, insbesondere etwa 40.000, und Polyacrylsäurebutylester mit einem mittleren Molekulargewicht von etwa 20.000 bis etwa 3.000.000, insbesondere etwa 90.000 bis etwa 110.000, insbesondere etwa 99.000.

In weiteren Ausführungsformen des erfindungsgemäßen Verfahrens erfolgt die Inkubation bei einem pH-Wert von 5 bis 14, vorzugsweise 7 bis 12, 8,5 bis 11,5, insbesondere bei einem pH-Wert von 9 bis 11 oder bei einem pH-Wert von 7 bis 9.

Gemäß weiteren Ausführungsformen des erfindungsgemäßen Verfahrens liegt das Enzym in einer Lösung vor, insbesondere in einer wässrigen, organischen oder wässrig-organischen, organisch-wässrigen oder organischen Lösung vor. Im Rahmen der vorliegenden Erfindung umfassen organisch-wässrige oder wässrig-organische Lösungen nicht nur homogene Lösungen vollständig mischbarer Komponenten (beispielsweise Wasser und organisches Lösungsmittel), sondern auch Zweiphasensysteme oder Mehrphasensysteme, beispielsweise Wasser-in-Öl-Emulsionen, Öl-in-Wasser-Emulsionen, Wasser-in-Öl-in-Wasser-Emulsionen, usw. Das Enzym liegt dabei vorzugsweise vollständig oder überwiegend in der wässrigen Phase vor, der Polyacrylsäureester vorzugsweise vollständig oder überwiegend in der organischen Phase. Gemäß besonderen Ausführungsformen beträgt das Volumenverhältnis von wässriger zu organischer Komponente bzw. von wässriger zu organischer Phase etwa 75:25 bis 25:75, beispielsweise etwa 60:40 bis etwa 40:60, etwa 55:45 bis etwa 45:55, insbesondere etwa 50:50.

Gemäß besonderen Ausführungsformen liegt das Enzym in nicht-immobilisierter Form vor.

Gemäß einer weiteren Ausführungsform des erfindungsgemäßen Verfahrens liegt das Enzym in immobilisierter Form vor. Beispiele für immobilisierte Enzyme sind kovalent oder nicht-kovalent an Mikrosphären oder flächenartig geformte Träger gebundene Enzyme. Geeignete Methode zur Immobilisierung von Enzymen sind dem Fachmann bekannt und beispielsweise beschrieben in S. Fukui, A. Tanaka, "Enzymes", in: Ullmanns Encyclopedia of Industrial Chemistry, 5. Aufl., Wiley-VCH, New York, Berlin, 1985; J.E. Prenosil, O.M. Kut, I.J. Dunn, E. Heinzle, "Immobilized Biocatalysts", in: Ullmanns Encyclopedia of Industrial Chemistry, 6. Aufl., VCH, New York, Berlin, 2003, S. 503-554; J.F. Kennedy, J.M.S Cabral, in: H.J.Rehm, G. Reed (Hrsg.), Biotechnology: A comprehensive treatise in 8 vol.; Band 7a, S. 349-404; VCH, Weinheim, 1987. Nicht-kovalente Bindung des Enzyms kann beispielsweise erreicht werden durch Markierung des Enzyms mit Biotin und Immobilisierung an einen mit Avidin oder Streptavidin versehenen Träger, Markierung des Enzyms durch Einbau von Histidin-Tags in dessen Aminosäuresequenz und Immobilisierung an einen mit Nickel-Chelat versehenen Träger, Immobilisierung des Enzyms durch gegen das Enzym gerichtete Antikörper (wobei die Bindungsepitope vorzugsweise so gewählt sind, dass die Bindung zwischen Antikörper und Bindungsepitop den Zugang von Substratmolekülen zum aktiven Zentrum nicht oder nicht wesentlich beeinträchtigt), Herstellung von Fusionsproteinen aus dem Enzym und einem Fremdprotein und Immobilisierung des Proteins durch gegen das Fremdprotein gerichtete Antikörper. In Analogie zu den oben gemachten Ausführungen können in immobilisierter Form vorliegende Enzyme Bestandteil eines Zweiphasensystems oder Mehrphasensystems sein. Beispielsweise kann ein auf einem festen Träger (wie beispielsweise einer Mikrosphäre) immobilisiertes Enzym als feste Phase angesehen werden, die im Rahmen eines Zweiphasensystems in einer flüssigen Phase (Wasser oder organisches Lösungsmittel) vorliegt, oder das auf einem festen Träger immobilisierte Enzym kann als feste Phase in einem bereits zwei- oder mehrphasigen flüssigen System (beispielsweise einer Wasser-in-Öl-Emulsion oder einer Öl-in-Wasser-Emulsion) vorliegen.

Gemäß einer weiteren Ausführungsform wird das Verfahren in einem Bioreaktor durchgeführt.

In einer weiteren Ausführungsform des erfindungsgemäßen Verfahrens ist das Enzym eine Esterase, die ausgewählt ist unter Esterasen der Familie VIII, Typ C Esterasen, einer Cutinase gemäß SEQ ID NO:5 oder davon abgeleiteten Cutinasen, sowie einer Triacylglycerinlipase gemäß SEQ ID NO:6 oder davon abgeleiteten Triacylglycerinlipasen. In einer speziellen Ausführungsform ist die Esterase vom Typ VIII die Esterase B von *Burkholderia gladioli* (ATCC 10248 ) gemäß SEQ ID NO:1, in einer weiteren speziellen Ausführungsform ist die Typ C Esterase die Esterase C aus *Burkholderia gladioli* (ATCC 10248) gemäß SEQ ID NO:2. Gegenstand der Erfindung sind weiterhin funktionale Mutanten der genannten Esterasen.

Gemäß weiteren Ausführungsformen des erfindungsgemäßen Verfahrens ist die Esterase eine funktionale Mutante einer Esterase gemäß SEQ ID NO:1 oder SEQ ID NO:2 mit gegenüber SEQ ID NO:1 bzw. SEQ ID NO:2 vergleichbarer oder gesteigerter Aktivität hinsichtlich der Hydrolyse von Polyacrylsäureestern, und/oder eine funktionale Mutante einer Esterase gemäß SEQ ID NO:1 oder SEQ ID NO:2 mit gegenüber SEQ ID NO:1 bzw. SEQ ID NO:2 erhöhter Stabilität, beispielsweise gegenüber Einfluss von organischen Lösungsmitteln oder bezüglich der Denaturierung durch erhöhte Temperaturen.

Gemäß weiteren Ausführungsformen des erfindungsgemäßen Verfahrens zeigt die Mutante im Vergleich mit einer Esterase gemäß SEQ ID NO:1 oder SEQ ID NO:2 eine gesteigerte Hydrolyse-Aktivität gegenüber Polyacrylsäuremethylester und/oder Polyacrylsäurebutylester. Die Hydrolyse-Aktivität kann beispielsweise quantitativ ermittelt werden durch einen Titrationsassay, bei dem eine Esterspaltung in Lösung stattfindet und der durch die freiwerdenden Säuregruppen absinkende pH-Wert durch Zugabe von NaOH (oder eines anderen pH-Korrekturmittels) kompensiert wird. Eine gesteigerte Hydrolyse-Aktivität liegt beispielsweise vor, wenn die Mutante im Vergleich mit der entsprechenden Esterase gemäß SEQ ID NO:1 oder SEQ ID NO:2 eine schnellere Einstellung eines Reaktionsgleichgewichts herbeiführt (d.h. die gleiche Menge NaOH in kürzerer Zeit zugegeben werden muss) und/oder das Gleichgewicht in Richtung einer vollständigeren Hydrolyse verschiebt (d.h. mehr NaOH zugegeben werden muss). Die Hydrolyse-Aktivität kann weiterhin halb-quantitativ in einem pH-Shift-Assay bestimmt werden durch Inkubation der Enzym-Mutante oder eines die Enzym-Mutante produzierenden Mikroorganismus mit einem Polyacrylsäureester enthaltenden Substrat, das beispielsweise in einem Agarmedium oder auf einer Filtermembran angeboten werden kann, und Bestimmung des Abfalls des pH-Wertes durch freiwerdende Säuregruppen durch Farbumschlag eines gleichzeitig anwesenden pH-Indikators. Eine gesteigerte Hydrolyse-Aktivität liegt vor, wenn die Mutante im Vergleich mit der entsprechenden Esterase gemäß SEQ ID NO:1 oder SEQ ID NO:2 einen schnelleren Umschlag zu niedrigen pH-Werten oder einen intensiveren Umschlag (größere Farbumschlagshöfe um Enzym-haltige Stellen herum) zu niedrigeren pH-Werten bewirkt.

Gemäß weiteren Ausführungsformen ist das erfindungsgemäße Verfahren **dadurch gekennzeichnet, dass**

(a) die Esterase eine Mutante einer Esterase gemäß SEQ ID NO:1 ist, die wenigstens eine Mutation in einem oder mehreren der Aminosäurereste Ser17, Gly132, Trp134, Arg155, Glu251, Ala311 und Glu316 aufweist; oder
(b) die Esterase eine Mutante einer Esterase gemäß SEQ ID NO:2 ist, die wenigstens eine Mutation in einem oder mehreren der Aminosäurereste Phe138, Val150, Leu160, Thr188 und Leu193 aufweist.

In weiteren Ausführungsformen ist das erfindungsgemäße Verfahren **dadurch gekennzeichnet, dass** die Esterase von SEQ ID NO:1 abgeleitet ist und

a) wenigstens eine der Mutationen Ser17Leu, Gly132Ser, Glu251Gly, Ala311Val und Glu316Lys und/oder
b) wenigstens eine der Mutationen Pro8Leu, Gly132Ser, Trp134Arg, Arg155Cys, Glu251Gly, Ala311Val und Glu316Lys umfasst.

In besonders bevorzugten Ausführungsformen ist das erfindungsgemäße Verfahren **dadurch gekennzeichnet, dass** die Esterase von SEQ ID NO:1 abgeleitet ist und

(a) die Mutationen Ser17Leu, Gly132Ser, Glu251Gly, Ala311Val und Glu316Lys; oder
(b) die Mutationen Pro8Leu, Gly132Ser, Trp134Arg, Arg155Cys, Glu251Gly, Ala311Val und Glu316Lys umfasst.

Besondere Ausführungsformen stellen dabei Esterasen gemäß SEQ ID NO:3 bzw. SEQ ID NO:4 dar.
In weiteren besonderen Ausführungsformen ist das erfindungsgemäße Verfahren **dadurch gekennzeichnet, dass** die Esterase von SEQ ID NO:2 abgeleitet ist und eine der nachfolgenden Mutationen oder Kombinationen von Mutationen umfasst:

(a) Phe138Ala
(b) Phe138Ala, Thr188Ser
(c) Phe138Ala, Leu160Ala, Thr188Ser
(d) Leu193Ala
(e) Leu193Ala, Phe138Ala, Thr188Ser, Val150Ala
(f) Leu193Ala, Phe138Ala, Thr188Ser
(g) Leu193Ala, Phe138Ala, Thr188Ser, Leu160Ala, Val150Ala
(h) Val150Ala
(i) Val150Ala, Thr188Ser
(j) Leu193Ala, Phe138Val
(k) Leu193Ala, Phe138Val, Thr188Ser, Val150Ala
(l) Leu193Ala, Thr188Ser
(m) Leu193Ala, Phe138Val, Thr188Ser
(n) Leu193Ala, Phe138Val, Thr188Ser, Leu160Ala
(o) Phe138Val, Va1150Ala, Thr188Ser
(p) Phe138Val
(q) Phe138Val, Thr188Ser

Gemäß weiteren Ausführungsformen des erfindungsgemäßen Verfahrens ist die Esterase eine Deletionsmutante einer Esterase vom Typ VIII oder einer Typ C Esterase. Vorzugsweise weist die Esterase dabei eine Loop-Kürzung auf. Geeignete Bereiche für eine Loop-Kürzung bei einer Esterase gemäß SEQ ID NO:1 (EstB von *B. gladioli*) stellen beispielsweise die Bereiche Glu246 bis Arg 258 und Gly312 bis 323. Dabei können Loop-Kürzungen durchgeführt werden durch Entfernen einer oder mehrerer Aminosäuren, wobei im Fall des Entfernens mehrerer Aminosäuren wiederum ein oder mehrere in SEQ ID NO:1 benachbart oder nicht benachbart gelegene Aminosäuren entfernt werden können. Besondere Ausführungsformen stellen Deletionsmutanten der unter SEQ ID NO:37 und SEQ ID NO:38 angegebenen Aminosäuresequenzen dar.
Ein weiterer Gegenstand der Erfindung betrifft die zuvor genannten funktionalen Esterase-Mutanten.
Ein weiterer Gegenstand der Erfindung betrifft Nukleinsäuren,

a) die für funktionale Esterase-Mutanten kodieren, oder
b) die zu a) komplementäre Nukleinsäuren darstellen, und/oder
c) mit einer Nukleinsäure gemäß a) oder b) unter stringenten Bedingungen hybridisierende Nukleinsäuren, insbesondere solche Nukleinsäuren, welche eine Sequenzidentität von wenigstens 80 % aufweisen und für eine Mutante einer Esterase der Familie VIII oder eine Typ C Esterase-Mutante kodieren, die Polyacrylsäureester hydrolysiert.

Ein weiterer Gegenstand der Erfindung betrifft einen Vektor, umfassend eine der vorstehend genannten Nukleinsäuren. Gemäß einer besonderen Ausführungsform ist die Nukleinsäure operativ mit einem Promotor verknüpft.
Ein weiterer Gegenstand der Erfindung betrifft einen Mikroorganismus, umfassend wenigstens einen der vorstehend genannten Vektoren.
Ein weiterer Gegenstand der Erfindung betrifft ein Verfahren zur Herstellung einer der vorstehend genannten funktionalen Esterase-Mutanten, wobei man

a) eine zur Expression der Esterase befähigten Wirtsorganismus, beispielsweise den vorstehend genannten Mikroorganismus, der wenigstens einen der vorstehend genannten Vektoren enthält, kultiviert
b) gegebenenfalls die Expression der Esterase induziert, und

c) gegebenenfalls die Esterase aus dem Wirtsorganismus und/oder dem Kulturmedium isoliert.

Ein weiterer Gegenstand der Erfindung betrifft die Verwendung einer der vorstehend genannten Esterasen, eines der vorstehend genannten Vektoren, oder eines der vorstehend genannten Mikroorganismen zur Durchführung eines der vorstehend genannten Verfahren.

Ein weiterer Gegenstand der Erfindung betrifft ein polymeres Reaktionsprodukt, das erhältlich ist durch eines der vorstehend genannten Verfahren.

## III. Weitere Angaben zur Ausführung der Erfindung

### 1. Verfahren zur enzymkatalysierten Hydrolyse von Polyacrylsäureestern

Das erfindungsgemäße Verfahren betrifft eine enzymkatalysierte Hydrolyse von Polyacrylsäureestern, die hier auch als enzymatische Esterspaltung (oder in Kurzform: Esterspaltung) bezeichnet wird.

Im Rahmen des erfindungsgemäßen Verfahrens ist die Bereitstellung wenigstens eines Polyacrylsäureesters und dessen Inkubation mit wenigstens einer Esterase vorgesehen. Die Bereitstellung erfolgt vorzugsweise in einer Lösung, wobei eine wässrige Lösung, eine organische Lösung (umfassend ein oder mehrere organische Lösungsmittel) oder wässrig-organische Lösung (wobei die organische Lösungsmittelkomponente ein oder mehrere organische Lösungsmittel umfasst) vorliegen kann. Zu den verwendbaren organischen Lösungsmitteln gehören beispielsweise Alkohole, wie beispielsweise Methanol, Ethanol, Propanol, Isopropanol und Butanol, aromatische Kohlenwasserstoffe, wie beispielsweise Benzol und Toluol, Ether, wie beispielsweise Dimethylether, Diethylether, 1,2-Dimethoxyethan, und Tetrahydrofuran. Bevorzugt sind wässrige Lösungen oder wässrig-organische Lösungen. In einer wässrig-organischen Lösung kann die organische Lösung bzw. die Summe der organischen Lösungsmittelkomponenten einen Volumenanteil von 1 bis 80 Vol.-%, vorzugsweise von 10 bis 60 Vol.-%, und insbesondere von etwa 40 Vol.-% einnehmen. Die wässrig-organischen Lösungen bzw. organisch-wässrigen Lösungen können im Rahmen der Erfindung homogene Lösungen sein, oder Zwei- oder Mehrphasensysteme (wie beispielsweise Öl-in-Wasser-, Wasser-in-Öl-, Wasser-in-Öl-in-Wasser-Emulsionen). Um einen einfachen Kontakt zwischen Enzym und Polyacrylsäureester zu ermöglichen, sind die Lösungen vorzugsweise nicht gelartig. Die Lösungen weisen vorzugsweise Viskositäten von weniger als 4000 mPa*s, insbesondere weniger als 2000 mPa*s, weniger als 1000 mPa*s, weniger als 500 mPa*s, weniger als 400 mPa*s, weniger als 200 mPa*s, weniger als 100 mPa*s, weniger als 50 mPa*s, weniger als 25 mPa*s, weniger als 10 mPa*s, weniger als 5 mPa*s oder weniger als 2,5 mPa*s auf. Die wässrige Lösung oder die wässrige Komponente einer wässrig-organischen Lösung kann einen Puffer darstellen. Der pH-Wert des Puffers wird vorzugsweise eingestellt auf den pH-Wert, bei dem die enzymkatalysierte Esterspaltung erfolgen soll. Wird das erfindungsgemäße Verfahren in einem Bioreaktor durchgeführt, in dem Mikroorganismen die wenigstens eine Esterase produzieren, welche den wenigstens einen Polyacrylsäureester spaltet, so wird der pH-Wert vorzugsweise auf einen für die Kultivierung des Mikroorganismus geeigneten Wert eingestellt. Der Fachmann ist mit dem Ermitteln geeigneter pH-Werte vertraut. Geeignete Puffer zur Verwendung im erfindungsgemäßen Verfahren sind dem Fachmann ebenfalls bekannt und umfassen beispielsweise PBS, Tris-HCl-Puffer, Triethanolamin-Hydrochlorid/NaOH-Puffer, Diethanolamin/HCl-Puffer, Natriumborat/HCl-Puffer, Glycin/NaOH-Puffer, Natriumcarbonat/Natriumbicarbonat-Puffer, $Na_2HPO_4$/NaOH-Puffer, 2-(Cyclohexylamino)ethansulfonsäure/NaOH-Puffer und 3-(Cyclohexylamino)-1-propansulfonsäure/NaOH-Puffer. Dem Fachmann sind weitere Puffersysteme bekannt, die beispielsweise beschrieben sind in Harris und Angal (Hrsg.), Protein purification methods - a practical approach, IRL Press at Oxford University Press, 1. Aufl. (Nachdruck) 1990.

Das erfindungsgemäße Verfahren kann bei allen Temperaturen durchgeführt werden, bei der die verwendeten Esterasen aktiv sind, beispielsweise bei Temperaturen von 5°C bis 85°C, vorzugsweise von 10°C bis 50°C, besonders bevorzugt von 20°C bis 40°C. Der Fachmann ist mit der Tatsache vertraut, dass Enzyme Temperaturoptima hinsichtlich ihrer Stabilität und/oder ihrer katalytischen Aktivität haben, die gegebenenfalls auch in Abhängigkeit vom verwendeten Lösungsmittel oder Lösungsmittelgemisch variieren können, und kann für jede verwendete Esterase die optimale Temperatur oder den optimalen Temperaturbereich bestimmen. Wird das erfindungsgemäße Verfahren durchgeführt unter Verwendung von Mikroorganismen, welche die wenigstens eine, zur hydrolytischen Spaltung des Polyacrylsäureesters verwendete Esterase produzieren, so können die Temperaturerfordernisse der Mikroorganismen durch den Fachmann bei der Wahl der Verfahrenstemperatur ebenfalls berücksichtigt werden.

Die bereitgestellten Polyacrylsäureester können vollständig verestert sein (d.h. jede Acrylsäuregruppe ist mit einem Alkohol oder Alkoholderivat verestert) oder teilweise verestert sein (d.h. es liegen noch freie Acrylsäuregruppen im Molekül des Polyacrylsäureesters vor). Gemäß bevorzugten Ausführungsformen ist der bereitgestellte Polyacrylsäureester bzw. sind die bereitgestellten Polyacrylsäureester vollständig verestert oder im Wesentlichen vollständig verestert. Im Wesentlichen vollständige Veresterung liegt vor, wenn mindestens 75%, insbesondere mindestens 80%, mindestens 85%, mindestens 90%, mindestens 95%, mindestens 96%, mindestens 97%, mindestens 98% oder mindestens 99% der Acrylsäuregruppen der Polyacrylsäure verestert sind. Im Wesentlichen vollständig veresterte Polyacrylsäureester weisen dementsprechend beispielsweise Veresterungsgrade von 75% bis 100%, 75% bis 99%, 75% bis 98%, 75% bis 97%, 75% bis 96%, 75% bis 95%, 85% bis 100%, 85% bis 99%, 85% bis 98%, 85% bis 97%, 85% bis 96%, 85% bis 95%, 90% bis 100%, 90% bis 99%, 90% bis 98%, 90% bis 97%, 90% bis 96%, 90% bis 95%, 95% bis 100%, 95% bis 99%, 95% bis 98%, 95% bis 97% oder 95% bis 96% auf.

Das erfindungsgemäße Verfahren kann im diskontinuierlichen Betrieb (Chargenbetrieb, Batch-Betrieb) oder im kontinuierlichen Betrieb erfolgen. Im diskontinuierlichen Betrieb werden der wenigstens eine Polyacrylsäureester und die wenigstens eine Esterase bereitgestellt, die Inkubation durchgeführt und das Reaktionsgemisch zu einem gewählten Zeitpunkt aus dem Reaktionsgefäß entnommen und gegebenenfalls der Weiterverarbeitung zugeführt (beispielsweise des Isolierung des Reaktionsproduktes oder der Rückgewinnung der wenigstens einen Esterase). Die Entnahme kann nach vollständig verlaufener Esterspaltung (d.h. nach Einstellung eines Gleichgewichts zwischen Polyacrylsäureester einerseits und Polyacrylsäure oder teilweise veresterter Polyacrylsäure andererseits) oder nach unvollständig verlaufener Esterspaltung erfolgen. Den geeigneten Zeitpunkt der Entnahme der jeweiligen Charge kann der Fachmann über verschiedene Methoden bestimmen, beispielsweise durch vorherige Probenentnahme und -analyse, oder durch kontinuierliche Überwachung des Reaktionsverlaufs mittels geeigneter Parameter, beispielsweise des pH-Wertes oder der Viskosität der Lösung, die sich mit fortschreitender Esterspaltung ändern. Die Charge kann gegebenenfalls auch nach Ablauf einer vorbestimmten Reaktionsdauer entnommen werden. Im diskontinuierlichen Betrieb kann nach erstmaliger Bereitstellung des wenigstens einen Polyacrylsäureesters und der wenigstens einen Esterase vor der endgültigen Entnahme des Reaktionsgemisches unabhängig voneinander eine ein- oder mehrmalige erneute Zugabe von Polyacrylsäureester und/oder Esterase erfolgen. Im kontinuierlichen Betrieb wird nach der erstmaligen Bereitstellung des wenigstens einen Polyacrylsäureesters und der wenigstens einen Esterase zu bestimmten Zeitpunkten ein Teil des Reaktionsgemisches entnommen und das im Reaktionsgefäß verbleibende Reaktionsgemisch durch Zugabe von Polyacrylsäureester und/oder Esterase ergänzt. Die Zeitpunkte können periodisch oder in Abhängigkeit von Messungen des Reaktionsverlaufs gewählt sein. Ebenfalls möglich ist eine kontinuierliche Zugabe des wenigstens einen Polyacrylsäureesters und/oder der wenigstens einen Esterase. Unabhängig davon ist auch eine kontinuierliche Entnahme des Reaktionsgemisches möglich. Dem Fachmann ist bekannt, wie die einzelnen Prozessparameter (z.B. Menge und Zeitpunkt der Zugabe oder Entnahme) optimiert werden, um das gewünschte Reaktionsprodukt zu erhalten. Der kontinuierliche Betrieb kann über unterschiedlich lange Zeiträume erfolgen, beispielsweise über Stunden, Tage, Wochen, Monate oder Jahre, und kann beispielsweise für Reinigungs-, Inspektions- oder Wartungsarbeiten unterbrochen oder abgestellt werden.

Sofern die Isolierung des als Ergebnis der Esterspaltung erhaltenen modifizierten Polymers vorgesehen ist, kann diese über fachübliche Methoden erfolgen, beispielsweise durch chromatographische Methoden, Dialyse, chemische Fällung, Extraktion mit Lösungsmitteln oder Evaporation des im Reaktionsgemisch enthaltenen Lösungsmittels. Soll die wenigstens eine Esterase wiederverwendet werden, so kann diese über fachübliche Methoden abgetrennt werden, vorzugsweise bevor die Isolierung des modifizierten Polymers durch möglicherweise enzymschädigende Methoden erfolgt. Eine Abtrennung der Esterase kann beispielsweise erfolgen durch Affinitätsreinigung mittels Esterase-bindender Moleküle (beispielsweise Antikörper), Dialyse oder Fällung (beispielsweise mit Ammoniumsulfat). Gemäß weiteren Ausführungsformen liegt die wenigstens eine Esterase in immobilisierter Form vor. Dem Fachmann sind verschiedene Verfahren der Immobilisierung bekannt, die beispielsweise beschrieben sind in S. Fukui, A. Tanaka, "Enzymes", in: Ullmanns Encyclopedia of Industrial Chemistry, 5. Aufl., Wiley-VCH, New York, Berlin, 1985; J.E. Prenosil, O.M. Kut, I.J. Dunn, E. Heinzle, "Immobilized Biocatalysts", in: Ullmanns Encyclopedia of Industrial Chemistry, 6. Aufl., VCH, New York, Berlin, 2003, S. 503-554; J.F. Kennedy, J.M.S Cabral, in: H.J.Rehm, G. Reed (Hrsg.), Biotechnology: A comprehensive treatise in 8 vol.; Band 7a, S. 349-404; VCH, Weinheim, 1987. Eine nicht-kovalente Bindung des Enzyms kann beispielsweise erreicht werden durch Markierung des Enzyms mit Biotin und Immobilisierung an einen mit Avidin oder Streptavidin versehenen Träger, Markierung des Enzyms durch Einbau von Histidin-Tags in dessen Aminosäuresequenz und Immobilisierung an einen mit Nickel-Chelat versehenen Träger, Immobilisierung des Enzyms durch gegen das Enzym gerichtete Antikörper (wobei die Bindungsepitope vorzugsweise so gewählt sind, dass die Bindung zwischen Antikörper und Bindungsepitop den Zugang von Substratmolekülen zum aktiven Zentrum nicht oder nicht wesentlich beeinträchtigt), Herstellung von Fusionsproteinen aus dem Enzym und einem Fremdprotein und Immobilisierung des Proteins durch gegen das Fremdprotein gerichtete Antikörper, oder durch adsorptive Anheftung an Mikrosphären (Beads) oder auf Trägermaterialien. Durch die Immobilisierung wird bewirkt, dass die Esterasemoleküle stationär im Reaktionsgefäß verbleiben (beispielsweise auf den Wänden des Reaktionsgefäßes oder auf im Reaktionsgefäß befindlichen Oberflächen) und bei der Entnahme des Reaktionsgemisches nur die Polyacrylsäureester beziehungsweise die durch Esterspaltung modifizierten Polymere entnommen werden. Alternativ können sich die Esterasemoleküle bei Anheftung an Mikrosphären frei flotierend in der Reaktionslösung befinden und mit den Polyacrylsäureestermolekülen in Wechselwirkung treten, werden jedoch durch verschiedene Maßnahmen (beispielsweise Filter oder Verwendung von Wirbelbettreaktoren) am Verlassen des Reaktionsgefäßes zusammen mit den Polyacrylsäureestermolekülen und/oder den Polyacrylsäuremolekülen gehindert. Ebenfalls möglich ist die Entnahme der Mikrosphären zusammen mit den Polyacrylsäureestermolekülen und/oder den Polyacrylsäuremolekülen und eine anschließende Abtrennung, beispielsweise durch Zentrifugation oder Filtration.

Das erfindungsgemäße Verfahren kann durchgeführt werden, bis die in dem Polyacrylsäureester enthaltenen Estergruppen teilweise oder vollständig hydrolytisch gespalten sind, wobei man im Falle einer vollständigen Hydrolyse Polyacrylsäure erhalten würde. Bevorzugt ist die Entnahme nach teilweiser Hydrolyse. In diesem Fall enthalten die anfangs bereitgestellten Polyacrylsäureester noch Estergruppen, die den im Reaktionsverlauf modifizierten Polymeren bestimmte Eigenschaften verliehen. Die modifizierten Polymere können anschließend für weitere Zwecke verwendet werden, beispielsweise als Ausgangsstoffe für weitere chemische Modifikationen.

## 2. Enzyme

Im erfindungsgemäßen Verfahren können Esterasen im allgemeinen und insbesondere auf Esterbindungen einwirkende Enzyme der EC-Klasse 3.1 gemäß der Einteilung des "Nomenclature Committee of the International Union of Biochemistry and Molecular Biology" verwendet werden. Die Einteilung der EC-Klassen ist einzusehen in "Enzyme Nomenclature, 1992 herausgegeben von Academic Press für die "International Union of Biochemistry and Molecular Biology" unter der ISBN 0-12-227164-5, mit nachfolgenden Ergänzungsbänden, oder online im Internet unter http://www.chem.qmul.ac.uk/iupac/jcbn/index.html#6. Bevorzugte Ausführungsformen sind Carbonsäureesterhydrolasen (E.C. 3.1.1), insbesondere Carboxylesterasen (E.C. 3.1.1.1), Triacylglycerinlipasen (E.C. 3.1.1.3) und Cutinasen (E.C. 3.1.1.74).

Für die erfindungsgemäßen Verfahren können auch "funktionale Mutanten" (auch als "funktionale Esterase-Mutanten", "Esterase-Mutanten" oder "Mutanten" bezeichnet) der konkret offenbarten Esterasen verwendet werden, wobei die funktionalen Mutanten, gegebenenfalls nach Isolierung aus einem diese produzierenden Mikroorganismus, selbst Gegenstand der Erfindung sind. Funktionale Mutanten sind im Rahmen der vorliegenden Erfindung Polypeptide, die sich von den konkret offenbarten Esterasen unterscheiden, wie z. B. solche mit einer Identität von weniger als 100% zu den Esterasen gemäß SEQ ID NO:1 bis SEQ ID NO:38 (beispielsweise 40 % bis weniger als 100%, 50% bis weniger als 100%, 60 % bis weniger als 100%, 75% bis weniger als 100%, 85 % bis weniger als 100%, 90% bis weniger als 100%, 95% bis weniger als 100% oder 99% bis weniger als 100%), welche aber weiterhin die gewünschte enzymatische Aktivität besitzen. Die gewünschte enzymatische Aktivität kann beispielsweise nachgewiesen werden durch Spaltung von Polyacrylsäureestern, beispielsweise Polyacrylsäuremethylester oder Polyacrylsäurebutylester. Die gewünschte enzymatische Aktivität wird als vorliegend angesehen, wenn die funktionale Mutante wenigstens 10% der Spaltungsaktivität der Esterase (die beispielsweise bestimmt werden kann als Umsetzung des Substrats pro Zeiteinheit), auf die Bezug genommen wird, aufweist, insbesondere wenigstens 20%, wenigstens 30%, wenigstens 40%, wenigstens 50%, wenigstens 60%, wenigstens 70%, wenigstens 80%, wenigstens 90%, wenigstens 95% oder 100% dieser Spaltungsaktivität. Die gewünschte enzymatische Aktivität liegt selbstverständlich insbesondere auch dann vor, wenn die funktionale Mutante eine höhere Spaltungsaktivität aufweist als die Esterase, auf die Bezug genommen wird, beispielsweise bis zu 105%, 110%, 120%, 150%, 200% oder mehr als 200% der Spaltungsaktivität der betreffenden Esterase. Sie liegt weiterhin auch dann vor, wenn die funktionale Mutante in der Lage ist, ein neues Substrat zu spalten, also einen Polyacrylsäureester, der von der Esterase, auf die Bezug genommen wird, nicht oder unter vergleichbaren Reaktionsbedingungen (wie beispielsweise Temperatur, pH-Wert, Lösungsmittel oder Lösungsmittelzusammensetzung, Salzkonzentration, Substratkonzentration, Enzymkonzentration) nicht gespalten wird.

Unter "funktionalen Mutanten" versteht man erfindungsgemäß insbesondere veränderte Proteine, welche in wenigstens einer der Sequenzpositionen der oben genannten konkreten Sequenzen eine andere als die konkret genannte Aminosäure aufweisen, jedoch trotzdem gewünschte enzymatische Aktivität besitzen. "Funktionale Mutanten" umfassen somit die durch eine oder mehrere Aminosäure-Additionen, -Substitutionen, -Deletionen und/oder - Inversionen erhältlichen veränderten Proteine, wobei die genannten Veränderungen in jeglicher Sequenzposition auftreten können, solange sie zu einer funktionalen Mutante mit dem erfindungsgemäßen Eigenschaftsprofil führen. Funktionale Mutanten liegen insbesondere auch dann vor, wenn die Reaktivitätsmuster zwischen Mutante und unverändertem Polypeptid qualitativ übereinstimmen, d.h. beispielsweise gleiche enzymatische Parameter (wie beispielsweise Substrataffinität, Wechselzahl) vorliegen, jedoch quantitativ unterschiedlich stark ausgeprägt sind. Beispiele für geeignete Substitutionen von Aminosäureresten sind folgende:

| Ursprünglicher Rest | Beispiele der Substitution |
|---|---|
| Ala | Val, Gly, Ser |
| Arg | Lys |
| Asn | Gln; His |
| Asp | Glu |
| Cys | Ser |
| Gln | Asn |

(fortgesetzt)

| Ursprünglicher Rest | Beispiele der Substitution |
|---|---|
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Funktionale Mutanten" im obigen Sinne sind auch Präkursoren der beschriebenen Esterasen sowie funktionale Derivate und Salze der Esterasen. Unter dem Ausdruck "Salze" versteht man sowohl Salze von Carboxylgruppen als auch Säureadditionssalze von Aminogruppen der erfindungsgemäßen Proteinmoleküle. Salze von Carboxylgruppen können in an sich bekannter Weise hergestellt werden und umfassen anorganische Salze, wie zum Beispiel Natrium-, Calcium-, Ammonium-, Eisen- und Zinksalze, sowie Salze mit organischen Basen, wie zum Beispiel Aminen, wie Triethanolamin, Arginin, Lysin, Piperidin und dergleichen. Säureadditionssalze, wie zum Beispiel Salze mit Mineralsäuren, wie Salzsäure oder Schwefelsäure und Salze mit organischen Säuren, wie Essigsäure und Oxalsäure sind ebenfalls Gegenstand der Erfindung. "Funktionale Derivate" von in der Erfindung beschriebenen Esterasen oder erfindungsgemäßer funktionaler Esterase-Mutanten können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

"Funktionale Mutanten" umfassen natürlich auch Esterasen, welche aus anderen Organismen zugänglich sind, sowie natürlich vorkommende Varianten. Beispielsweise lassen sich durch Sequenzvergleiche Bereiche homologer Sequenzregionen festlegen und in Anlehnung an die konkreten Vorgaben der Erfindung funktionale Mutanten ermitteln. Beispiele für Organismen sind *Burkholderia* (beispielsweise *B. gladioli, B. mallei, B. pseudomallei, B. thailandensis, B. cenocepacia, B. aurantiaca, B. vietnamensis, B. dolosa, B. multivorans, B. ambifaria*), Stigmatella (beispielsweise *S. aurantiaca*), *Streptomyces* (beispielsweise *S. ambofaciens, S. coelicolor*), *Saccharopolyspora* (beispielsweise *S. erythraea*), *Mycobacterium* (beispielsweise *M. smegmatis, M. bovis, M. tuberculosis, M. leprae*)

"Funktionale Mutanten" sind außerdem Fusionsproteine, welche eine der oben genannten Polypeptidsequenzen von Esterasen oder davon abgeleiteter funktionaler Mutanten und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nicht-limitierende Beispiele für derartige heterologe Sequenzen sind z.B. Enzyme, Protein A und Immunglobuline, wobei Protein A- und Immunglobulin-Fusionen beispielsweise zur nicht-kovalenten Immobilisierung der funktionalen Mutanten auf Trägern (beispielsweise Mikrosphären) verwendet werden können.

Dem Fachmann sind darüber hinaus Verfahren zur Erzeugung funktionaler Mutanten bekannt. Methoden zur Veränderung von Genen und somit zur Veränderung der durch diese kodierten Proteine sind dem Fachmann seit langem geläufig, beispielsweise die ortsspezifische Mutagenese, bei der gezielt einzelne oder mehrere Nukleotide eines Gens ausgetauscht werden (Trower MK (Hrsg.) 1996; In vitro mutagenesis protocols. Humana Press, New Jersey), die Sättigungsmutagenese, bei der an jeder beliebigen Stelle eines Gens ein Codon für eine beliebige Aminosäure ausgetauscht oder hinzugefügt werden kann (Kegler-Ebo DM, Docktor CM, DiMaio D (1994) Nucleic Acids Res 22:1593; Barettino D, Feigenbutz M, Valcärel R, Stunnenberg HG (1994) Nucleic Acids Res 22:541; Barik S (1995) Mol Biotechnol 3:1), die fehleranfällige Polymerase-Kettenreaktion (error-prone PCR), bei der Nukleotidsequenzen durch fehlerhaft arbeitende DNA-Polymerasen mutiert werden (Eckert KA, Kunkel TA (1990) Nucleic Acids Res 18:3739); das Passagieren von Genen in Mutator-Stämmen, in denen beispielsweise aufgrund defekter DNA-Reperaturmechanismen eine erhöhte Mutationsrate von Nukleotidsequenzen auftritt (Greener A, Callahan M, Jerpseth B (1996) An efficient random mutagenesis technique using an E.coli mutator strain. In: Trower MK (Hrsg.) In vitro mutagenesis protocols. Humana Press, New Jersey), oder DNA-Shuffling, bei dem ein Pool aus nahe verwandeten Genen gebildet und verdaut wird und die Bruchstücke als Templates (Matrizen) für eine Polymerase-Kettenreaktion verwendet werden, bei der durch wiederholte Strangtrennung und Wiederannäherung letztendlich Mosaikgene voller Länge erzeugt werden (Stemmer WPC (1994) Nature 370:389; Stemmer WPC (1994) Proc Natl Acad Sci USA 91:10747). Je nach verwendeter Technik kann der Fachmann völlig zufällige oder auch gezieltere Mutationen in Gene oder auch nicht kodierende Nukleinsäurebereiche (die beispielsweise für die Regulation der Expression wichtig sind) einbringen und anschließend Genbanken erstellen. Die dazu erforderlichen molekularbiologischen Methoden sind dem Fachmann bekannt und beispielsweise beschrieben in Sambrook und Russell, Molecular Cloning. 3. Edition, Cold Spring Harbor Laboratory Press 2001.

Unter Anwendung der sogenannten gerichteten Evolution ("directed evolution"; beschrieben unter anderem in Reetz MT und Jaeger K-E (1999), Topics Curr Chem 200:31; Zhao H, Moore JC, Volkov AA, Arnold FH (1999), Methods for optimizing industrial enzymes by directed evolution, In: Demain AL, Davies JE (Hrsg.) Manual of industrial microbiology and biotechnology. American Society for Microbiology) kann der Fachmann auch in gezielter Weise und auch in großem Maßstab funktionale Mutanten erzeugen. Dabei werden in einem ersten Schritt zunächst Genbanken der jeweiligen Proteine erzeugt, wobei beispielsweise die oben angegebenen Methoden zur Anwendung kommen können. Die Genbanken werden auf geeignete Weise exprimiert, beispielsweise durch Bakterien oder durch Phagen-Display-Systeme. Dem Fachmann sind weiterhin Methoden bekannt, um durch Wahl geeigneter Expressionsvektoren die Lokalisierung des Proteins im Wirt zu steuern, beispielsweise als intrazelluläre Proteine im Cytoplasma, als Membranprotein durch Hinzufügen eines Membranankers, oder als extrazelluläres Protein durch Hinzufügen eines Signalpeptids mit Erkennungsstelle für eine Signalpeptidase. In einem zweiten Schritt werden anschließend Klone, die Proteine mit gewünschter Eigenschaft exprimieren, selektiert oder gescreent. Bei der Selektion überleben nur die Klone, die Proteine mit gewünschten Eigenschaften exprimieren, da diese den Wirtsorganismen einen Überlebensvorteil verschaffen (beispielsweise Enzyme, welche die Verwertung bestimmter Substrate ermöglichen oder das Wachstum bei bestimmten Temperaturen). Beim Screening (auch als Screenen bezeichnet) überleben alle Klone, so dass über geeignete Assays bestimmt wird, welcher der Klone ein Protein mit gewünschter Eigenschaft exprimiert. Dem Fachmann ist bekannt, wie Assays für den jeweiligen Zweck geeignet gestaltet werden. Beispielsweise kann bei der Suche nach Proteinen mit bestimmten Bindungseigenschaften auf Wirtsorganismen gescreent werden, welche die fraglichen Proteine auf ihrer Oberfläche exprimieren und auf einem mit dem Bindungspartner beschichteten Substrat haften (im Gegensatz zu Wirtsorganismen, die nicht funktionsfähige Proteine exprimieren); bei der Suche nach funktionalen Mutanten mit katalytischen Eigenschaften können Wirtsorganismen beispielsweise in Mikrotierplatten mit substrathaltigem Medium oder auf substrathaltigen Agarplatten kultiviert werden und das Vorliegen von funktionalen Mutanten (gegebenenfalls nach Lyse der Wirtsorganismen, um Kontakt mit dem Medium zu ermöglichen) beispielsweise durch Farbreaktionen nach Umsatz des Substrat angezeigt werden. In diesem Zusammenhang können automatisierte Systeme (z.B. Pipettierroboter für Mikrotiterplatten, Screeningroboter, Bilderkennungssysteme zur Identifizierung von Kolonien auf Agarplatten) zum Einsatz kommen, um ein Screening mit hohem Durchsatz (High Throughput Screening) zu ermöglichen. Die betreffenden Gene von Wirtsorganismen, die funktionale Mutanten mit Eigenschaften exprimieren, welche den gewünschten Eigenschaften weitgehend entsprechen, werden einer weiteren Mutationsrunde unterworfen. Die Schritte der Mutation und der Selektion oder des Screenings andererseits können iterativ solange wiederholt werden, bis die vorliegenden funktionalen Mutanten die gewünschten Eigenschaften in ausreichendem Maße aufweisen.

Durch ein iteratives Vorgehen ist es möglich, gezielt auf Proteine mit gewünschten Eigenschaften zu screenen, obwohl die Einführung von vielen Mutationen in eine Proteinsequenz mit höherer Wahrscheinlichkeit zum Verlust einer Funktion führt als zum Erwerb bzw. der Verbesserung einer erwünschten Funktion. Im Rahmen des iterativen Vorgehens geht man von einem Referenzprotein aus, beispielsweise einem Wildtyp-Protein, und erstellt auf der Grundlage von dessen Nukleotidsequenz eine Genbibliothek mit Mutationen. Dabei wird die Mutationsrate auf Nukleotidebene so gewählt, dass nach Expression der mutierten Nukleinsäuren nur eine relativ geringe Anzahl von Aminosäuren in den translatierten Peptiden oder Proteinen mutiert sind, beispielsweise 1 bis 3 Aminosäuren. Die erhaltenen mutierten Peptide oder Proteine werden auf Vertreter mit gewünschten Eigenschaften gescreent (beispielsweise eine höhere katalytische Aktivität für ein oder mehrere Substrate, ein erweitertes oder verändertes Substratspektrum, eine verbesserte Stabilität bei erhöhten oder veränderten Temperaturen oder pH-Werten oder in bestimmten Lösungsmitteln). Auf der Grundlage der Sequenzen von gefundenen Peptiden oder Proteinen mit solchen gewünschten Eigenschaften (die gegebenenfalls nur schwach ausgeprägt sind) wird eine zweite Genbibliothek erstellt, in der wiederum eine relativ geringe Rate von Mutationen eingeführt wird, und die daraus translatierten Proteine werden erneut auf die gewünschte Eigenschaft gescreent. Dieser Zyklus aus dem Erstellen einer mutierten Genbibliothek und dem Screenen der daraus exprimierten Peptide oder Proteine auf gewünschte Eigenschaften kann beliebig oft wiederholt werden. Einerseits wird durch die pro Zyklus verwendete geringe Mutationsrate vermieden, dass praktisch alle Proteine aufgrund zu vieler Mutationen in deren Aminosäuresequenz einen Funktionsverlust aufweisen, andererseits können durch die iterative Wiederholung von Mutation und Selektion günstige Mutationen akkumuliert werden und so letztendlich mit hohen Erfolgsaussichten Proteine bereitgestellt werden, welche die gewünschten, deutlich verbesserten Eigenschaften aufweisen. Durch die Sequenzanalyse von Mutanten mit verbesserten Eigenschaften erhält der Fachmann darüber hinaus Sequenzinformation, die ihm anzeigen, welche Sequenzpositionen oder Sequenzbereiche eines Peptids oder Proteins für eine gewünschte Eigenschaft wichtig sind. Basierend auf diesen Informationen kann er Mutationen bewusst an diesen Stellen oder in diesen Bereichen setzen und dementsprechend noch zielgerichteter Mutanten mit gewünschten Eigenschaften erhalten. Beispiele in der Literatur für die Erzeugung von Proteinen mit gewünschten Eigenschaften, aus denen auch dafür in Frage kommende Methoden der Erzeugung von Mutationen ersichtlich werden, sind beschrieben in Zhao und Arnold, Protein Engineering 12:47-53 (1999) oder May et al., Nature Biotechnology 18:317-320 (2000):

Weitere erfindungsgemäß mit umfasste "funktionale Mutanten" sind Homologe zu den konkret offenbarten Esterasen. Diese besitzen wenigstens 40 % oder wenigstens 50%, oder wenigstens 60 %, wie z.B. wenigstens 75% oder insbesondere wenigstens 85 %, wie z.B. 90%, 95% oder 99%, Homologie zu einer der konkret offenbarten Sequenzen, z.B. berechnet nach dem Algorithmus von Pearson und Lipman, Proc. Natl. Acad, Sci. (USA) 85(8), 1988, 2444-2448. Eine prozentuale Homologie eines erfindungsgemäßen homologen Polypeptids bedeutet insbesondere prozentuale Identität der Aminosäurereste bezogen auf die Gesamtlänge einer der hierin konkret beschriebenen Aminosäuresequenzen.

Unter einer "abgeleiteten" Aminosäuresequenz wird erfindungsgemäß, wenn keine anderen Angaben gemacht werden, eine Sequenz verstanden, die mit der Ausgangssequenz eine Identität von mindestens 80% oder mindestens 90%, insbesondere etwa 91%, 92%, 93%, 94%, 95%, 96%, 97%, 98% und 99% aufweist. Gegenstand der Erfindung sind beispielsweise die von allen hier offenbarten Aminosäuresequenzen abgeleiteten Aminosäuresequenzen.

Unter "Identität" bzw. "Homologie" zwischen zwei Sequenzen wird Identität der Aminosäurereste über die jeweils gesamte Sequenzlänge verstanden, wie z.B. die Identität, die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:

Multiple alignment parameters:

| | |
|---|---|
| Gap opening penalty | 10 |
| Gap extension penalty | 10 |
| Gap separation penalty range | 8 |
| Gap separation penalty | off |
| % identity for alignment delay | 40 |
| Residue specific gaps | off |
| Hydrophilic residue gap | off |
| Transition weighing | 0 |
| | |
| Pairwise alignment parameters: | |
| FAST algorithm | on |
| K-tuplesize | 1 |
| Gap penalty | 3 |
| Window size | 5 |
| Number of best diagonals | 5 |

Der Fachmann kann diese Werte in Abhängigkeit von den konkret zu vergleichenden Sequenzen falls erforderlich weiter variieren.

Im Falle einer möglichen Proteinglykosylierung umfassen die erfindungsgemäßen funktionalen Mutanten Proteine des oben bezeichneten Typs in deglykosylierter bzw. glykosylierter Form sowie durch Veränderung des Glykosylierungsmusters erhältliche abgewandelte Formen.

Homologe der beschriebenen Esterasen oder von deren erfindungsgemäßen funktionalen Mutanten können durch Screening kombinatorischer Banken von Mutanten, wie z.B. Verkürzungsmutanten, identifiziert werden. Beispielsweise kann eine Bank von Peptid-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al. (1984) Science 198:1056; Ike et al. (1983) Nucleic Acids Res. 11:477).

Im Rahmen der Untersuchungen, die der vorliegenden Erfindung zugrunde lagen, stellten die Erfinder überraschend fest, dass Esterbindungen von Polyacrylsäureestern, also polymerer Substrate, von Esterasen gespalten werden können. Dabei können Esterasen aus verschiedenen Organismen verwendet werden, wobei als nicht-limitierende Beispiele genannt werden können: *Burkholderia* (beispielsweise *B. gladioli, B. mallei, B. pseudomallei, B. thailandensis, B. cenocepacia, B. aurantiaca, B. vietnamensis, B. dolosa, B. multivorans, B. ambifaria*), *Stigmatella* (beispielsweise *S. aurantiaca*), *Streptomyces* (beispielsweise *S. ambofaciens, S. coelicolor), Saccharopolyspora* (beispielsweise *S. erythraea*), *Mycobacterium* (beispielsweise *M. smegmatis, M. bovis, M. tuberculosis, M. leprae*).

Zur Durchführung des erfindungsgemäßen Verfahrens bevorzugte Esterasen sind auf Esterbindungen einwirkende Enzyme der EC-Klasse 3.1, insbesondere Carbonsäureesterhydrolasen (EC 3.1.1). Besonders bevorzugt sind Carboxylesterasen, Triacylglycerinlipasen und Cutinasen. Weitere besondere Ausführungsformen stellen Esterasen der Familie VIII (insbesondere Esterase B von *Burkholderia gladioli* gemäß SEQ ID NO:1 und deren funktionale Mutanten) sowie Typ C Esterasen (insbesondere Esterase C von *Burkholderia gladioli* gemäß SEQ ID NO:2 und deren funktionale Mutanten) dar. Weitere, nicht-limitierende Beispiele für verwendbare Esterasen sind die Sequenzen gemäß SEQ ID NO:7, SEQ ID NO:8, SEQ ID NO:9, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:19, SEQ ID NO:20, SEQ ID NO:21, SEQ ID NO:21, SEQ ID NO:22, SEQ ID NO:23, SEQ ID NO:24, SEQ ID NO:25, SEQ ID NO:26, SEQ ID NO:27, SEQ ID NO: 28, SEQ ID NO:29, SEQ ID NO:30, SEQ ID NO:31, SEQ ID NO:32, SEQ ID NO:33, SEQ ID NO:34, SEQ ID NO:35, SEQ ID NO:36, auf deren Grundlage wiederum funktionale Mutanten erzeugt oder abgeleitet werden können.

Im Rahmen der vorliegenden Erfindung wurden somit weiterhin funktionale Mutanten bekannter Esterasen hergestellt, die ebenfalls in den erfindungsgemäßen Verfahren verwendet werden können und darüber hinaus - gegebenenfalls nach Isolierung aus einem die funktionalen Mutanten produzierenden Mikroorganismus - selbst Gegenstand der Erfindung sind.

Diese funktionalen Mutanten betreffen insbesondere Esterasen mit gegenüber dem bekannten Wildtyp veränderter Aminosäuresequenz, wobei durch die veränderte Sequenz eine neue Esterase bereitgestellt wird, bei der gegebenenfalls der Zugang von polymeren Substraten zum katalytisch aktiven Zentrum ermöglicht oder erleichtert wird, und/oder die Stabilität der Esterase erhöht wird, beispielsweise die Stabilität bei bestimmten pH-Werten oder in verschiedenen Lösungsmitteln oder Lösungsmittelgemischen Gegenstand der Erfindung sind somit unter anderem funktionale Mutanten von Esterasen, bei denen Strukturelemente verändert oder entfernt werden, die den Zugang von Substraten zum aktiven Zentrum verhindern oder erschweren, beispielsweise Loops. Geeignete Bereiche für eine Loop-Kürzung bei einer Esterase gemäß SEQ ID NO:1 (EstB von *B.gladioli*) stellen beispielsweise die Bereiche Glu246 bis Arg 258 und Gly312 bis 323. Dabei können Loop-Kürzungen durchgeführt werden durch Entfernen einer oder mehrerer Aminosäuren, wobei im Fall des Entfernens mehrerer Aminosäuren wiederum eine oder mehrere in SEQ ID NO:1 benachbart oder nicht benachbart gelegene Aminosäuren entfernt werden können. Gegenstand der Erfindung sind weiterhin funktionale Mutanten von Esterasen, bei denen der Zugang zum katalytisch aktiven Zentrum (hier auch als aktives Zentrum bezeichnet), der beispielsweise tunnel-, trichter-, furchen- oder spaltenförmig ausgebildet sein kann, für Moleküle aus der Umgebung der Esterase besser zugänglich gemacht wurde. Dies kann beispielsweise erfolgen, indem Aminosäuren mit großen Seitenketten, die Bestandteil des Zugangs zum katalytisch aktiven Zentrum sind, entfernt oder durch Aminosäuren mit kleineren Seitenketten ersetzt werden (beispielsweise Alanin durch Glycin, Valin durch Alanin oder Glycin, Leucin durch Valin, etc.). Dadurch wird der räumliche Zugang von Teilbereichen von Polyacrylsäureestern, die Makromoleküle mit großer Raumausfüllung darstellen, zum katalytischen Zentrum ermöglicht oder erleichtert. Mit Hinblick auf die Ladung von Polyacrylsäureestern, die aufgrund noch freier Carboxylgruppen eine negative Ladung aufweisen können, kann dies weiterhin geschehen durch Austausch von sauren Aminosäuren im Zugangsbereich des aktiven Zentrums durch neutrale oder basische Aminosäuren. Mit Hinblick auf Polyacrylsäureester, die beispielsweise aufgrund von mit basischen Gruppen substituierten Alkoholbestandteilen des Esters eine eher positive Ladung aufweisen, kann der Zugang zum katalytisch aktiven Zentrum entsprechend durch Austausch von basischen Aminosäuren im Zugangsbereich des katalytisch aktiven Zentrums durch weniger basische, gegebenenfalls neutrale oder saure Aminosäuren erleichtert oder ermöglicht werden. Beispielsweise sind geeignete Aminosäuren, bei deren Austausch durch Aminosäuren mit kleineren Seitenketten der Zugang zum aktiven Zentrum verbessert werden sollte, Tyr181, Trp348 undTrp149 im Falle von SEQ ID N0:1. Bei SEQ ID NO:2 kommen dafür beispielsweise Leu163, Val213 und Pro168 in Frage,

wobei insbesondere im Falle von Pro168 durch einen Austausch durch Ala oder Gly auch die Flexibilität des zugehörigen Loops erhöht werden kann, wodurch ein größeres Substrat zugelassen werden kann. Ein weiterer Gegenstand der Erfindung sind funktionale Mutanten von Esterasen mit erhöhter Stabilität, beispielsweise erhöhter pH-, Temperatur- oder Lösungsmittelstabilität. Eine solche erhöhte Stabilität kann beispielsweise erreicht werden durch Aminsäureaustausche, mit denen intramolekulare Wechselwirkungen innerhalb eines Proteinmoleküls erstmals möglich werden oder verstärkt werden, durch die instabile Loops des Proteinmoleküls besser fixiert werden oder einzelne Proteindomänen einen besseren Zusammenhalt erhalten.

Dementsprechend ist sind besondere Ausführungsformen der vorliegenden Erfindung funktionale Mutanten von EstB gemäß SEQ ID NO:1, bei denen über dem katalytisch aktiven Zentrum liegende Loops durch mehr oder weniger vollständige Deletion des Loop-bildenden Aminosäuren beseitigt oder verkürzt wurden. In Fig. 1 ist ein Molekülmodell von EstB gemäß SEQ ID NO:1 gezeigt, bei dem zwei Loops identifiziert wurden, die zwischen dem katalytisch aktiven Zentrum und dem Außenmedium der EstB liegen.

Weitere besondere Ausführungsformen der vorliegenden Erfindung sind dementsprechend funktionale Mutanten von EstC gemäß SEQ ID NO:2. Im Rahmen der vorliegenden Erfindung wurde ein Strukturmodell von EstC verwendet, auf dessen Grundlage funktionale Mutanten hergestellt wurden. In diesem Zusammenhang wurden von den Erfindern Proteinbereiche identifiziert, die den Zugang zum katalytisch aktiven Zentrum auskleiden. Anschließend wurden durch ortsspezifische Mutagenese gezielt einzelne oder mehrere Aminosäuren von SEQ ID NO:2 verändert, um den Zugang zu erweitern und einen besseren Zutritt von großen, polymeren Substraten zu ermöglichen. Als eine den Zugang zum aktiven Zentrum auskleidende Aminosäure wurde Phe138 (d.h. das Phenylalanin an der Aminosäureposition 138) identifiziert (Fig. 2). In Fig. 2 ist zu erkennen, dass Phe138 mit seiner Seitenkette zwischen den Aminosäuren Ser112, Asp242 und His275 des katalytisch aktiven Zentrums und dem Außenmedium des Proteins liegt. Durch den Austausch von Phe138 mit Alanin (Phe138Ala) wird angesichts der kleineren Seitengruppe von Alanin der Zugang raumbeanspruchender Substratmoleküle, beispielsweise Polyacrylsäureester, zum katalytisch aktiven Zentrum erleichtert (entsprechend der Mutation 1 K22 in Fig. 5). Die physikochemischen Eigenschaften der Proteinoberfläche an der Position 138 bleiben im Wesentlichen gleich, da eine hydrophobe Aminosäure durch eine andere hydrophobe Aminosäure ersetzt wird. Als weitere, den Zugang zum katalytisch aktiven Zentrum behindernde Aminosäure wurde erfindungsgemäß Leu193 identifiziert (Fig. 4), die bei einer Ausführungsform durch Alanin ersetzt wurde (entsprechend der Mutation 2K20 in Fig. 5). Als weiteres Beispiel ist Thr188 zu nennen, das in einer Randlage des Zugangs zum katalytisch aktiven Zentrum liegt und den Zutritt von Substratmolekülen noch zu einem gewissen Grad beeinflussen kann (Fig. 4). Gemäß einer Ausführungsform wird Thr188 durch Serin ersetzt, beispielsweise in der Dreifachmutation Phe138Ala, Leu193Ala, Thr188Ser (Fig. 4). Von der Erfindung umfasst sind beliebige Kombinationen dieser oder der in Fig. 5 angegebenen Mutationen. Der Fachmann ist anhand der hier aufgezeigten Vorgehensweise in der Lage, geeignete Positionen für Aminosäureaustausche auch bei zu EstC homologen Proteinen zu ermitteln. Beispielsweise kann er unter Verwendung des Programms "Tripos" von Sybly (St. Louis, MO, USA) Überlagerungen der hier offenbarten Struktur von EstC und einer Esterase mit ähnlicher 3-D-Struktur vornehmen und bei dieser geeignete Positionen ermitteln, so dass die vorliegende Erfindung nicht auf die hier konkret offenbarten Proteine und Mutationen beschränkt ist.

## 3. Nukleinsäuren

Gegenstand der Erfindung sind weiterhin die kodierenden Nukleinsäuresequenzen für die oben beschriebenen funktionalen Esterase-Mutanten, wie insbesondere gemäß SEQ ID NO: 54 bis SEQ ID NO:57.

Alle erfindungsgemäßen Nukleinsäuresequenzen (einzel- und doppelsträngige DNA- und RNA-Sequenzen, wie z.B. cDNA und mRNA) sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragments der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Unter einer "abgeleiteten" Nukleinsäuresequenz wird erfindungsgemäß, wenn keine anderen Angaben gemacht werden, eine Sequenz verstanden, die mit der Ausgangssequenz eine Identität von mindestens 80% oder mindestens 90%, insbesondere etwa 91%, 92%, 93%, 94%, 95%, 96%, 97%, 98% und 99% aufweist. Gegenstand der Erfindung sind beispielsweise alle von den hier offenbarten Nukleinsäuresequenzen abgeleiteten Nukleinsäuresequenzen.

Unter "Identität" zwischen zwei Nukleinsäuren wird die Identität der Nukleotide über die jeweils gesamte Nukleinsäurelänge verstanden, insbesondere die Identität, die durch Vergleich mit Hilfe der Vector NTI Suite 7.1 Software der Firma Informax (USA) unter Anwendung der Clustal Methode (siehe oben) ermittelt wird.

Gegenstand der Erfindung sind auch Nukleinsäuresequenzen, kodierend für eine der obigen Esterasen und deren funktionalen Mutanten, welche z.B. unter Verwendung künstlicher Nukleotidanaloga zugänglich sind.

Die Erfindung betrifft sowohl isolierte Nukleinsäuremoleküle, welche für erfindungsgemäße Esterasen oder enzymatisch aktive Abschnitte davon kodieren, sowie Nukleinsäurefragmente, die z.B. als Hybridisierungssonden oder Primer zur Identifizierung oder Amplifizierung von erfindungsgemäßer kodierenden Nukleinsäuren verwendet werden können.

Die erfindungsgemäßen Nukleinsäuremoleküle können zudem nicht-translatierte Sequenzen vom 3'- und/oder 5'-Ende des kodierenden Genbereichs enthalten.

Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Ein erfindungsgemäßes Nukleinsäuremolekül kann mittels molekularbiologischer Standard-Techniken und der erfindungsgemäß bereitgestellten Sequenzinformation isoliert werden. Beispielsweise kann cDNA aus einer geeigneten cDNA-Bank isoliert werden, indem eine der konkret offenbarten vollständigen Sequenzen oder ein Abschnitt davon als Hybridisierungssonde und Standard-Hybridisierungstechniken (wie z.B. beschrieben in Sambrook, J., Fritsch, E.F. und Maniatis, T. Molecular Cloning: A Laboratory Manual. 2. Aufl., Cold Spring Harbor Laboratory, Cold Spring Harbor Laboratory Press, Cold Spring Harbor, NY, 1989) verwendet werden. Überdies lässt sich ein Nukleinsäuremolekül, umfassend eine der erfindungsgemäßen Sequenzen oder ein Abschnitt davon, durch Polymerasekettenreaktion isolieren, wobei die Oligonukleotidprimer, die auf der Basis dieser Sequenz erstellt wurden, verwendet werden. Die so amplifizierte Nukleinsäure kann in einen geeigneten Vektor kloniert werden und durch DNA-Sequenzanalyse charakterisiert werden. Die erfindungsgemäßen Oligonukleotide können ferner durch Standard-Syntheseverfahren, z.B. mit einem automatischen DNA-Synthesegerät, hergestellt werden.

Die Erfindung umfasst weiterhin die zu den konkret beschriebenen Nukleotidsequenzen komplementären Nukleinsäuremoleküle oder einen Abschnitt davon.

Die erfindungsgemäßen Nukleotidsequenzen ermöglichen die Erzeugung von Sonden und Primern, die zur Identifizierung und/oder Klonierung von homologen Sequenzen in anderen Zelltypen und Organismen verwendbar sind. Solche Sonden bzw. Primer umfassen gewöhnlich einen Nukleotidsequenzbereich, der unter stringenten Bedingungen an mindestens etwa 12, vorzugsweise mindestens etwa 25, wie z.B. etwa 40, 50 oder 75 aufeinanderfolgende Nukleotide eines Sense-Stranges einer erfindungsgemäßen Nukleinsäuresequenz oder eines entsprechenden Antisense-Stranges hybridisiert.

Weitere erfindungsgemäße Nukleinsäuresequenzen sind abgeleitet von kodierenden Sequenzen der erfindungsgemäßen Esterasen und unterscheiden sich davon durch Addition, Substitution, Insertion oder Deletion einzelner oder mehrerer Nukleotide, kodieren aber weiterhin für Esterasen mit der gewünschten enzymatischen Aktivität.

Erfindungsgemäß umfasst sind auch solche Nukleinsäuresequenzen, die sogenannte stumme Mutationen umfassen oder entsprechend der Codon-Nutzung eines speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon. Gegenstand sind ebenso durch konservative Nukleotid-Substitutionen (d.h. die betreffende Aminosäure wird durch eine Aminosäure gleicher Ladung, Größe, Polarität und/oder Löslichkeit ersetzt) erhältliche Sequenzen.

Gegenstand der Erfindung sind auch die durch Sequenzpolymorphismen von den konkret offenbarten Nukleinsäuren abgeleiteten Moleküle. Diese genetischen Polymorphismen können zwischen Individuen innerhalb einer Population aufgrund der natürlichen Variation existieren. Diese natürlichen Variationen bewirken üblicherweise eine Varianz von 1 bis 5 % in der Nukleotidsequenz eines Gens.

Weiterhin umfasst die Erfindung auch Nukleinsäuresequenzen, welche mit oben genannten kodierenden Sequenzen hybridisieren oder dazu komplementär sind. Diese Polynukleotide lassen sich bei Durchmusterung von genomischen oder cDNA-Banken auffinden und gegebenenfalls daraus mit geeigneten Primern mittels PCR vermehren und anschließend beispielsweise mit geeigneten Sonden isolieren. Eine weitere Möglichkeit bietet die Transformation geeigneter Mikroorganismen mit erfindungsgemäßen Polynukleotiden oder Vektoren, die Vermehrung der Mikroorganismen und damit der Polynukleotide und deren anschließende Isolierung. Darüber hinaus können erfindungsgemäße Polynukleotide auch auf chemischem Wege synthetisiert werden.

Unter der Eigenschaft, an Polynukleotide "hybridisieren" zu können, versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen zu 70-100%, insbesondere zu 90-100%, wie z.B. 95%, 96%, 97%, 98% oder 99%, komplementär sein. Die Eigenschaft komplementärer Sequenzen, spezifisch aneinander binden zu können, macht man sich beispielsweise in der Northern- oder Southern-Blot-Technik oder bei der Primerbindung in PCR oder RT-PCR zunutze. Üblicherweise werden dazu Oligonukleotide ab einer Länge von 30 Basenpaaren eingesetzt. Unter stringenten Bedingungen versteht man beispielsweise in der Northern-Blot-Technik die Verwendung einer 50 - 70 °C, vorzugsweise 60 - 65 °C warmen Waschlösung, beispielsweise 0,1x SSC-Puffer mit 0,1% SDS (20x SSC: 3M NaCl, 0,3M Na-Citrat, pH 7,0), zur Elution unspezifisch hybridisierter cDNA-Sonden oder Oligonukleotide. Dabei bleiben, wie oben erwähnt, nur in hohem Maße komplementäre Nukleinsäuren aneinander gebunden. Die Einstellung stringenter Bedingungen ist dem Fachmann bekannt und ist z.B. in Ausubel et al., Current Protocols in Molecular Biology, John Wiley & Sons, N.Y. (1989), 6.3.1-6.3.6. beschrieben.

## 4. Expressionskonstrukte und Vektoren

Gegenstand der Erfindung sind außerdem Expressionskonstrukte, enthaltend unter der genetischen Kontrolle regulativer Nukleinsäuresequenzen eine für eine erfindungsgemäße Esterase oder eine funktionale Mutante kodierende Nukleinsäuresequenz; sowie Vektoren, umfassend wenigstens eines dieser Expressionskonstrukte.

Vorzugsweise umfassen solche erfindungsgemäßen Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz. Unter einer "operativen Verknüpfung" versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Enhancer, Polyadenylierungssignale und dergleichen. Weitere regulative Elemente umfassen selektierbare Marker, Amplifikationssignale, Replikationsursprünge und dergleichen. Geeignete regulatorische Sequenzen sind z.B. beschrieben in Goeddel, Gene Expression Technology: Methods in Enzymology 185, Academic Press, San Diego, CA (1990).

Zusätzlich zu den artifiziellen Regulationssequenzen kann die natürliche Regulationssequenz vor dem eigentlichen Strukturgen noch vorhanden sein. Durch genetische Veränderung kann diese natürliche Regulation gegebenenfalls ausgeschaltet und die Expression der Gene erhöht oder erniedrigt werden. Das Genkonstrukt kann aber auch einfacher aufgebaut sein, das heißt es werden keine zusätzlichen Regulationssignale vor das Strukturgen insertiert und der natürliche Promotor mit seiner Regulation wird nicht entfernt. Stattdessen wird die natürliche Regulationssequenz so mutiert, dass keine Regulation mehr erfolgt und die Genexpression gesteigert oder verringert wird. Die Nukleinsäuresequenzen können in einer oder mehreren Kopien im Genkonstrukt enthalten sein.

Beispiele für brauchbare Promotoren sind: cos-, tac-, trp-, tet-, trp-tet-, Ipp-, lac-, Ipp-lac-, laclq-, T7-, T5-, T3-, gal-, trc-, ara-, SP6-, lambda-PR- oder lambda-PL-Promotor, die vorteilhafterweise in gram-negativen Bakterien Anwendung finden; sowie die gram-positiven Promotoren amy und SPO2, die Hefepromotoren ADC1, MFalpha, AC, P-60, CYC1, GAPDH oder die Pflanzenpromotoren CaMV/35S, SSU, OCS, lib4, usp, STLS1, B33, not oder der Ubiquitin- oder Phaseolin-Promotor. Besonders bevorzugt ist die Verwendung induzierbarer Promotoren, wie z.B. licht- und insbesondere temperaturinduzierbarer Promotoren, wie der $P_rP_l$-Promotor. Prinzipiell können alle natürlichen Promotoren mit ihren Regulationssequenzen verwendet werden. Darüber hinaus können auch synthetische Promotoren vorteilhaft verwendet werden.

Die genannten regulatorischen Sequenzen sollen die gezielte Expression der Nukleinsäuresequenzen und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.
Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Die Herstellung einer Expressionskassette erfolgt durch Fusion eines geeigneten Promotors mit einer geeigneten kodierenden Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Als Beispiele für geeignete Expressionsvektoren können genannt werden:

Übliche Fusionsexpressionsvektoren, wie pGEX (Pharmacia Biotech Inc; Smith, D.B. und Johnson, K.S. (1988) Gene 67:31-40), pMAL (New England Biolabs, Beverly, MA) und pRIT 5 (Pharmacia, Piscataway, NJ), bei denen Glutathion-S-Transferase (GST), Maltose E-bindendes Protein bzw. Protein A an das rekombinante Zielprotein fusioniert wird.

Nicht-Fusionsprotein-Expressionsvektoren wie pTrc (Amann et al., (1988) Gene 69:301-315) und pET 11d (Studier et al. Gene Expression Technology: Methods in Enzymology 185, Academic Press, San Diego, Kalifornien (1990) 60-89).
Hefe-Expressionsvektor zur Expression in der Hefe *S. cerevisiae*, wie pYepSec1 (Baldari et al., (1987) Embo J. 6: 229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie filamentösen Pilzen, eignen, umfassen diejenigen, die eingehend beschrieben sind in: van den Hondel, C.A.M.J.J. & Punt, P.J. (1991) "Gene transfer systems and vector development for filamentous fungi, in: Applied Molecular Genetics of Fungi, J.F. Peberdy et al., Hrsg., S. 1-28, Cambridge University Press: Cambridge.
Baculovirus-Vektoren, die zur Expression von Proteinen in gezüchteten Insektenzellen (bspw. Sf9-Zellen) verfügbar sind, umfassen die pAc-Reihe (Smith et al., (1983) Mol. Cell Biol.. 3:2156-2165) und die pVL-Reihe (Lucklow und Summers (1989) Virology 170:31-39).

Pflanzen-Expressionsvektoren, wie solche, die eingehend beschrieben sind in: Becker, D., Kemper, E., Schell, J. und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721.

Säugetier-Expressionsvektoren, wie pCDM8 (Seed, B. (1987) Nature 329:840) und pMT2PC (Kaufman et al. (1987) EMBO J. 6:187-195).

Weitere geeignete Expressionssysteme für prokaryontische und eukaryotische Zellen sind in Kapitel 16 und 17 von Sambrook, J., Fritsch, E.F. und Maniatis, T., Molecular cloning: A Laboratory Manual, 2. Auflage, Cold Spring Harbor Laboratory, Cold Spring Harbor Laboratory Press, Cold Spring Harbor, NY, 1989 beschrieben.

## 5. Rekombinante Wirtsorganismen

Mit Hilfe der erfindungsgemäßen Vektoren sind rekombinante Organismen herstellbar, welche beispielsweise mit wenigstens einem erfindungsgemäßen Vektor transformiert sind und zur Produktion der erfindungsgemäßen Domänen oder Polypeptide eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden, wie beispielsweise Co-Präzipitation, Protoplastenfusion, Elektroporation, chemische Transformation, retrovirale Transfektion und dergleichen, verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, oder Sambrook et al. Molecular Cloning: A Laboratory Manual. 2. Aufl., Cold Spring Harbor Laboratory, Cold Spring Harbor Laboratory Press, Cold Spring Harbor, NY, 1989 beschrieben.

Als Wirtsorganismen sind prinzipiell alle Organismen geeignet, die eine Expression der erfindungsgemäßen Nukleinsäuren, ihrer Allelvarianten, ihrer Ableitungen, die funktionale Mutanten kodieren, oder ihrer Derivate ermöglichen. Unter Wirtsorganismen sind beispielsweise Bakterien, Pilze, Hefen, pflanzliche oder tierische Zellen zu verstehen. Bevorzugte Organismen sind Bakterien, wie solche der Gattungen *Escherichia,* wie z. B. *Escherichia coli,* Streptomyces, *Bacillus, Pseudomonas* oder *Burkholderia,* eukaryotische Mikroorganismen, wie *Saccharomyces cerevisiae, Aspergillus*, höhere eukaryotische Zellen aus Tieren oder Pflanzen, beispielsweise Sf9-, CHO- oder HEK293-Zellen, wobei im Rahmen der vorliegenden Erfindung einzelne oder zu Zellhaufen aggregierte oder gewachsene Zellen aus höheren eukaryotischen Lebensformen wie Tieren oder Pflanzen ebenfalls als Mikroorganismen bezeichnet werden.
Die Selektion erfolgreich transformierter Organismen kann durch Markergene erfolgen, die ebenfalls im Vektor oder in der Expressionskassette enthalten sind. Beispiele für solche Markergene sind Gene für Antibiotikaresistenz und für Enzyme, die eine farbgebende Reaktion katalysieren, die ein Anfärben oder eine Fluoreszenz der transformierten Zelle bewirkt. Diese können dann mittels automatischer Zellsortierung selektiert werden. Erfolgreich mit einem Vektor transformierte Mikroorganismen, die ein entsprechendes Antibiotikaresistenzgen (z.B. G418 oder Hygromycin) tragen, lassen sich durch entsprechende Antibiotikaenthaltende Medien oder Nährböden selektieren. Markerproteine, die an der Zelloberfläche präsentiert werden, können zur Selektion mittels Affinitätschromatographie genutzt werden.

Gegenstand der Erfindung sind weiterhin Verfahren zur rekombinanten Herstellung erfindungsgemäßer Esterasen oder funktioneller, enzymatisch aktiver Fragmente davon, wobei man einen Esterase-produzierenden rekombinanten Wirtsorganismus kultiviert, gegebenenfalls die Expression der Esterase induziert und diese aus der Kultur isoliert. Die Esterase oder die Esterasen können so auch in großtechnischem Maßstab produziert werden, falls dies erwünscht ist.
Der rekombinante Wirt kann nach bekannten Verfahren kultiviert und fermentiert werden. Bakterien können beispielsweise in TB- oder LB-Medium und bei einer Temperatur von 20 bis 40°C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Die Zellen werden dann, falls die Esterasen nicht in das Kulturmedium sezerniert werden, aufgeschlossen und das Produkt nach bekannten Proteinisolierungsverfahren aus dem Lysat gewonnen. Die Zellen können wahlweise durch hochfrequenten Ultraschall, durch hohen Druck, wie z.B. in einer French-Druckzelle (French Press), durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden.

Eine Aufreinigung der Esterasen kann mit bekannten, chromatographischen Verfahren erzielt werden, wie Molekularsieb-Chromatographie (Gelfiltration), wie Q-Sepharose-Chromatographie, lonenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Besonders geeignet ist es, zur Isolierung des rekombinanten Esterase Vektorsysteme oder Oligonukleotide zu verwenden, die die cDNA um bestimmte Nukleotidsequenzen verlängern und damit für veränderte Polypeptide oder Fusionsproteine kodieren, die z.B. einer einfacheren Reinigung dienen. Derartige geeignete Modifikationen sind beispielsweise als Anker fungierende sogenannte "Tags", wie z.B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Peptide an einen festen Träger, wie z.B. einer Polymermatrix, dienen, die beispielsweise in einer Chromatographiesäule eingefüllt sein kann, oder an einer Mikrotiterplatte oder an einem sonstigen Träger verwendet werden kann. Gleichzeitig können diese Anker auch zur Erkennung der Esterasen verwendet werden. Zur Erkennung der Esterasen können außerdem übliche Marker, wie Fluoreszenzfarbstoffe, Enzymmarker, die nach Reaktion mit einem Substrat ein detektierbares Reaktionsprodukt bilden, oder radioaktive Marker, allein oder in Kombination mit den Ankern zur Derivatisierung der Esterasen verwendet werden.

## 6. Reaktoren

Das erfindungsgemäße Verfahren kann in fachüblichen Reaktoren durchgeführt und in verschiedenen Maßstäben durchgeführt werden, beispielsweise im Labormaßstab (wenige Milliliter bis Dutzende von Litern) oder im industriellen Maßstab (Liter bis mehrere Tausend Kubikmeter). Wird die Esterase in Form eines isolierten, mehr oder weniger aufgereinigten Enzyms verwendet, so kann ein chemischer Reaktor verwendet werden. Wird im Rahmen der erfindungsgemäßen Verfahren die verwendete Esterase von einem Mikroorganismus produziert und gegebenenfalls in das Reaktionsmedium abgegeben, so dass dort die Esterspaltung stattfinden kann, so wird ein Bioreaktor (Fermenter) verwendet, in dem neben geeigneten Verfahrensbedingungen für die Esterspaltung auch geeignete Lebensbedingungen für die Esterase produzierenden Mikroorganismen (z.B. durch Bereitstellung eines geeigneten Nährmediums und pH-Werts, einer geeignete Temperatur, Versorgung mit Sauerstoff oder anderen Gasen, Antibiotika, etc.) bereitgestellt werden. Ein Bioreaktor ist somit geeignet für die Bereitstellung der Esterasen durch ein Ganzzellsystem, in dem lebende Mikroorganismen (beispielsweise prokaryotische Zellen wie Bakterien oder Archaebakterien, oder eukaryotische Zellen wie Hefen, Säugetierzellen oder Insektenzellen) kultiviert werden. Der Fachmann ist vertraut mit den verschiedenen Aspekten der Verwendung von Reaktoren, beispielsweise dem Upscaling von chemischen oder biotechnologischen Verfahren vom Labormaßstab bis hin zum großtechnischen Produktionsmaßstab sowie der Optimierung von Prozessparameter und kann gegebenenfalls auf entsprechende Fachliteratur zurückgreifen (für biotechnologische Verfahren beispielsweise Crueger und Crueger, Biotechnologie - Lehrbuch der angewandten Mikrobiologie, 2. Aufl., R. Oldenbourg Verlag, München, Wien, 1984).

## 7. Reaktionsprodukte

Ein weiterer Gegenstand der vorliegenden Erfindung sind die durch die erfindungsgemäßen Verfahren erhaltenen Reaktionsprodukte. Die Reaktionsprodukte können zusammen mit dem im erfindungsgemäßen Verfahren verwendeten Reaktionsgemisch vorliegen, oder teilweise oder weitgehend aufgereinigt werden. Geeignete Aufreinigungsgrade können vom Fachmann je nach Bedarf und weiterem Verwendungszweck routinemäßig bestimmt werden und um umfassen beispielsweise chemische Reinheit oder Analysenreinheit. Geeignete Methoden zur Aufreinigung sind dem Fachmann bekannt und umfassen beispielsweise Fällung und chromatographische Methoden.

Die erhaltenen Reaktionsprodukte, die je nach Grad der vorangegangenen Esterspaltung Polyacrylsäuren oder teilweise veresterte Polyacrylsäure darstellen, können beispielsweise eingesetzt werden in Farben und Beschichtungen, wie Wandfarben, Beschichtungen (z.B. von Metalloberflächen, insbesondere in der Automobilindustrie) und Lacken. Weitere Anwendungen finden sich in der Papierindustrie (z.B. zur Beeinflussung der Druckfähigkeit, des Aussehens oder des Glanzes), bei Klebstoffen und Mitteln zur Versiegelung oder Abdichtung, in der Textilindustrie (z.B. als Bindemittel für Pigmentfärbungen oder Bedruckungsvorgänge) oder in der Lederindustrie (beispielsweise, um Lederoberflächen hydrophob zu machen). Weitere Anwendungen umfassen die Herstellung von Textil- und Glasfasern, Poliermittel für Fußböden, Autos oder Schuhe, Zusatzstoffe für hydraulische Bindemittel, Mörtel oder Beton. Polyacrylate werden weiterhin eingesetzt beim Pflanzenschutz und dem Aussprühen von Düngemitteln sowie als Matrizes für Ionenaustauscher. Anwendungen von Polymethacrylaten (d.h. Poly(methylmethacrylaten) umfassen insbesondere die sogenannten Acrylgläser sowie weiterhin Zusatzstoffe für die Erdölindustrie, Verdickungsmittel für Dispersionen und in der kosmetischen Industrie. Polymethacrylate werden weiterhin als Prothesen in der Zahnheilkunde, als Knochenzement in der Chirurgie und als galenische Hilfsstoffe bei Tabletten mit kontrollierter Freisetzung eingesetzt.

Durch das erfindungsgemäße Verfahren ist es insbesondere möglich, regiospezifisch veränderte Polymere bereitzustellen. Überraschenderweise wurde gefunden, dass unter Verwendung von Enzymen, die nur endständige Estergruppen des Polymermoleküls hydrolytisch spalten, nicht jedoch weiter im Inneren des Polymermoleküls befindliche Estergruppen, derartig selektiv modifizierte Polymere im Vergleich zu chemischen Verfahren vergleichsweise einfach hergestellt werden können.

Die Erfindung wird nun unter Bezugnahme auf die nachfolgenden, nicht-limitierenden Beispiele näher erläutert.

## Experimenteller Teil

### Beispiel 1: Herstellung einer EstC-Genbank

Es wurde eine Genbibliothek mit mutierter EstC von Burkholderia gladioli (SEQ ID NO:51) hergestellt, um funktionale Mutanten von EstC zu erhalten. Dabei wurden durch zufällige Mutagenese ("random mutagenesis") Mutationen in EstC eingeführt, wobei das Plasmid pMSPM1.17 in einer fehleranfälligen PCR ("error-prone PCR") als Template laut Protokoll eingesetzt wurde und unter Verwendung des GeneMorph® II Random Mutagenesis Kit (Stratagene) Sequenzfehler in die PCR-amplifizierten EstC-Gene eingebracht wurden. Lit: Instruction Manual GeneMorph® II Random Mutagenesis Kit (Cat# 200550). Anschließend wurden die PCR-amplifizierten Gene mit dem Promega Wizard SV Gel and PCR Clean-Up System laut Protokoll gereinigt und mit den Restriktionsenzymen Ndel und HindIII in Puffer R von Fermentas geschnitten. Unabhängig davon wurde als Vektor-DNA das Plasmid pos470Δ8 (Balzer et al., Nucleic Acids Research, 1992, Vol. 20, No. 8, 1851-1858) mit den gleichen Restriktionsenzymen verdaut, und nach thermischer Inaktivierung der Restriktionsenzyme (80˚C, 20 min.) mit 1 μl Calf Intestine Alkaline Phosphatase (CIAP) von Fermentas für 1 Stunde bei 37 ˚C dephosphoryliert. Die gesamte DNA wurde auf ein Agarosegel aufgetragen, der entsprechende Vektorteil ausgeschnitten und mit dem Wizard SV Gel and PCR Clean-Up System von Promega laut Protokoll aufgereinigt. Anschließend wurden die Vektor-DNA und die hergestellten Inserts mit T4 DNA Ligase von Fermentas 1 Stunde bei 20˚C ligiert. Das Ligationsprodukt wurde anschließend mit dem Wizard SV Gel and PCR Clean-Up System von Promega gereinigt und entsalzt und mit dem Micro PulserTM von BioRad durch Elektroporation (2,5kV) je 2 μl der Plasmid-DNA in 80μl in kompetente E. coli BL21 (DE3) Zellen transformiert. Sofort nach dem Elektroschock wurden die Zellen in 1 ml SOC Medium aufgenommen und für 35 min. bei 37˚C und 550 rpm auf einem Eppendorf "Thermomixer comfort" regeneriert. Die kompetenten Zellen wurden hergestellt laut "Current Protocols in Molecular Biology" 1.8.4. Transformierte Klone wurde auf LB-Amp Platten (100μg/ml) selektioniert, 10 Transformanten auf den oben angegebenen Selektionsplatten ausgestrichen und für eine Plasmidpräparation gemäß OIAprep® Spin Miniprep Kit von Qiagen verwendet. Die isolierten Plasmide wurden mit den oben angegebenen Restriktionsenzymen verdaut und durch Agarose-Gelelektrophorese das Vorliegen eines Inserts der korrekten Größe bestätigt. Zusätzlich wurde durch Sequenzierung die Mutationsrate bestimmt.

### Beispiel 2: Herstellung von mutierten EstC-Varianten durch ortspezifische Mutagenese

Für die ortsspezifische Mutagenes wurde der "QuickChange Multi-Site-directed Kit" von Stratagene (Katalog-Nr. #200514 und #100515) verwendet, der eine schnelle und zuverlässige Methode für die ortsspezifische Mutagenese einer Plasmid-DNA an bis zu fünf Stellen gleichzeitig bietet. Für die Mutation jeder einzelnen Stelle ist ein einziges mutagenes Oligonukleotid erforderlich, wobei ein doppelsträngiges DNA-Template verwendet wird. Folgende mutagene Oligonucleotid-Primer, die gemäß den Empfehlungen im Protokoll des QuickChange Multi-Site-directed Kit entworfen und von Invitrogen synthetisiert wurden, wurden verwendet, wobei die erzeugten Mutationen in Klammern angegeben sind:
SEQ ID NO:39:

(Phe138Val) 5'-gctgctcttgtatatcttgctgcggtcatgcctgc- 3'

SEQ ID NO:40:

(Phe138Ala) 5'-gctgctcttgtatatcttgctgcggccatgcctgc- 3'

SEQ ID NO:41:

(Glu154Ala) 5'-gtaagagcaccagcaaaccatggcgaaatgctggc- 3'

SEQ ID NO:42:

(Leu163Ala) 5'-ctggcctcggcgatctgcgccagccct- 3'

SEQ ID NO:43:

(Leu189Ala) 5'-cggcctatctcgccacggcgaagca- 3'

SEQ ID NO:44:

(Leu189AlaLeu193Ala) 5'-gccacggcgaagcaggcggcgttcgaggatgttga- 3' SEQ ID NO:45:

(Leu193Ala) 5'-gcaggcggcgttcgaggatgttgac- 3' SEQ ID NO:46:

(Val150Ala) 5'-gtacctggtcttgattacgcgagagctcct- 3' SEQ ID NO:47:

(Thr188Ser) 5'-gcctatctcgcctcgctgaagcagg- 3' SEQ ID NO:48:

(Leu160Ala) 5'-cgaaatggcggcctcgctgatctgc- 3'

SEQ ID NO:50:

(Thr188AlaLeu189AlaLeu193Ala) 5'-tatctcgcctcggcgaagcaggcggcgttcgagga-3'

Für die ortspezifische Mutagenese wurde unter Verwendung eines üblichen Protokolls für DNA-Minipräparationen (OIAprep Spin Miniprep Kit von Qiagen) doppelsträngiges DNA-Template hergestellt. Anschließend wurde die Reaktionsmischung für die Synthese des mutierten Strangs über PCR gemäß folgender Aufstellung hergestellt, wobei die einzelnen Bestandteile in der Reihenfolge, die in der Aufstellung angegeben ist, in das Reaktionsgefäß gegeben und anschließend gemischt wurden:

Experimentelle Reaktion:

2,5 µl 10xQuikChange Multi-Reaktionspuffer
2,0 µl ds-DNA Template: pMS470[*EstC*] (50 ng)
1,0 µl dNTP-Mix
1,0 µl QuikChange Multi-Enzymmischung
x µl mutagene Primer (bei Verwendung von 1-3 Primern wurden ca. 100 ng jedes Primers, bei Verwendung von 4-5 Primern ca. 50 ng jedes Primers zugegeben)
x µl zweifach destilliertes $H_2O$ für ein Endvolumen von 25 µl

Kontrollreaktion:

2,5 µl 10xQuikChange Multi-Reaktionspuffer
18,5 µl zweifach destilliertes $H_2O$
1,0 µl (50 ng/µl) QuikChange Multi-Kontrolltemplate
1,0 µl QuikChange Multi-Kontrolprimermischung (100 ng/µl pro Primer)
1,0 µl dNTP-Mix
1,0 µl QuikChange Multi-Enzymmischung

Die Reaktionen unter Verwendung der in der nachfolgenden Tabelle 1 angegebenen Parameter durchgeführt, wobei für die Kontrollreaktion eine Dauer von 8 Minuten für die Strangsynthese verwendet wurde:

Tabelle 1:

| Segment | Zyklen | Temperatur | Dauer |
|---|---|---|---|
| 1 | 1 | 95°C | 1 Minute |
| 2 | 30 | 95°C | 1 Minute |
| | | 55°C | 1 Minute |
| | | 65°C | 10 Minuten |

Anschließend wurde zu jeder Reaktionsmischung der jeweiligen Amplifizierungsreaktionen 1 μl des Restriktionsenzyms DpnI gegeben. Jede Reaktionsmischung wurde durch mehrfaches Auf- und Abpipettieren vorsichtig und gründlich gemischt. Anschließend wurden die Reaktionsmischungen in einer Mikrozentrifuge 1 min zentrifugiert und unmittelbar danach 1 h bei 37°C inkubiert, um die elterliche (nicht-mutierte) doppelsträngige DNA zu verdauen.

Anschließend wurden die erhaltenen PCR-Fragmente nach der Reinigung mit dem Promega Wizard SV Gel und PCR Clean-Up System laut Protokoll gereinigt und mit den Restriktionsenzymen NdeI und HindIII in Puffer R (Fermentas) verdaut und über die in Beispiel 1 beschriebene Vorgehensweise in ebenfalls mit den Restriktionsenzymen NdeI und HindIII geschnittenen Vektor pMS470A8 ligiert

Anschließend erfolgte eine Transformation von XL10-Gold Ultra-kompetenten Zellen gemäß den Herstellerangaben (Stratagene).

Für jede Transformationsreaktion wurde ein geeignetes Volumen gemäß den Angaben in Tabelle 2 auf $LB_{amp}$-Platten ausplattiert. Für die Kontrolle der Mutagenese wurden die Zellen auf $LB_{amp}$-Platten ausplattiert, die mit 80 μg/ml X-gal und 20 mM IPTG hergestellt worden waren.

Tabelle 2

| Reaktionsart | Volumen auf die Platte |
|---|---|
| Experimentelle Mutagenese | 1 μl, 10 μl und 100 μl |
| Mutagenese-Kontrolle | 10 μl |

Die Transformationsplatten wurden anschließend über Nacht bei 37°C inkubiert.
Die zu erwartende Kolonienanzahl aus der Mutagenese-Kontrolltransformation liegt zwischen 50 und 800 Kolonien. Mehr als 50% der Kolonien aus der Mutagenese-Kontrolltransformation sollten alle drei Mutationen aufweisen und als blaue Kolonien auf IPTG- und X-gal-haltigen Agarplatten erscheinen. Die zu erwartende Kolonienanzahl aus der experimentellen Mutagenese-Transformation liegt zwischen 10 und 1000 Kolonien. Nach Erreichen der erwarteten Kolonienanzahl wurden etwa 150 Transformanten auf $LB_{amp}$-IPTG-Platten (0,2 mM IPTG) ausgestrichen und über Nacht bei 37°C inkubiert.

Beispiel 3: Aktivitätsnachweis mittels Filterassay

Die in Beispiel 2 hergestellten EstC-Mutanten wurden auf ihre Fähigkeit untersucht, Substrate mit Esterbindungen zu spalten. Dazu wurden die entsprechenden Plasmide in jeweils in den Expressionsstamm E. coli BL21 (DE3) (Invitrogen) transformiert. 10 Kolonien mit der gleichen Mutation wurden zuerst mit α-Naphtylacetat getestet.

a) Farbassay mit Fast Blue B auf Spaltung von Naphtolestern

Dazu wurden die Kolonien sowie Positivkontrollen EstC, EstB und die Negativkontrolle *E. coli* BL21 [pos470Δ8] auf LB-Amp-IPTG-Platten (100 μg/ml Ampicillin, 0,2 mM IPTG) in einem festgelegten Muster ausgestrichen und über Nacht inkubiert. Anschließend wurden die Zellkolonien mit einem sterilen Papierfilter (Whatman, Cat No 1001-085) von der Agarplatte abgezogen und die Filter 10 Minuten bei 37°C getrocknet. Die Bakterienzellen wurden 25 min bei Raumtemperatur mit 500 μl Bug Buster (Novagen) aufgeschlossen und die Filtermembranen anschließend 10 min mit 0,02 M Kaliumphosphatpuffer (pH 7,0) äquilibriert. Für den Test mit α-Naphtylacetat wurden 5 ml Puffer (0,05 M Tris-HCl, pH 7,0) mit 100 μl Substratlösung (10mg/ml a-Naphtylacetat in Aceton) und 100 μl Farblösung (10mg/ml Fast Blue B Salz in $H_2O$) in einer Glaspetrischale gemischt und die Filtermembranen getränkt. Die Bildung eines violett-roten Farbkörpers zeigte die Aktivität der Esterase an.

b) pH-Shift Assay

Die, mit dem Substrat α-Naphtylacetat, jeweils aktivsten Klone wurden auf die Substrate Polymethylacrylat (PMA), Polybutylacrylat (PBA) sowie 2,4-Dimethylglutarsäure-dimethylester (DG) und 2,4-Dimthylglutarsäure-dibutylester (DB) getestet. Dazu wurden wie oben beschrieben, Kolonien auf Agarplatten angezüchtet, und wie in Beispiel 3a beschrieben auf Papierfilter übertragen.
Für die Herstellung der Substratlösung wurde zunächst eine Screening-Lösung vorbereitet, die 400 μL Phosphatpuffer (0.1 M, pH = 7.0), 800 μL Aqua dest., 500 μL DMSO, 750 μL Phenolrot (10 g/l) und 400 μL NaOH (0.1 M) enthielt. Für pH-Shift-Assays mit DG beziehungsweise DB wurden jeweils 200 μl dieser Substrate in jeweils 9,5 ml Screening-Lösung gelöst und die oben vorbereiteten Filtermembranen mit 1 ml der so zubereiteten Substratlösung getränkt. Für pH-Shift-Assays mit den Substraten PMA beziehungsweise PBA wurden diese jeweils auf trockene Membranen pipettiert, die oben hergestellten Filtermembranen mit den lysierten Zellkolonien wurden mit 1 ml der Screening-Lösung getränkt und auf die Membranen mit den Substraten gelegt. Nach Inkubation bei RT wurde der durch Bildung von Säuregruppen nach Esterspaltung ausgelöste Farbumschlag von rot nach gelb bestimmt. Der Farbumschlag konnte ab ca. 20 Minuten detektiert werden.

In Fig. 5 ist die Aktivität der verwendeten Klone angegeben, wobei folgende Symbole verwendet wurden:

+++: sehr aktiv
++: aktiv
+: schwach aktiv
○: nicht aktiv

Beispiel 4: Herstellung von Esterase enthaltenden bakteriellen Rohlysaten

Zur Herstellung von enzymhaltigem bakteriellen Rohlysat wurden als Vorkultur zunächst 30 ml LB-Medium, das 100 μg/ml Ampicillin enthielt (nachfolgend: $LB_{amp}$) in einem 100 ml Erlenmeyerkolben mit einer Bakterienkolonie beimpft und über Nacht auf einem Schütteltisch bei 37°C und 180 U/min kultiviert. Für die anschließende Hauptkultur wurden 500 ml Kulturmedium [2xTY (10g/L Hefeextrakt, 16g/L Trypton, 5g/L NaCl)] mit einem Aliquot der Vorkultur auf eine $OD_{600}$ von 0,1 beimpft und bei 30°C kultiviert, bis eine $OD_{600}$ von 0,5 - 0,8 erreicht war. Anschließend erfolgte eine Induktion der Expression durch 0,1 mM IPTG und eine 16-stündige Inkubation bei 28°C. Die Bakterienkultur wurde danach 30 min bei 4°C in 1000 ml Zentrifugenbechern bei 4000 U/min (= 3062 x g) zentrifugiert und die Pellets jeweils in 20 ml 0,1 M Phosphatpuffer (pH 7) suspendiert. Zum Aufschließen der Zellen erfolgte eine 5-minütige Ultraschallbehandlung, gefolgt von 1 min Pause und einer weiteren 5-minütigen Ultraschallbehandlung mit einem Branson Sonifier 250, wobei der Duty Cycle auf 50% und die Leistungskontrolle auf Stufe 5 eingestellt waren. Das erhaltene Aufschlussprodukt wurde 1 h bei 40 000 U/min ( = 117734 x g) zentrifugiert, unter Verwendung einer Membran mit einer Porengröße von 0,2 μm sterilfiltiert und bis zur Verwendung bei -20°C aufbewahrt.

Beispiel 5: Standardisierung der Enzymaktivität

Wie in Beispiel 4 hergestelltes Rohlysat wurde in einem photometrischen Assay zur Spaltung von para-Nitrophenylbutyrat (p-NPB) als Substrat verwendet (wobei der Fachmann aufgrund seines Fachwissen in der Lage ist, Assays auf der Grundlage anderer geeigneter Substrate, wie beispielsweise para-Nitrophenylacetat, p-NPA, zu entwickeln). Bei diesem Test zur Esterase-Aktivitätsbestimmung mit para-Nitrophenylestern wurden in einer Küvette 980μl Puffer (1M Tris-Puffer, pH 7.0) mit 10μl Enzymlösung (oder Verdünnung der Enzymlösung mit 1M Tris-Puffer, pH 7.0) gemischt. Die Reaktion wurde mit 10μl Substratlösung (400 mM para-Nitrophenylester in DMSO) gestartet und in einem Beckmann UV Spektrophotometer bei der Wellenlänge von 405nm die Zunahme des para-Nitrophenols verfolgt.

$$\varepsilon = \text{(para-Nitrophenol) } 11.86\,mM^{-1}cm^{-1} \text{ (bestimmt bei pH 7.0)}$$

Formel für die Berechnung der Aktivität:

$$U/ml = \frac{\Delta Abs/\min \times V}{\varepsilon \times 1 \times v} \times Verdünnung$$

Eine Einheit (Unit, U) Esterase entspricht der Menge, die in diesem Assay einem Umsatz von 1 μmol/min führt.

Beispiel 6: Nachweis der enzymkatalysierten Spaltung

Zum Unterscheidung von enzymkatalysierter Substratspaltung und chemischer Substratspaltung wurde ein Rohlysat des Stammes NJ70 (enthaltend eine funktionale Mutante der Esterase B von Burkholderia gladioli, EstB_NJ70, gemäß SEQ ID NO:4) gemäß Beispiels 4 hergestellt. Als Substratlösung 750 μl PBA-Lösung (CAS Nr: 9003-49-0, Sigma Aldrich, 25-30 Gew.-% in Toluol) (entsprechend 1,75 mmol monomere Estergruppen) zu 35 ml eines Mastermix (340 ml 0,9% NaCl, 220 ml Toluol, 70 ml Emulgen (10%ige Lösung in $H_2O$), pH 7) Emulgen 913, Lot. 2265, Kao Cemicals, Osaka Japan, alternativ kann Tergitol NP-9 von Alrich verwendet werden) gegeben und der pH-Wert der Lösung mit 10 mM NaOH-Lösung auf 10 eingestellt. Anschließend wurden 500 μl Rohlysat (entsprechend 1000 Einheiten gemäß dem in Beispiel 5 etablierten Standard) zugegeben und die Absenkung des pH-Werts durch im Zuge der Spaltung der Esterbindungen freiwerdende Säuregruppen durch Zugabe von 10 mM NaOH kompensiert. Zur Bestimmung der chemischen Autolyse wurden in einem unabhängigen Ansatz 500 μl Rohlysat mit durch 30-minütiges Kochen bei 80°C denaturiertem Enyzm zugegeben. Die Ergebnisse sind in Fig. 6 gezeigt. Nach Zugabe von aktivem Enzym ist aufgrund der freiwerdenden Säuregruppen eine hohe Zugabe von 10 mM NaOH erforderlich, um den pH-Wert auf 10 zu halten (Kurve mit Rautensymbolen in Fig. 6). Dagegen erfolgt bei Zugaben von denaturiertem Enzym nur eine geringe Esterspaltung durch chemische Autolyse (Kurve mit Dreiecken in Fig. 6). Der sprunghafte Anstieg an pH- Korrekturmittelverbrauch (NaOH) unmittelbar nach Zugabe von Rohlysat mit denaturiertem Enzym kann durch den pH-Wert des Rohlysats von 7 erklärt werden.

Beispiel 7: Inhibierung der enzymkatalysierten Spaltung durch Polyacrylsäure

Die entsprechenden Versuche erfolgten analog zu Beispiel 6 in wässriger Lösung, wobei durch die enzymkatalysierte Spaltung von Esterbindungen des vorgegebenen Substrats Polybutylacrylat (PBA) Säuregruppen gebildet wurden. Diese führten zu einem Absinken des pH-Werts, der durch Zugabe von 10 mM NaOH kompensiert wurde. Die zugegebene Menge NaOH spiegelt also den Verlauf der Reaktion wieder. Alle Messungen wurden in 35 ml Reaktionsansatz bei pH = 9.0 und einer Temperatur von 37°C durchgeführt. Es wurden 350 Einheiten der Esterase EstB_NJ70 (SEQ ID NO:4) und 750 μl der PBA-Lösung eingesetzt, was 1,75 mmol monomeren Estergruppen entspricht. Als Titrationslösung wurde 10mM NaOH verwendet.

Zur Bestimmung einer möglichen Produkthemmung wurden in einem Parallelansatz zu Beginn der Messung 850 μl einer 20%igen Sokalan-Lösung zugesetzt (Sokalan PA: 15 (Polyacrylsäure, Mw 1200, pH 8.0), was 1,74mmol monomeren Acrylsäureeinheiten entspricht. Wie aus den Fig. 2 gezeigten Ergebnissen ersichtlich ist, wurden die eingesetzten Enzyme von *Burkholderia gladioli* (SEQ ID NO:1; WT in Fig. 7) sowie deren funktionale Mutanten EstB_N27 (SEQ ID NO:3) bzw. EstB_NJ70 (SEQ ID NO:4) durch Sokalan nicht oder nur minimal inhibiert.

Beispiel 8: pH-Abhängigkeit der enzymkatalysierten Esterspaltung

Die pH-Abhängigkeit der enzymkatalysierten Esterspaltung wurde analog zu Beispiel 6 durchgeführt. Die Rohlysate des Stammes NJ70 (der die funktionale Mutante NJ70, d.h.

SEQ ID NO:4 exprimiert) wurden wie in Beispiel 4 beschrieben hergestellt und eine 1000 Einheiten entsprechende Menge zu Substratlösungen gegeben, die auf verschiedene pH-Werte im Bereich von 5 bis 11 eingestellt waren. Zur Erfassung einer möglichen chemischen Autohydrolyse bei höheren pH-Werten (im Bereich von etwa pH ~ 9.0 - pH 11.0) wurden die Rohlysate erst nach 10 min zugegeben, wobei in keinem Fall vor der Zugabe ein nennenswerter pH-Korrekturmittelverbrauch (NaOH) zu verzeichnen war. Aus den in Fig. 8 gezeigten Ergebnissen ist zu ersehen, dass im Bereich von pH 5 bis pH 11 eine Esterspaltung erfolgte.

Beispiel 9: Temperaturabhängigkeit der enzymkatalysierten Esterspaltung

Die Temperaturabhängigkeit der enzymkatalysierten Esterspaltung wurde analog Beispiel 6 bei konstantem pH-Wert 9 durchgeführt. Wie in Beispiel 4 beschrieben, wurden Rohlysate des Stammes NJ70 hergestellt, und in einer jeweils 1000 Einheiten entsprechenden Menge zu Substratlösungen gegeben und bei den verschiedenen Temperaturen im Bereich von 10°C bis 50°C autotitriert. Als Kontrolle für die chemische Autohydrolyse wurde Substratlösung mit einem pH von 9 für 10 min bei der angegebenen Temperatur inkubiert. Bei ausbleibender pH-Änderung (kein Verbrauch an pH-Korrekturmittel) wurde auf fehlende Autohydrolyse geschlossen. Aus den in Fig. 9 gezeigten Daten ist zu ersehen, dass ein Temperaturoptimum im Bereich von 20°C bis 40°C lag, insbesondere bei 30°C.

Beispiel 10: Enzymatische Aktivität immobilisierter Esterase

35 ml eines Mastermixes (340 mL 0,9% NaCl-Lösung, 220 mL Toluol und 70 mL Tergitol (10% in $H_2O$)), der 2 ml PBA-Lösung enthielt (Sigma Aldrich , Mw 99 000, 25-30 Gew.-% in Toluol) (entsprechend 4,66 mmol monomere Estergruppen), wurden auf einen pH-Wert von 9 eingestellt und bei einer Temperatur von 37˚C gehalten. 3,6 ml EstB_ NJ70 enthaltendes Rohlysat wurden mit 0,5 g Eupergit C250L (Aldrich) und 30 ml 0,5 M $K_2HPO_4$-Puffer (pH 9,5) 48 h bei Raumtemperatur rotiert. Anschließend wurde die Suspension abgesaugt und das Matrixmaterial mit 0,1 M Tris-Puffer (pH 7,0) gewaschen. Anschließend wurde eine Eine Proteinbestimmung nach Bradford ergab, dass 86% des Proteins an den Träger gebunden hatten. Jeweils zu 35 ml des oben genannten Reaktionsansatzes wurden 50 Einheiten EstB_NJ70, eine 50 Einheiten auf Eupergit immobilisierte EstB_NJ70, 50 Einheiten auf Eupergit immobilisierte und denaturierte EstB_NJ70 bzw. die entsprechende Menge Eupergit ohne immobilisiertes Enzym zugegeben. Die Kompensation des pH-Werts erfolgte durch Zugabe von 10 mM NaOH. Wie in Fig. 10 gezeigt war bei Zugabe der beiden Kontrollen (Eupergit ohne EstB_ NJ70 bzw. mit denaturiertem EstB_NJ70) eine geringfügige Freisetzung von Säuregruppen zu verzeichnen. Die Zugabe von EstB_NJ70 bzw. immobilisierter EstB_ NJ70 führte dagegen zu einer deutlich höheren Freisetzung von Säuregruppen,
wobei der Reaktionsverlauf bei EstB_NJ70 einer Sättigungskurve mit vergleichsweise hoher anfänglicher Reaktionsgeschwindigkeit und späterer Verlangsamung entsprach, während der Reaktionsverslauf bei immobilisierter EstB_ NJ70 mit anfänglich geringerer Geschwindigkeit erfolgte, dafür aber während des Messzeitraums konstant blieb.

Beispiel 11: Esterspaltung bei kurzkettigen und langkettigen Substraten

Jeweils 35 ml des in Beispiel 10 angegebenen Reaktionsansatzes wurden auf eine pH-Wert von 9,0 und eine Temperatur von 37˚C eingestellt. Anschließend wurden jeweils 350 Einheiten der in den Figur 11 angegebenen Esterasen sowie entweder kurzkettiges PMA (Sigma Aldrich) mit einem Molekulargewicht von 30.000 (PMAkurz in Fig. 11) oder langkettiges PMA (PMAkurz in Fig.11) mit einem Molekulargewicht von 40000 zugegeben, was in beiden Fällen 1,75 mmol monomerer Estergruppen entsprach, und die Reaktion entweder bis zur Einstellung eines Gleichgewichts (Fig. 6) oder über den angegebenen Zeitraum (Fig. 11) durchgeführt. Die Kompensation des durch die Esterspaltung absinkenden pH-Wertes erfolgte durch Zugabe von 10 mM NaOH, wobei bei Fig.11 aus dem Verbrauch an NaOH der prozentuale Anteil der hydrolysierten Estergruppen berechnet wurde. Aus Fig. 11 ist zu ersehen, dass sowohl kurzkettiges als auch langkettiges PMA enzymatisch gespalten wird. Im Falle denaturierter Enzyme (NJ70_den in Fig. 11) ist die Änderung im Korrekturmittelverbrauch (NaOH) auf die Zugabe der jeweiligen Komponente zum Reaktionsansatz zu erklären, da dieser keine Pufferkapazität aufweist, so dass sich bereits kleinste Menge von Lösungen mit abweichendem pH-Wert auf den Gesamt-pH-Wert auswirken.

Auf die Offenbarung der in vorliegender Beschreibung zitierten Druckschriften wird ausdrücklich Bezug genommen.

Übersicht über die verwendeten SEQ ID NOs und Stammbezeichnungen

SEQ ID NO:1
Proteinsequenz (Wildtyp) von EstB aus Burkholderia gladioli

SEQ ID NO:2
Proteinsequenz (Wildtyp) von EstC aus *Burkholderia gladioli*

SEQ ID NO:3
Mutierte Proteinsequenz (interne Bezeichnung: N27; EstB_N27), bei der gegenüber dem Wildtyp EstB (SEQ ID NO:1) folgende Aminosäureaustausche vorgenommen wurden:

Ser17Leu; Gly132Ser; Glu251Gly; Ala311Val; Glu316Lys

SEQ ID NO:4
Mutierte Proteinsequenz (interne Bezeichnung: NJ70; NJ_70; EstB_NJ70), bei der gegenüber dem Wildtyp EstB (SEQ ID NO:1) folgende Aminosäureaustausche vorgenommen wurden:

Pro8Leu; Gly132Ser; Trp134Arg; Arg155Cys; Glu251Gly; Ala311Val; Glu316Lys

SEQ ID NO:5
Cutinase aus *Humicola insolens*

SEQ ID NO:6
Lipase B aus *Candida antarctica*

SEQ ID NO:7
Esterase aus *Burkholderia ambifaria* (interne Bezeichnung: A0TF86_9BURK@1)

SEQ ID NO:8
Esterase aus *Burkholderia cenocepacia* (interne Bezeichnung: Q1BK05_BURCA@1)

SEQ ID NO:9
Esterase aus *Burkholderia cenocepacia* (interne Bezeichnung: A2W245_9BURK@1)

SEQ ID NO:10
Esterase aus *Burkholderia cenocepacia* (interne Bezeichnung: A0U6R7_9BURK@1)

SEQ ID NO:11
Esterase aus *Burkholderia cenocepacia* (interne Bezeichnung: A0B440_BURCH@1)

SEQ ID NO:12
Esterase aus *Burkholderia cepacia* (interne Bezeichnung: Q0B5R9_BURCM@1)

SEQ ID NO:13
Esterase aus *Burkholderia dolosa* (interne Bezeichnung: A2WH69_9BURK@1)

SEO ID NO:14
Esterase aus *Burkholderia mallei* (interne Bezeichnung: ZP_00928253@1)

SEO ID NO:15
Esterase aus *Burkholderia mallei* (interne Bezeichnung: Q629M1_BURMA@1)

SEO ID NO:16
Esterase aus *Burkholderia mallei* (interne Bezeichnung: A5XMRO_BURMA@1)

SEQ ID NO:17
Esterase aus *Burkholderia mallei* (interne Bezeichnung: A3MH33_BURM7@1)

SEO ID NO:18
Esterase aus *Burkholderia mallei* (interne Bezeichnung: A1UXMB_BURMS@1)

SEO ID NO:19
Esterase aus *Burgholderia multivorans* (interne Bezeichnung: A0UE35_9BURK@1)

SEQ ID NO:20
Esterase aus *Burkholderia pseudomallei* (interne Bezeichnung: ZP_0131527@1)

SEQ ID NO:21
Esterase aus *Burkholderia pseudomallei* (interne Bezeichnung: ZP_01209854@1)

SEQ ID NO:22
Esterase aus *Burkholderia pseudomallei* (interne Bezeichnung: ZP_00893464@1)

SEQ ID NO:23
Esterase aus *Burkholderia pseudomallei* (interne Bezeichnung: Q3JIF4_BURP1@1)

SEQ ID NO:24

Esterase aus *Burkholderia pseudomallei* (interne Bezeichnung: A4LP64_BURPS@1)

SEQ ID NO:25
Esterase aus *Burkholderia pseudomallei* (interne Bezeichnung: A3PA33_BURPO@1)

SEQ ID NO:26
Esterase aus *Burkholderia pseudomallei* (interne Bezeichnung: A3NPJ8_BURP6@1)

SEQ ID NO:27
Esterase aus *Burkholderia sp.* (interne Bezeichnung: Q39BM9_BURS3@1)

SEQ ID NO:28
Esterase aus *Burkholderia thailandensis* (interne Bezeichnung: Q2T2QO_BURTA@1)

SEQ ID NO:29
Esterase aus *Burkholderia vietnamensis* (interne Bezeichnung: A4JKI4_BURVG@1)

SEQ ID NO:30
Esterase aus *Mycobacterium smegmatis* (interne Bezeichnung: A0R6Y0_MYCS2@1)

SEQ ID NO:31
Esterase aus *Saccharopolyspora erythraea* (interne Bezeichnung: A4FPB3_SACEN@1)

SEQ ID NO:32
Esterase aus *Saccharopolyspora erythraea* (interne Bezeichnung: A4FDC0_SACEN@1)

SEQ ID NO:33
Esterase aus *Saccharopolyspora erythraea* (interne Bezeichnung: A4F6M6_SACEN@1)

SEQ ID NO:34
Esterase aus *Stigmatella aurantiaca* (interne Bezeichnung: Q096X7_STIAU@1)

SEQ ID NO:35
Esterase aus *Streptomyces ambofaciens* (interne Bezeichnung: A3KIK7_STRAM@1)

SEQ ID NO:36
Esterase aus *Streptomyces coelicolor* (interne Bezeichnung: NP_630279@1)

SEQ ID NO:37
als EstB_Short4 (oder EstB_N27_Short4) bezeichnete Deletionsmutante von EstB_N27, bei der die Aminosäuren 317-319 (RGP) von SEQ ID NO:3 (EstB_N27) entfernt wurden

SEQ ID NO:38
als EstB_Short5 (oder EstB_N27_Short5) bezeichnete Deletionsmutante von EstB_N27, bei der die Aminosäuren 248-255 (PLPGGHGA) von SEQ ID NO:3 (EstB_N27) durch die kürzere Sequenz SLGTT ersetzt wurden

SEQ ID NO:39 bis SEQ ID NO:49:
PCR-Primer gemäß Beispiel 2

SEQ ID NO:50
Nukleinsäuresequenz von EstB (gemäß SEQ ID NO:1)

SEQ ID NO:51
Nukleinsäuresequenz von EstC (gemäß SEQ ID NO:2)

SEQ ID NO:52
Nukleinsäuresequenz von Cutinase (gemäß SEQ ID NO:5)

SEQ ID NO:53
Nukleinsäuresequenz von Lipase B (gemäß SEQ ID NO:6)

SEQ ID NO:54
Nukleinsäuresequenz für EstB_N27 (gemäß SEQ ID NO:3)

SEQ ID NO:55
Nukleinsäuresequenz für EstB_NJ70 (gemäß SEQ ID NO:4)

SEQ ID NO:56
Nukleinsäuresquenz für EstB_N27 Short 4 (gemäß SEQ ID NO:37)

SEQ ID NO:57
Nukleinsäuresequenz für EstB_N27 Short 5 (gemäß SEQ ID NO:38)

SEQ ID NO:58
Hydroxynitril-Lyase aus *Hevea brasiliensis* (GenBank-Nr. AAC49184)

SEQ ID NO:59
Hydroxynitril-Lyase aus *Manihot esculenta* (SwissProt-Nr. P52705)

## Patentansprüche

1. Verfahren zur enzymkatalysierten Hydrolyse von Polyacrylsäureestern, wobei man

   a) wenigstens einen Polyacrylsäureester bereitstellt,
   b) den wenigstens einen Polyacrylsäureester mit wenigstens einem Enzym inkubiert, das ausgewählt ist unter auf Esterbindungen einwirkenden Enzymen (EC 3.1), bis die in dem Polyacrylsäureester enthaltenen Estergruppen teilweise oder vollständig hydrolytisch gespalten sind; und gegebenenfalls
   c) das modifizierte Polymer isoliert.

2. Verfahren nach Anspruch 1, wobei das Enzym ausgewählt ist unter Carbonsäureesterhydrolasen (EC 3.1.1), insbesondere unter Carboxylesterasen (E.C. 3.1.1.1), Triacylglycerinlipasen (EC. 3.1.1.3) und Cutinasen (3.1.1.74).

3. Verfahren nach Anspruch 1 oder 2, wobei der Polyacrylsäureester ein Homo- oder Copolymer ist.

4. Verfahren nach einem der vorhergehenden Ansprüche, wobei der Polyacrylsäureester ein alternierendes Copolymer, ein statistisches Copolymer, ein Gradientencopolymer, ein Blockcopolymer oder ein Pfropfcopolymer ist.

5. Verfahren nach einem der vorhergehenden Ansprüche, wobei das Polymer MonomerBausteine der allgemeinen Formel I

   $$R^1R^2C=CR^3_COOR^4 \qquad (I)$$

   umfasst, worin
   $R^1$, $R^2$ und $R^3$ gleich oder verschieden sind und ausgewählt sind unter H, einem geradkettigen $C_1$-$C_{20}$ Hydrocarbylrest und einem verzweigten $C_3$-$C_{20}$ Hydrocarbylrest, und
   $R^4$ ausgewählt ist unter H, einem geradkettigen $C_1$-$C_{20}$ Hydrocarbylrest, einem verzweigten $C_3$-$C_{20}$ Hydrocarbylrest und einem cyclischen $C_3$-$C_{20}$ Hydrocarbylrest, wobei der Hydrocarbylrest gegebenenfalls substituiert ist durch eine oder mehrere gleiche oder verschiedene Gruppen, die ausgewählt sind unter Hydroxy-, Amino-, Epoxid-, Thiolgruppen und Halogenatomen,
   wobei im Polymer in wenigstens einem Monomerbaustein der Formel I $R^4$ nicht für H steht.

6. Verfahren nach Anspruch 5, **dadurch gekennzeichnet, dass** der Polyacrylsäureester zusätzlich zu den Monomeren der Formel I wenigstens eine weitere davon verschiedene Monomerkomponente in einem molaren Anteil von 0 bis 15 Mol-% enthält, welche vorzugsweise ausgewählt ist unter N-Vinylformamid, Methacrylsäure, Methacrylsäureester, Itaconsäure, Itaconsäureester, Vinylphosphonsäure, Vinylsulfonsäure, Vinylalkohol, N-Vinylimidazol, N-Vinylformamid, Styrol, Maleinsäure, Maleinsäureester, Ethylen und/oder Propylen, und Acrylamid und substituierten Acrylamiden, wobei der Substituent ausgewählt ist unter einem geradkettigen $C_1$-$C_{20}$ Hydrocarbylrest, einem verzweigten $C_3$-$C_{20}$ Hydrocarbylrest und einem cyclischen $C_3$-$C_{20}$ Hydrocarbylrest, wobei der Hydrocarbylrest gegebenenfalls substituiert ist durch eine oder mehrere gleiche oder verschiedene Gruppen, die ausgewählt sind unter Hydroxy-, Amino-, Epoxid, Thiolgruppen und Halogenatomen.

7. Verfahren nach einem der vorhergehenden Ansprüche, **dadurch gekennzeichnet, dass** das Enzym ausgewählt ist unter einer Esterase der Familie VIII, einer Typ C Esterase, einer Cutinase gemäß SEQ ID NO:5 oder einer davon abgeleiteten Cutinase, und einer Triacylglycerinlipase gemäß SEQ ID NO:6 oder einer davon abgeleiteten Triacylglycerinlipase.

8. Verfahren nach einem der vorhergehenden Ansprüche, **dadurch gekennzeichnet, dass** das Enzym ausgewählt ist unter einem Protein gemäß SEQ ID NO:1 (Esterase B von *Burkholderia gladioli*), SEQ ID NO:2 (Esterase C von *Burkholderia gladioli*) oder davon abgeleiteten funktionalen Mutanten.

9. Verfahren nach Anspruch 8, **dadurch gekennzeichnet, dass** das Enzym eine Mutante einer Esterase gemäß SEQ ID NO:1 oder SEQ ID NO:2 mit gegenüber SEQ ID NO:1 bzw. SEQ ID NO:2 vergleichbarer oder gesteigerter Aktivität hinsichtlich der Hydrolyse von Polyacrylsäureestern, und/oder eine Mutante einer Esterase gemäß SEQ ID NO:1 oder SEQ ID NO:2 mit gegenüber SEQ ID NO:1 bzw. SEQ ID NO:2 erhöhter Stabilität ist.

10. Verfahren nach einem der Ansprüche 8 und 9, wobei die Mutante im Vergleich mit einer Esterase gemäß SEQ ID NO:1 oder SEQ ID NO:2 eine gesteigerte Hydrolyse-Aktivität gegenüber Polyacrylsäuremethylester und/oder Polyacrylsäurebutylester zeigt.

**11.** Verfahren nach einem der Ansprüche 8 bis 10, **dadurch gekennzeichnet, dass**

(a) die Mutante einer Esterase gemäß SEQ ID NO:1 wenigstens eine Mutation in einem oder mehreren der Aminosäurereste Ser17, Gly132, Trp134, Arg155, Glu251, Ala311 und Glu316 aufweist; oder
(b) die Mutante einer Esterase gemäß SEQ ID NO:2 wenigstens eine Mutation in einem oder mehreren der Aminosäurereste Phe138, Va1150, Leu160, Thr188 und Leu193 aufweist.

**12.** Verfahren nach einem der Ansprüche 8 bis 11, **dadurch gekennzeichnet, dass** die Mutante von SEQ ID NO:1 abgeleitet ist und

(a) wenigstens eine der Mutationen Ser17Leu, Gly132Ser, Glu251Gly, Ala311Val und Glu316Lys umfasst; und/oder
(b) wenigstens eine der Mutationen Pro8Leu, Gly132Ser, Trp134Arg, Arg155Cys, Glu251Gly, A1a311Va1 und GIu316Lys umfasst.

**13.** Verfahren nach einem der Ansprüche 8 bis 10, **dadurch gekennzeichnet, dass** die Mutante von SEQ ID NO:2 abgeleitet ist und eine der nachfolgenden Mutationen oder Kombinationen von Mutationen umfasst:

(a) Phe138Ala
(b) Phe138Ala, Thr188Ser
(c) Phe138Ala, Leu160Ala, Thr188Ser
(d) Leu193Ala
(e) Leu193Ala, Phe138Ala, Thr188Ser, Val150Ala
(f) Leu193Ala, Phe138Ala, Thr188Ser
(g) Leu193Ala, Phe138Ala, Thr188Ser, Leu160Ala, Val150Ala
(h) Val150Ala
(i) Val150Ala, Thr188Ser
(j) Leu193Ala, Phe138Val
(k) Leu193Ala, Phe138Val, Thr188Ser, Val150Ala
(l) Leu193Ala, Thr188Ser
(m) Leu193Ala, Phe138Val, Thr188Ser
(n) Leu193Ala, Phe138Val, Thr188Ser, Leu160Ala
(o) Phe138Val, Val150Ala, Thr188Ser
(p) Phe138Val
(q) Phe138Val, Thr188Ser

**14.** Verfahren nach Anspruch 7, **dadurch gekennzeichnet, dass** die Mutante eine Deletionsmutante einer Esterase der Familie VIII oder einer Typ C Esterase ist.

**15.** Verfahren nach Anspruch 14, **dadurch gekennzeichnet, dass** die Deletionsmutante wenigstens eine Loop-Kürzung aufweist.

**16.** Verfahren nach Anspruch 15, **dadurch gekennzeichnet, dass** die Deletionsmutante eine unter SEQ ID NO:37 und SEQ ID NO:38 ausgewählte Aminosäuresequenz aufweist.

**17.** Funktionale Esterase-Mutante gemäß der Definition in einem der Ansprüche 8 bis 16.

**18.** Nukleinsäure, die

a) für eine für eine Esterase-Mutante gemäß Anspruch 17 kodiert, oder
b) eine zu a) komplementäre Nukleinsäure darstellt, oder
c) mit einer Nukleinsäure gemäß a) oder b) unter stringenten Bedingungen hybridisiert, insbesondere eine Sequenzidentität von wenigstens 80% aufweist und für eine Mutante einer Esterase der Familie VIII oder für eine Typ C Esterase-Mutante kodiert, welche Polyacrylsäureester hydrolysiert.

**19.** Vektor, umfassend eine Nukleinsäure gemäß Anspruch 18.

**20.** Vektor gemäß Anspruch 19, wobei die Nukleinsäure operativ mit einem Promotor verknüpft ist.

**21.** Mikroorganismus, umfassend wenigstens einen Vektor gemäß Anspruch 19 oder 20.

**22.** Verfahren zur Herstellung einer Esterase gemäß Anspruch 17, wobei man

a) einen zur Expression der Esterase befähigten Wirtsorganismus gemäß Anspruch 21 kultiviert
b) gegebenenfalls die Expression der Esterase induziert, und
c) gegebenenfalls die Esterase aus dem Wirtsorganismus und/oder dem Kulturmedium isoliert.

**23.** Verwendung einer Esterase gemäß der Definition in einem der Ansprüche 7 bis 17, eines Vektors gemäß Anspruch 19 oder 20, oder eines Mikroorganismus gemäß Anspruch 21 zur Durchführung eines Verfahrens nach einem der Ansprüche 1 bis 16.

**24.** Polymeres Reaktionsprodukt, erhältlich nach einem Verfahren gemäß einem der Ansprüche 1 bis 16.

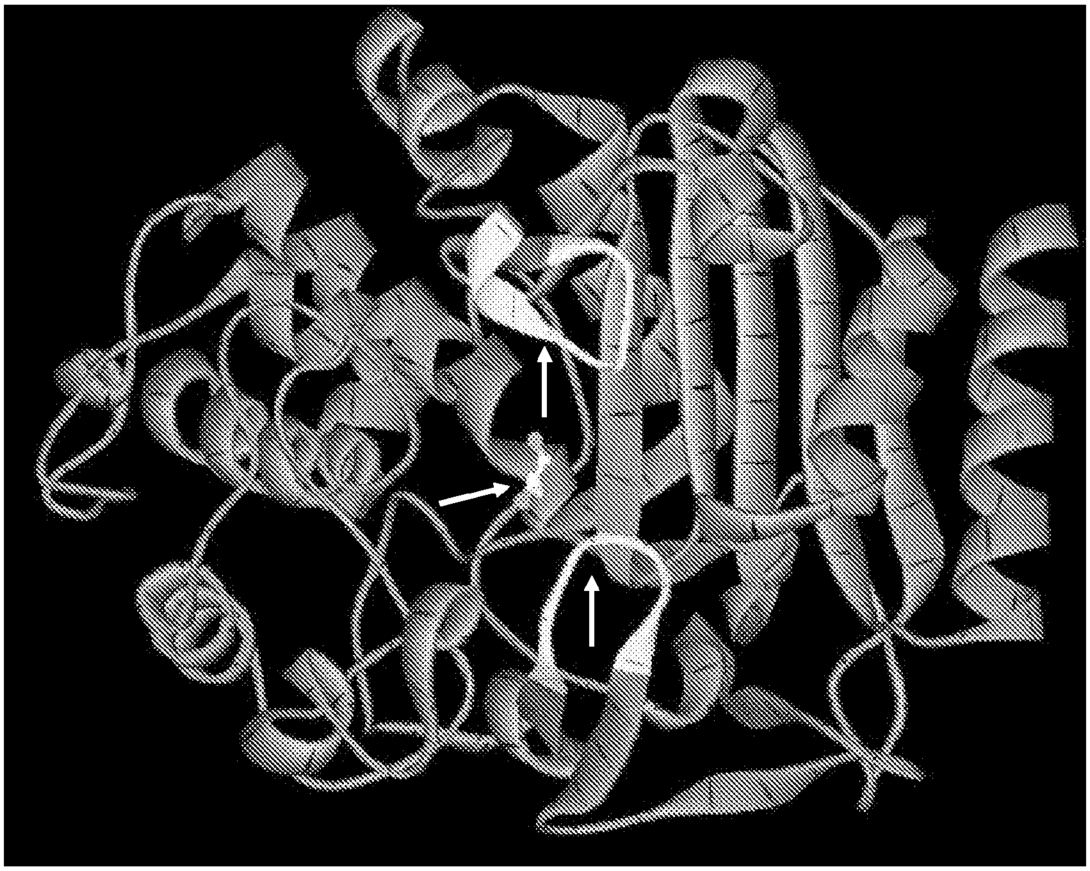

Fig. 1

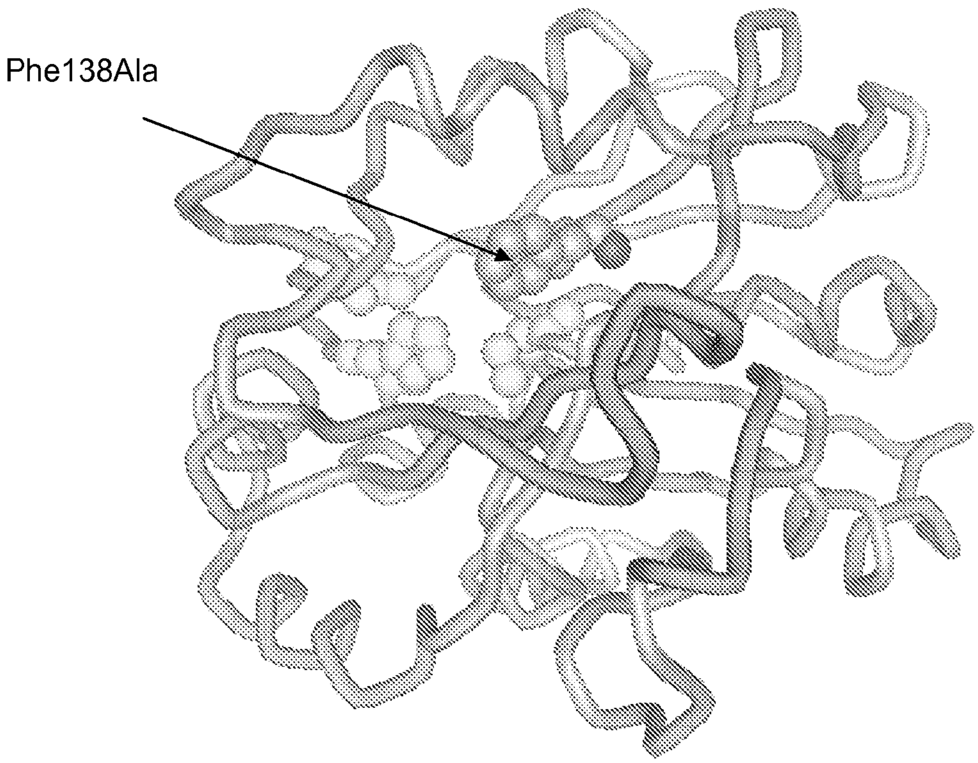


Fig. 2

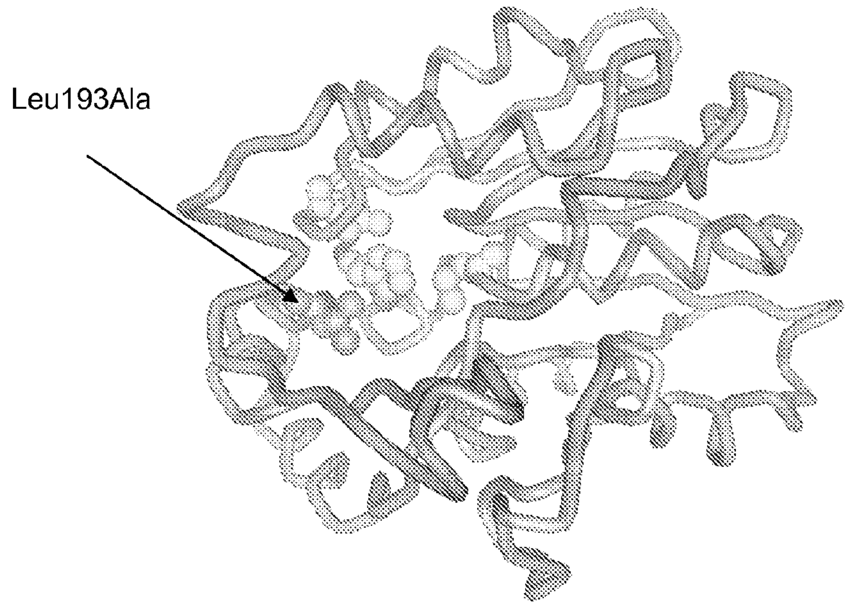


Fig. 3

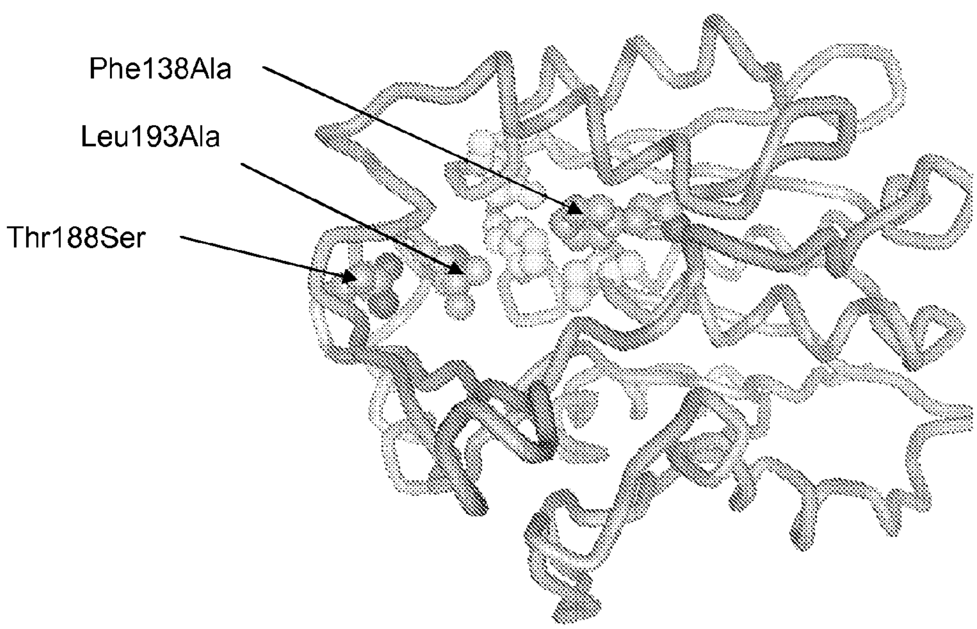


Fig. 4

| Variante | Mutation | PMA | PBA | DG | DB | α-N |
|---|---|---|---|---|---|---|
| EstC | ------ | ++ | o | ++ | o | ++ |
| 2K20 | Leu193Ala | +++ | o | +++ | o | +++ |
| 2K29 | Leu193Ala, Phe138Ala | + | + | ++ | + | +++ |
| B48 | Leu193Ala, Phe138Ala, Thr188Ser | ++ | + | ++ | + | +++ |
| A14 | Leu193Ala, Phe138Ala, Thr188Ser, Leu160Ala, Val150Ala | + | + | ++ | + | +++ |
| B31 | Leu193Ala, Phe138Ala, Thr188Ser, Val150Ala | + | + | ++ | + | + |
| C12 | Leu193Ala, Phe138Val | ++ | + | +++ | + | +++ |
| A4 | Leu193Ala, Phe138Val, Thr188Ser | ++ | + | +++ | + | +++ |
| A13 | Leu193Ala, Phe138Val, Thr188Ser, Leu160Ala | ++ | + | ++ | + | +++ |
| A18 | Leu193Ala, Phe138Val, Thr188Ser, Val150Ala | ++ | + | ++ | + | +++ |
| A7 | Leu193Ala, Thr188Ser | ++ | + | ++ | + | +++ |
| 1K22 | Phe138Ala | +++ | o | +++ | o | +++ |
| B4 | Phe138Ala, Leu160Ala, Thr188Ser | ++ | o | +++ | o | ++ |
| B7 | Phe138Ala, Thr188Ser | +++ | o | +++ | + | ++ |
| A11 | Phe138Val | ++ | o | +++ | o | ++ |
| C10 | Phe138Val, Glu154Ala | ++ | o | +++ | o | ++ |
| A2 | Phe138Val, Thr188Ser | ++ | o | +++ | o | ++ |
| A15 | Phe138Val,Val150Ala, Thr188Ser | ++ | o | +++ | o | ++ |
| A101 | Val150Ala | +++ | o | +++ | o | ++ |
| A102 | Val150Ala, Thr188Ser | +++ | o | +++ | o | ++ |

Fig. 5a Mutationen und Aktivitäten in der Übersicht. +++ = sehr aktiv, ++ = aktiv, + = schwach aktiv, o = nicht aktiv

| Variante | Mutation | PMA | PBA | DG | DB |
|---|---|---|---|---|---|
| EstB | ----- | ++ | +++ | o | o |
| NJ70 | Pro8Leu, Gly132Ser, Trp134Arg, Arg155Cys, Glu251Gly, Ala311Val, Glu316Lys | ++ | +++ | + | o |
| N27 | Ser17Leu, Gly132Ser, Glu251Gly, Ala311Val, Glu316Lys | ++ | +++ | + | o |

Fig.5b Mutationen und Aktivitäten der EstB Varianten in der Übersicht, +++ = sehr aktiv, ++ = aktiv, + = schwach aktiv, o = nicht aktiv

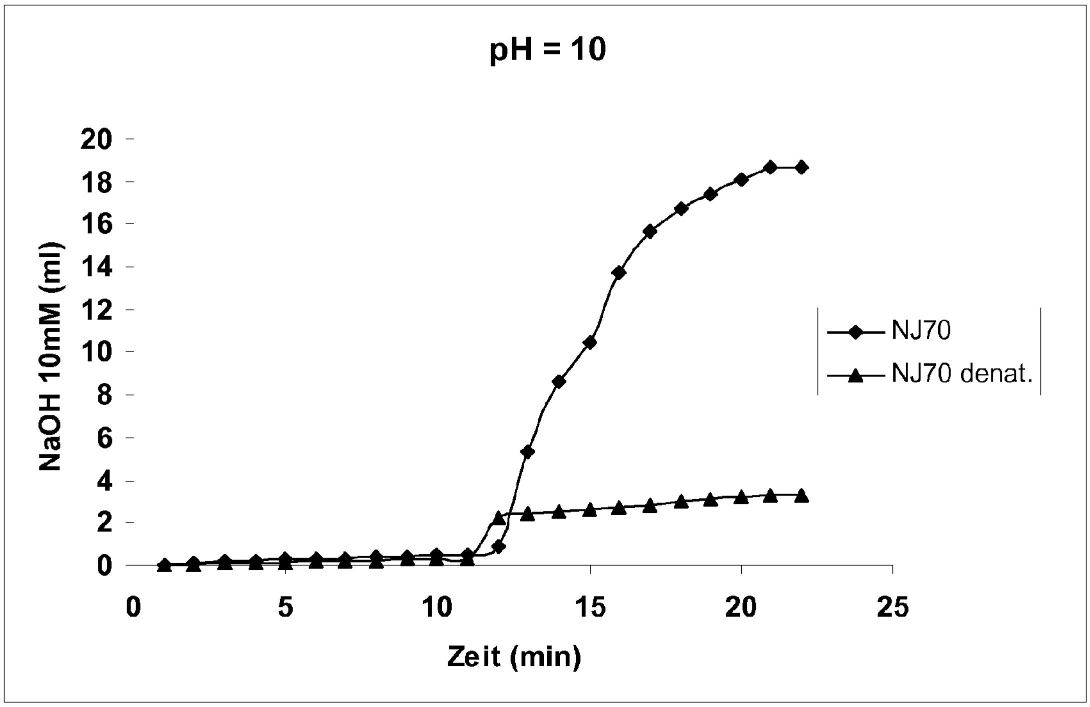
pH = 10
NaOH 10mM (ml)
Zeit (min)
NJ70
NJ70 denat.
0
2
4
6
8
10
12
14
16
18
20
0
5
10
15
20
25

Fig. 6

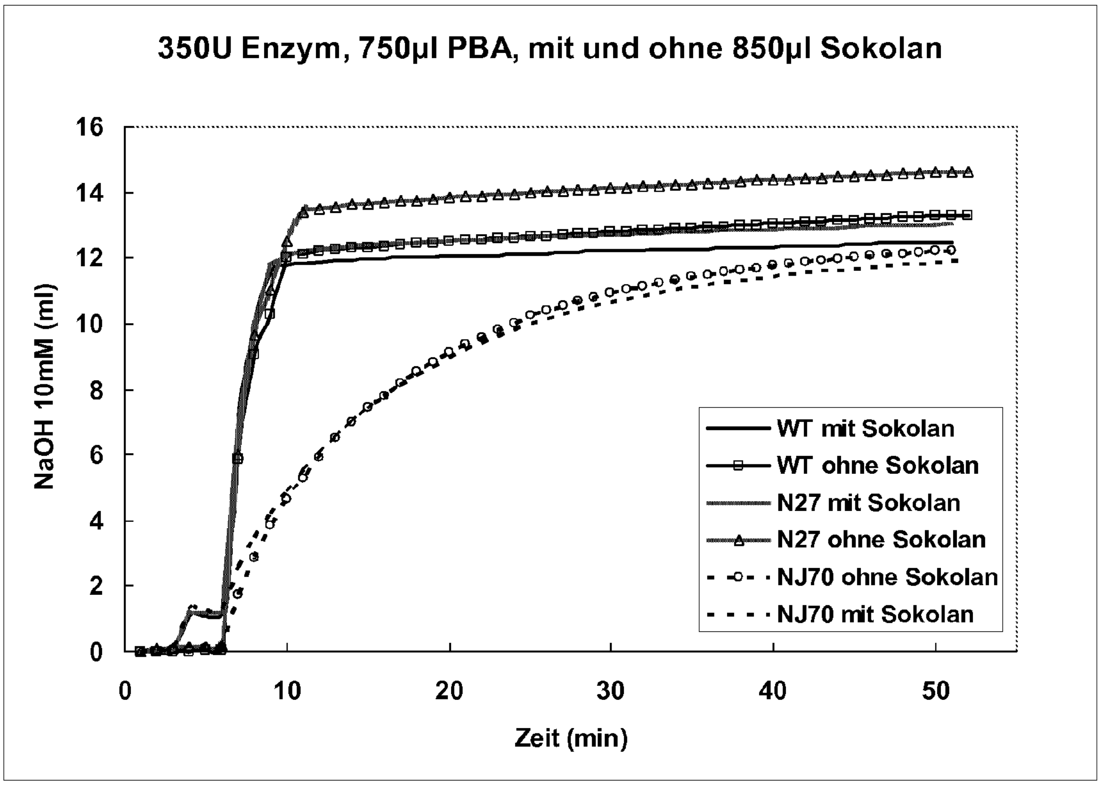
350U Enzym, 750µl PBA, mit und ohne 850µl Sokolan
NaOH 10mM (ml)
Zeit (min)
WT mit Sokolan
WT ohne Sokolan
N27 mit Sokolan
N27 ohne Sokolan
NJ70 ohne Sokolan
NJ70 mit Sokolan
0
2
4
6
8
10
12
14
16
0
10
20
30
40
50

Fig. 7

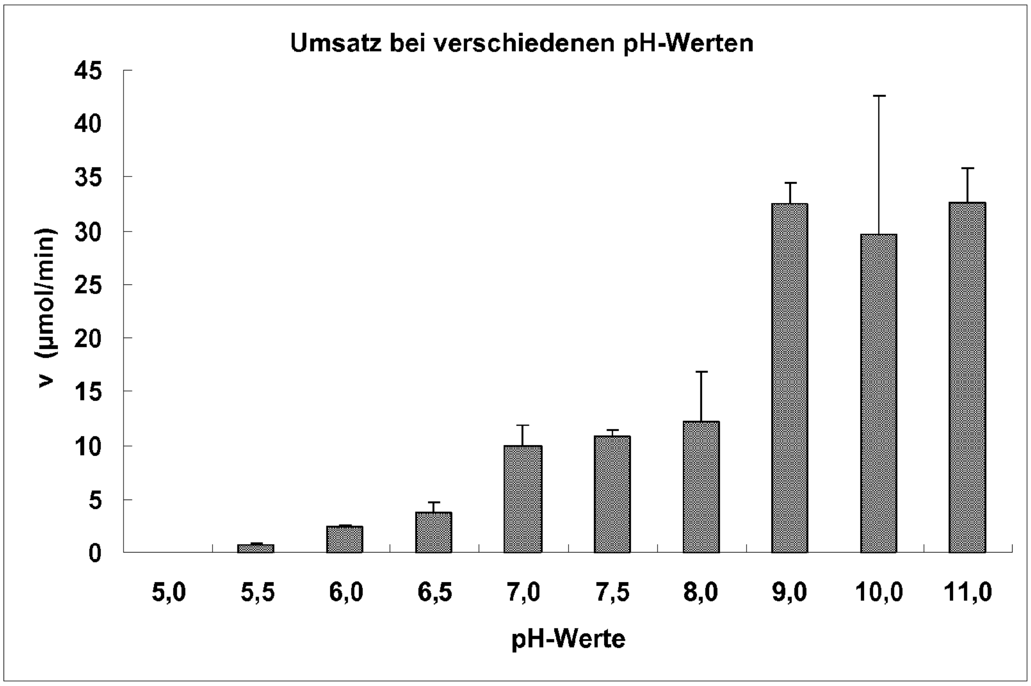
Umsatz bei verschiedenen pH-Werten
45
40
35
30
25
20
15
10
5
0
v (µmol/min)
5,0
5,5
6,0
6,5
7,0
7,5
8,0
9,0
10,0
11,0
pH-Werte

Fig. 8

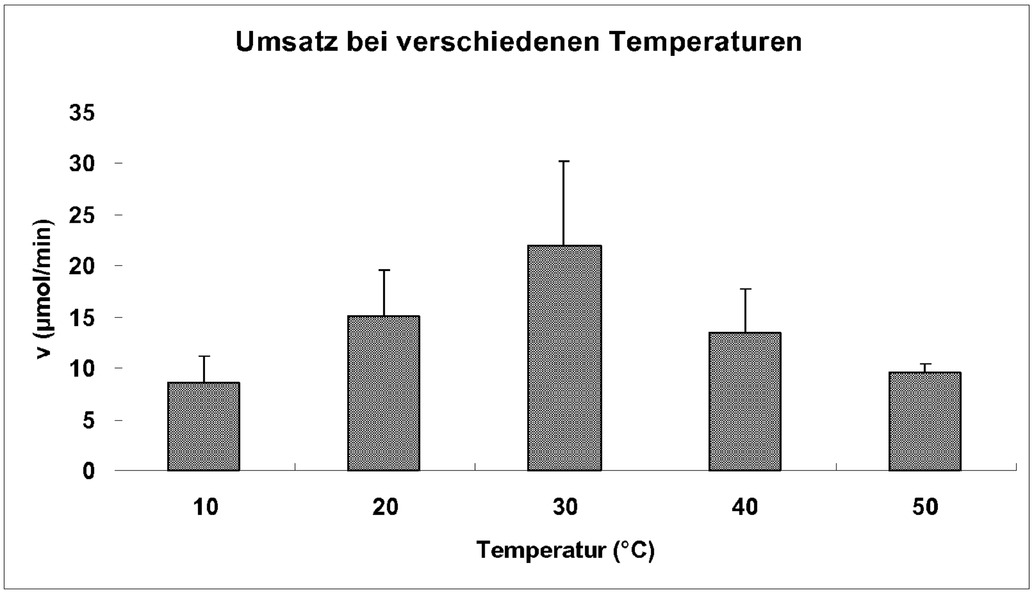
Umsatz bei verschiedenen Temperaturen
35
30
25
20
15
10
5
0
v (µmol/min)
10
20
30
40
50
Temperatur (°C)

Fig. 9

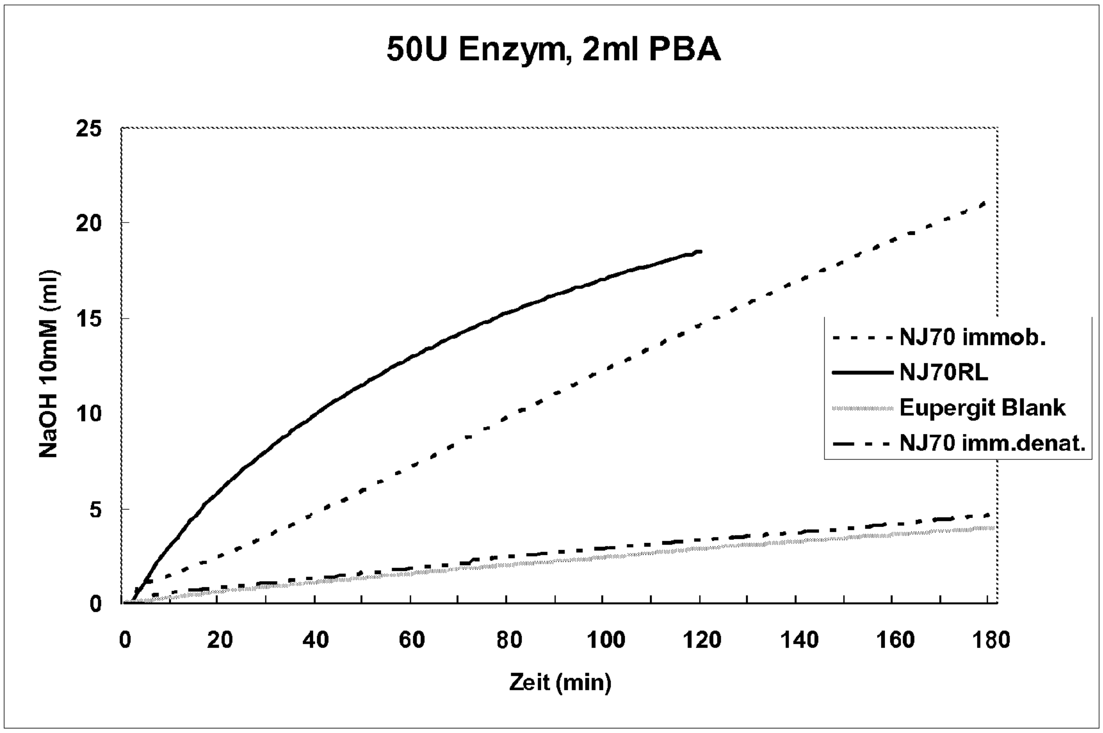
50U Enzym, 2ml PBA
NaOH 10mM (ml)
Zeit (min)
0
5
10
15
20
25
0
20
40
60
80
100
120
140
160
180
NJ70 immob.
NJ70RL
Eupergit Blank
NJ70 imm.denat.

Fig. 10

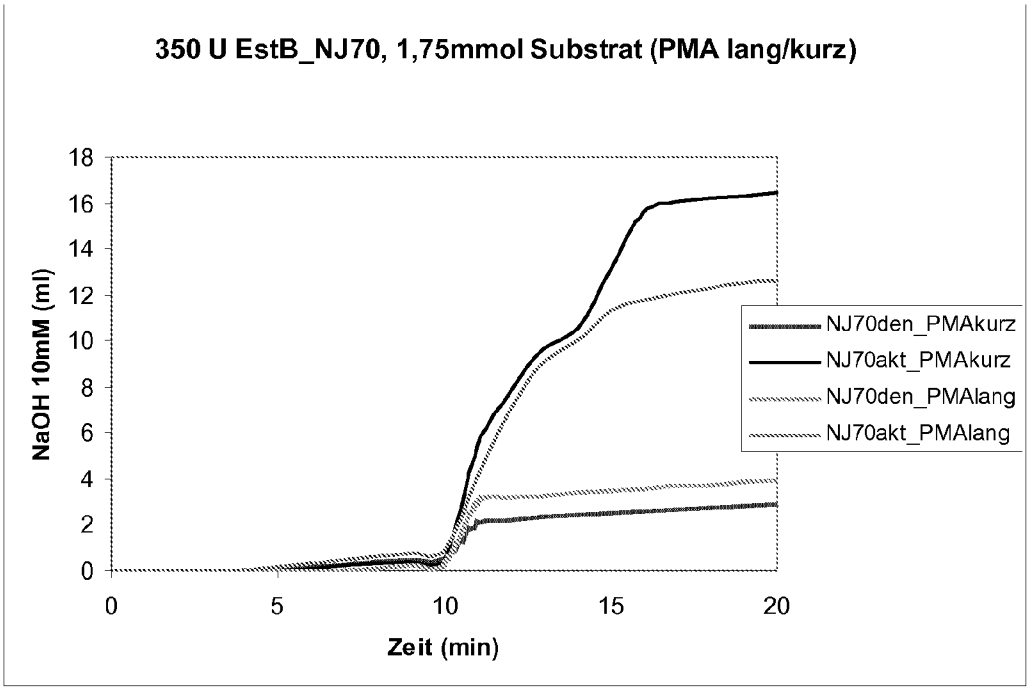
350 U EstB_NJ70, 1,75mmol Substrat (PMA lang/kurz)
NaOH 10mM (ml)
Zeit (min)
0
2
4
6
8
10
12
14
16
18
0
5
10
15
20
NJ70den_PMAkurz
NJ70akt_PMAkurz
NJ70den_PMAlang
NJ70akt_PMAlang

Fig.11

Europäisches Patentamt
European Patent Office
Office européen des brevets

# EUROPÄISCHER RECHERCHENBERICHT

Nummer der Anmeldung
EP 08 16 0774

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X,D | O'SULLIVAN C ET AL: "Hydrolysis of poly (n-butylcyanoacrylate) nanoparticles using esterase" POLYMER DEGRADATION AND STABILITY, BARKING, GB, Bd. 78, Nr. 1, 1. Januar 2002 (2002-01-01), Seiten 7-15, XP004374668 ISSN: 0141-3910 * Abbildung 11 * ----- | 1-3, 7-12,23 | INV. C08F8/12 C12N9/18 |
| Y | LALOT T ET AL: "LIPOZYME-CATALYZED TRANSESTERIFICATION OF OLIGO(METHYLACRYLATE)S" POLYMER BULLETIN, SPRINGER, HEIDELBERG, DE, Bd. 26, 1. Januar 1991 (1991-01-01), Seiten 55-62, XP001180443 ISSN: 0170-0839 * das ganze Dokument * ----- | 1-23 | |
| Y | INPRAKHON ET AL: "Regioselectivity of enzymatic modification of poly(methyl acrylate)" JOURNAL OF BIOTECHNOLOGY, ELSEVIER SCIENCE PUBLISHERS, AMSTERDAM, NL, Bd. 131, Nr. 4, 20. September 2007 (2007-09-20), Seiten 418-424, XP022259319 ISSN: 0168-1656 * das ganze Dokument * ----- | 1-23 | RECHERCHIERTE SACHGEBIETE (IPC) C08F C12N |
| Y | DE 103 05 076 A1 (BASF COATINGS AG [DE] BASF AG [DE]) 26. August 2004 (2004-08-26) * das ganze Dokument * ----- -/-- | 1-23 | |

Der vorliegende Recherchenbericht wurde für alle Patentansprüche erstellt

| Recherchenort | Abschlußdatum der Recherche | Prüfer |
|---|---|---|
| München | 2. Juni 2009 | Vogt, Titus |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................
& : Mitglied der gleichen Patentfamilie, übereinstimmendes Dokument

9

EPO FORM 1503 03.82 (P04C03)

Europäisches Patentamt
European Patent Office
Office européen des brevets

# EUROPÄISCHER RECHERCHENBERICHT

Nummer der Anmeldung
EP 08 16 0774

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | WO 2005/040487 A (UNIV DO MINHO [PT]; PAULO ARTUR MANUEL CAVACO [PT]; CARNEIRO ANA FILIP) 6. Mai 2005 (2005-05-06)<br>* das ganze Dokument *<br>----- | 1-4 | |
| A | TOR R ET AL: "ENZYMATICALLY CATALYSED TRANSESTERIFICATIONS OF ACRYL AND METHACYRL MONOMERIC ESTERS"<br>ENZYME AND MICROBIAL TECHNOLOGY, STONEHAM, MA, US,<br>Bd. 12, 1. April 1990 (1990-04-01), Seiten 299-304, XP000910508<br>ISSN: 0141-0229<br>----- | 1-23 | |
| X | REITER B ET AL: "Cloning and characterization of EstC from Burkholderia gladioli, a novel-type esterase related to plant enzymes."<br>APPLIED MICROBIOLOGY AND BIOTECHNOLOGY DEC 2000,<br>Bd. 54, Nr. 6, Dezember 2000 (2000-12), Seiten 778-785, XP002529455<br>ISSN: 0175-7598 | 18-22 | |
| Y | * das ganze Dokument *<br>----- | 1-10,23 | RECHERCHIERTE SACHGEBIETE (IPC) |
| X | JP. MAHALIK & G. MADRAS: "Effect of the Alkyl Group Substituents on the Thermal and Enzymatic Degradation of Poly(n-alkyl acrylates)"<br>IND. ENG. CHEM. RES.,<br>Bd. 44, 10. Mai 2005 (2005-05-10), Seiten 4171-4177, XP002529456 DOI: 1021/ie0500164<br>* das ganze Dokument *<br>----- | 1-3,5,7, 11-13,23 | |

9

~~Der vorliegende Recherchenbericht wurde für alle Patentans~~prüche erstellt

| Recherchenort | Abschlußdatum der Recherche | Prüfer |
|---|---|---|
| München | 2. Juni 2009 | Vogt, Titus |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

Nummer der Anmeldung

EP 08 16 0774

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☒ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

7-23(vollständig); 1-6(teilweise)

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches Patentamt
European Patent Office
Office européen des brevets

**MANGELNDE EINHEITLICHKEIT DER ERFINDUNG ERGÄNZUNGSBLATT B**

Nummer der Anmeldung

EP 08 16 0774

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 12, 16(vollständig); 1-11, 14-15, 17-23(teilweise)

   Verfahren zur enzymkatalysierten Hydrolyse von Polyacrylsäureestern, dadurch gekennzeichnet, dass das Enzym einer Esterase der Familie VIII ist.

   ---

2. Ansprüche: 13(vollständig); 1-11, 14-15, 17-23(teilweise)

   Verfahren zur enzymkatalysierten Hydrolyse von Polyacrylsäureestern, dadurch gekennzeichnet, dass das Enzym einer Typ C Esterase ist.

   ---

3. Ansprüche: 1-7, 23(teilweise)

   Verfahren zur enzymkatalysierten Hydrolyse von Polyacrylsäureestern, dadurch gekennzeichnet, dass das Enzym einer Cutinase gemäss SEQ ID NO: 5 ist.

   ---

4. Ansprüche: 1-7, 23(teilweise)

   Verfahren zur enzymkatalysierten Hydrolyse von Polyacrylsäureestern, dadurch gekennzeichnet, dass das Enzym einer Triacylglycerinlipase gemäss SEQ ID NO: 6 ist.

   ---

5. Ansprüche: 1-6(teilweise)

   Verfahren zur enzymkatalysierten Hydrolyse von Polyacrylsäureestern, dadurch gekennzeichnet, dass das Enzym nicht aus einer der in Anspruch 7 erwähnten Esterases ausgewählt wird.

   ---

6. Anspruch: 24

   Polymeres Reaktionsprodukt.

   ---

## ANHANG ZUM EUROPÄISCHEN RECHERCHENBERICHT ÜBER DIE EUROPÄISCHE PATENTANMELDUNG NR.

EP 08 16 0774

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-06-2009

| Im Recherchenbericht angeführtes Patentdokument | Datum der Veröffentlichung | Mitglied(er) der Patentfamilie | Datum der Veröffentlichung |
|---|---|---|---|
| DE 10305076 A1 | 26-08-2004 | KEINE | |
| WO 2005040487 A | 06-05-2005 | CA 2543818 A1 | 06-05-2005 |
| | | EP 1694903 A1 | 30-08-2006 |
| | | PT 103035 A | 29-04-2005 |
| | | US 2007275453 A1 | 29-11-2007 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 3926891 A
- US 20040082023 A1


### In der Beschreibung aufgeführte Nicht-Patentliteratur


- **O'Sullivan ; Birkinshaw.** *Polymer Degradation and Stability,* 2002, vol. 78, 7-15
- **Belluci et al.** *Studies in Conservation,* 1999, vol. 44, 278-281
- **Peterson et al.** *J. Biotechnol.,* 2001, vol. 89, 11-25
- **Valinger et al.** *J. Biotechnol.,* vol. 129, 98-108
- **Ivancic et al.** *J. Biotechnol.,* 2007, vol. 129, 109-122
- **Reiter et al.** *Appl. Microbiol. Biotechnol.,* 2000, vol. 54, 778-785
- **Petersen et al.** Esterasen der Familie VIII. *J Biotechnol,* 2001, vol. 89, 11-25
- **Arpigny ; Jaeger.** *Biochem J,* 1999, vol. 343, 177-183
- **Reiter et al.** *Appl Microbiol Biotechnol,* 2000, vol. 54, 778-785
- **Hasslacher M et al.** *J Biol Chem,* 1996, vol. 271, 5884-5891
- GenBank. AAC49184
- **Hughes et al.** *Arch Biochem Biophys,* 1994, vol. 311, 96-502
- Enzymes. **S. Fukui ; A. Tanaka.** Ullmanns Encyclopedia of Industrial Chemistry. Wiley-VCH, 1985
- Immobilized Biocatalysts. **J.E. Prenosil ; O.M. Kut ; I.J. Dunn ; E. Heinzle.** Ullmanns Encyclopedia of Industrial Chemistry. VCH, 2003, 503-554
- Biotechnology: A comprehensive treatise. VCH, 1987, vol. 7a, 349-404
- Protein purification methods - a practical approach. Oxford University Press, 1990
- Immobilized Biocatalysts. **J.E. Prenosil ; O.M. Kut ; I.J. Dunn, E. Heinzle.** Ullmanns Encyclopedia of Industrial Chemistry. VCH, 2003, 503-554
- **J.F. Kennedy ; J.M.S Cabral.** Biotechnology: A comprehensive treatise. VCH, 1987, vol. 7a, 349-404
- International Union of Biochemistry and Molecular Biology. Enzyme Nomenclature. Academic Press, 1992
- In vitro mutagenesis protocols. Humana Press, 1996
- **Kegler-Ebo DM ; Docktor CM ; DiMaio D.** *Nucleic Acids Res,* 1994, vol. 22, 1593
- **Barettino D ; Feigenbutz M ; Valcärel R ; Stunnenberg HG.** *Nucleic Acids Res,* 1994, vol. 22, 541
- **Barik S.** *Mol Biotechnol,* 1995, vol. 3, 1
- **Eckert KA ; Kunkel TA.** *Nucleic Acids Res,* 1990, vol. 18, 3739
- An efficient random mutagenesis technique using an E.coli mutator strain. **Greener A ; Callahan M ; Jerpseth B.** In vitro mutagenesis protocols. Humana Press, 1996
- **Stemmer WPC.** *Nature,* 1994, vol. 370, 389
- **Stemmer WPC.** *Proc Natl Acad Sci USA,* 1994, vol. 91, 10747
- **Sambrook ; Russell.** Molecular Cloning. Cold Spring Harbor Laboratory Press, 2001
- **Reetz MT ; Jaeger K-E.** *Topics Curr Chem,* 1999, vol. 200, 31
- Methods for optimizing industrial enzymes by directed evolution. **Zhao H ; Moore JC ; Volkov AA ; Arnold FH.** Manual of industrial microbiology and biotechnology. American Society for Microbiology, 1999
- **Zhao ; Arnold.** *Protein Engineering,* 1999, vol. 12, 47-53
- **May et al.** *Nature Biotechnology,* 2000, vol. 18, 317-320
- **von Pearson ; Lipman.** *Proc. Natl. Acad, Sci. (USA),* 1988, vol. 85 (8), 2444-2448
- **Higgins DG ; Sharp PM.** Fast and sensitive multiple sequence alignments on a microcomputer. *Comput Appl. Biosci.,* April 1989, vol. 5 (2), 151-1
- **Narang, S.A.** *Tetrahedron,* 1983, vol. 39, 3
- **Itakura et al.** *Annu. Rev. Biochem.,* 1984, vol. 53, 323
- **Itakura et al.** *Science,* 1984, vol. 198, 1056
- **Ike et al.** *Nucleic Acids Res.,* 1983, vol. 11, 477
- Phosphoamiditmethode. Wiley Press, 896-897
- **Sambrook et al.** Molecular Cloning: A laboratory manual. Cold Spring Harbor Laboratory Press, 1989

- **Sambrook, J. ; Fritsch, E.F. ; Maniatis, T.** Molecular Cloning: A Laboratory Manual. Cold Spring Harbor Laboratory Press, 1989
- **Ausubel et al.** Current Protocols in Molecular Biology. John Wiley & Sons, 1989, 6.3.1-6.3.6
- **Goeddel.** Gene Expression Technology: Methods in Enzymology. Academic Press, 1990, vol. 185
- **T. Maniatis ; E.F. Fritsch ; J. Sambrook.** Molecular Cloning: A Laboratory Manual. Cold Spring Harbor Laboratory, 1989
- **T.J. Silhavy ; M.L. Berman ; L.W. Enquist.** Experiments with Gene Fusions. Cold Spring Harbor Laboratory, 1984
- **Ausubel, F.M. et al.** Current Protocols in Molecular Biology. Greene Publishing Assoc. and Wiley Interscience, 1987
- Cloning Vectors. Elsevier, 1985
- **Smith, D.B. ; Johnson, K.S.** *Gene,* 1988, vol. 67, 31-40
- **Amann et al.** *Gene,* 1988, vol. 69, 301-315
- **Studier et al.** Gene Expression Technology: Methods in Enzymology. Academic Press, 1990, vol. 185, 60-89
- **Baldari et al.** *Embo J.,* 1987, vol. 6, 229-234
- **Kurjan ; Herskowitz.** *Cell,* 1982, vol. 30, 933-943
- **Schultz et al.** *Gene,* 1987, vol. 54, 113-123
- **van den Hondel, C.A.M.J.J. ; Punt, P.J. et al.** Gene transfer systems and vector development for filamentous fungi, in: Applied Molecular Genetics of Fungi. Cambridge University Press, 1991, 1-28
- **Smith et al.** *Mol. Cell Biol.,* 1983, vol. 3, 2156-2165
- **Lucklow ; Summers.** *Virology,* 1989, vol. 170, 31-39
- **Becker, D. ; Kemper, E. ; Schell, J. ; Masterson, R.** New plant binary vectors with selectable markers located proximal to the left border. *Plant Mol. Biol.,* 1992, vol. 20, 1195-1197
- **Bevan, M.W.** Binary Agrobacterium vectors for plant transformation. *Nucl. Acids Res.,* 1984, vol. 12, 8711-8721
- **Seed, B.** *Nature,* 1987, vol. 329, 840
- **Kaufman et al.** *EMBO J.,* 1987, vol. 6, 187-195
- **von Sambrook, J. ; Fritsch, E.F. ; Maniatis, T.** Molecular cloning: A Laboratory Manual. Cold Spring Harbor Laboratory Press, 1989
- Current Protocols in Molecular Biology. Wiley Interscience, 1997
- **Sambrook et al.** Molecular Cloning: A Laboratory Manual. Cold Spring Harbor Laboratory Press, 1989
- **Cooper, F. G.** Biochemische Arbeitsmethoden. Verlag Walter de Gruyter
- **Scopes, R.** Protein Purification. Springer Verlag
- **Harlow, E. ; Lane, D.** Antibodies: A Laboratory Manual. Press, 1988
- **Crueger ; Crueger.** Biotechnologie - Lehrbuch der angewandten Mikrobiologie. R. Oldenbourg Verlag, 1984
- **Balzer et al.** *Nucleic Acids Research,* 1992, vol. 20 (8), 1851-1858